(19) Europäisches Patentamt
European Patent Office
Office européen des brevets

(11) **EP 4 425 366 B1**

(12) **EUROPEAN PATENT SPECIFICATION**

(45) Date of publication and mention of the grant of the patent:
**31.12.2025 Bulletin 2026/01**

(21) Application number: **24160107.9**

(22) Date of filing: **27.02.2024**

(51) International Patent Classification (IPC):
***G06F 30/23*** *(2020.01)* ***G06F 30/28*** *(2020.01)*
***G16C 10/00*** *(2019.01)* ***G16C 60/00*** *(2019.01)*

(52) Cooperative Patent Classification (CPC):
**G06F 30/28; G06F 30/23; G16C 10/00; G16C 60/00;** G06F 2111/10; G16C 20/10

(54) **MULTISCALE REACTIVE FLOW IN COMPLEX MICROSTRUCTURES**

MEHRSTUFIGER REAKTIVER FLUSS IN KOMPLEXEN MIKROSTRUKTUREN

FLUX RÉACTIF MULTI-ÉCHELLE DANS DES MICROSTRUCTURES COMPLEXES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.02.2023 US 202363487509 P**

(43) Date of publication of application:
**04.09.2024 Bulletin 2024/36**

(73) Proprietor: **Dassault Systèmes Americas Corp.**
**Waltham, MA 02451 (US)**

(72) Inventors:
- **Salazar-Tio, Rafael**
  **San Ramon, 94582 (US)**
- **Paspureddi, Akhilesh**
  **Houston, 77024 (US)**
- **Balasubramanian, Ganapathi Raman**
  **Pleasanton, 94588 (US)**
- **Crouse, Bernd**
  **Berkeley, 94708 (US)**

(74) Representative: **Bandpay & Greuter**
**11 rue Christophe Colomb**
**75008 Paris (FR)**

(56) References cited:
- **AN SENYOU ET AL: "Lattice-Boltzmann simulation of dissolution of carbonate rock during CO2-saturated brine injection", CHEMICAL ENGENEERING JOURNAL, ELSEVIER, AMSTERDAM, NL, vol. 408, 21 October 2020 (2020-10-21), XP086450217, ISSN: 1385-8947, [retrieved on 20201021], DOI: 10.1016/J.CEJ.2020.127235**
- **J. A. GREATHOUSE ET AL: "Molecular Dynamics Simulation of Diffusion and Electrical Conductivity in Montmorillonite Interlayers", THE JOURNAL OF PHYSICAL CHEMISTRY C, vol. 120, no. 3, 20 January 2016 (2016-01-20), US, pages 1640 - 1649, XP055268089, ISSN: 1932-7447, DOI: 10.1021/acs.jpcc.5b10851**
- **CHEN LI ET AL: "Pore-scale simulation of multicomponent multiphase reactive transport with dissolution and precipitation", INTERNATIONAL JOURNAL OF HEAT AND MASS TRANSFER, ELSEVIER, AMSTERDAM, NL, vol. 85, 6 March 2015 (2015-03-06), pages 935 - 949, XP029213141, ISSN: 0017-9310, DOI: 10.1016/J.IJHEATMASSTRANSFER.2015.02.035**
- **PARKHURST DAVID L ET AL: "PhreeqcRM: A reaction module for transport simulators based on the geochemical model PHREEQC", ADVANCES IN WATER RESOURCES, vol. 83, 6 June 2015 (2015-06-06), GB, pages 176 - 189, XP093172360, ISSN: 0309-1708, DOI: 10.1016/j.advwatres.2015.06.001**
- **SOULAINE CYPRIEN ET AL: "porousMedia4Foam: Multi-scale open-source platform for hydro-geochemical simulations with OpenFOAM", ENVIRONMENTAL MODELLING & SOFTWARE, ELSEVIER, AMSTERDAM, NL, vol. 145, 15 September 2021 (2021-09-15), XP086815642, ISSN: 1364-8152, [retrieved on 20210915], DOI: 10.1016/J.ENVSOFT.2021.105199**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 425 366 B1

## Description

TECHNICAL FIELD

The disclosure relates to the field of computer programs and systems, and more specifically to a method, system, and program for determining behavior of a reactive flow system.

BACKGROUND

Reactive flow modeling is a tool to analyze processes that involve fluid flow and chemical reactions. Amongst other examples, such processes include geological carbon capture, groundwater remediation, enhanced oil recovery (EOR) with $CO_2$ injection, *in-situ* mining by leaching, corrosion and fouling of structural materials, recycling, and electrochemical systems for sustainable energy applications such as batteries, fuel cells, and electrolizers.

Also known is AN SENYOU ET AL: "Lattice-Boltzmann simulation of dissolution of carbonate rock during CO2-saturated brine injection", CHEMICAL ENGENEERING JOURNAL, ELSEVIER, AMSTERDAM, NL, vol. 408, 21 October 2020 (2020-10-21), DOI: 10.1016/J.CEJ.2020.127235, which discloses a pore-scale volumetric lattice-Boltzmann model coupled with the geochemical simulator, PhreeqcRM, to investigate the pore-scale reactive transport in carbonate rocks.

Also known is J. A. GREATHOUSE ET AL: "Molecular Dynamics Simulation of Diffusion and Electrical Conductivity in Montmorillonite Interlayers", THE JOURNAL OF PHYSICAL CHEMISTRY C, vol. 120, no. 3, 20 January 2016 (2016-01-20), pages 1640-1649, DOI: 10.1021/acs.jpcc.5b10851, which discloses molecular dynamics simulation to investigate trends in cation and water diffusion in montmorillonite interlayers, looking specifically at the effects of layer charge, interlayer cation and cation charge (sodium or calcium), water content, and temperature.

SUMMARY

While reactive flow modeling tools exists, these tools could benefit from improvements. Embodiments provide such improved functionality.

Reactive flow modeling has applications in many industries, however, the majority of the advancement in the field has been made through development of geochemical modeling in subsurface systems. Thus, the example embodiments disclosed herein illustrate representative applications selected in this class, namely reactive flow modeling of *in-situ* leaching of copper. However, it is noted that embodiments are not limited to simulating/modeling *in-situ* leaching of copper (metals generally) and, instead, embodiments can be used to analyze any reactive flow systems. *In-situ* leaching involves underground circulation of acid to dissolve a target mineral to extract or produce a metal of interest, copper in this example. Modeling the real-time change in composition of fluid and fluid-rock interface(s) through reactive flow modeling enables optimization of copper production through *in-situ* leaching and prediction of probable outcomes of alternate design and operating conditions, facilitating process optimization.

There are two broad categories of models that exist in reactive flow modeling: (i) continuum level models[42,47] and (ii) pore scale models,[28,29] depending on the scale of the physics of interest. At pore scale, the microstructure is fully resolved, effective species transport coupled with surface reactions per volume are directly simulated within the microstructure, and the inputs are the material's properties. At continuum scale, the microstructure is unresolved, and the model requires the input of certain properties such as the effective transport, the reaction rates restricted by the microstructure, material porosity, or the surface to volume ratio of the material. The use of three-dimensional (3D) microscopy imaging techniques, such as x-ray micro-tomography (microCT) as input for the actual pore-scale structure is possible, but not mainstream in reactive flow modeling. Real world applications require both aspects, scales and 3D imaging to be included in the model in order to produce accurate results without the need of experimental measurements or limiting simplified model assumptions. This missing multiscale aspect in current reactive flow solutions also includes a smaller molecular level modeling for materials, reaction, and diffusion parameters. Another limitation in current reactive flow solvers is related to accurately describing multi-phase flow (the flow of multiple fluids simultaneously, like oil, water, and gas).

In summary, existing pore scale models do not consider flow through unresolved pores (e.g., multiscale flow simulations), and cannot model multiphase flow accurately. Existing continuum scale models consider simplified flow and transport equations based on permeability and porosity. Hence, there is no solution to accurately solve multiphase and multiscale reactive flow at pore scale along with upscaling to simulate a field scale problem. Both pore scale and continuum scale models in the literature rely on open-source databases for material, reaction, and diffusion parameters. Finally, accurately accounting for secondary speciation in the flow solution after primary reaction is mostly common in continuum models, but somehow limited in pore-scale models. It is noted that "accounting for secondary speciation" is also referred to as accurately modeling the water chemistry or including homogeneous reactions in the reactive flow model.

The invention is defined by the independent claims. To accurately model the reactive flow applications as

described herein, embodiments implement a reactive flow model with a robust multiphase (coupled flow of liquid and gas) and multiscale (flow though resolved and unresolved pore) flow simulator, and multiscale (pore scale and continuum scale) reaction simulator. 3D imaging based microstructural models are also employed in embodiments to avoid simplistic approximations to property correlations. Further, it is noted that reactive flow modeling utilizes many inputs from materials, reaction, and diffusion parameters, which are not easy to find in literature and data repositories for all applications. To solve this problem, embodiments obtain chemical properties through use of a molecular modeling component that calculates chemical properties. Further still, the reactive flow modeling embodiments described herein include homogeneous reactions in addition to heterogeneous reactions in the reactive flow model.

Embodiments are directed to computer-implemented methods and systems for determining behavior of reactive flow systems. One such embodiment defines a plurality of models of the reactive flow system, wherein each defined model represents the reactive flow system at a respective scale. In turn, a velocity field for the reactive flow system is determined using a first model, at a first respective scale, of the defined plurality of models and a diffusivity for the reactive flow system is determined using a second model, at a second respective scale, of the defined plurality of models. In an embodiment, determining a velocity field and determining a diffusivity is automatically performed by one or more digital processors. Next, a plurality of reaction parameters for the reactive flow system are defined. Then, the behavior of the reactive flow system is automatically determined by using the determined velocity field, the determined diffusivity, and the defined plurality of reaction parameters as inputs to a reactive transport solver.

Another embodiment of the method provides computer implemented methods and systems for determining component concentrations of a reactive flow system.

In some aspects, at least one model of the defined plurality of models represents the reactive flow system at a microscale, a molecular scale, or a sub-surface scale.

In some aspects, at least one model of the defined plurality of models is a geometric model indicating properties of the reactive flow system.

In some aspects, defining a given model of the plurality of models of the reactive flow system comprises defining a model of one or more heterogeneous surface reactions and, in turn, modeling rate laws, for the defined model of the one or more heterogeneous surface reactions, as functions of mineral dissolution and precipitation. To continue, such an embodiment defines the given model based upon the modeled rate laws and a model of one or more homogeneous bulk reactions.

In some aspects, the determining the velocity field for the reactive flow system using the first model includes receiving an image of a material in the reactive flow system and segmenting (i.e., bisecting, classifying, etc.) the image into a plurality of phases where each phase represents a material, solid, or fluid. In turn, the velocity field of the reactive flow system is determined based on the plurality of phases of the image using the first model, wherein the first model is a single-phase fluid flow model.

In some aspects, the determined velocity field is a multiphase velocity field.

In some aspects, the material is porous. In some aspects, the material further comprises one or more fractures. In some aspects, the material is a nano-porous clay material. In some aspects, the material is Wyoming type montmorillonite having a formula of $Cu_{0.66}[Al_{3.33}Mg_{0.66}]$ $[Si_8]$ $O_{20}[OH]_4$.

In some aspects, the determining the diffusivity for the reactive flow system using the second model comprises providing parameters of a bulk salt solution and parameters of a salt solution in a clay interlayer nano-pore as inputs for a molecular dynamic simulation. These inputs are then used to perform the molecular dynamic simulation as a function of temperature and salt concentration. In such an embodiment, results from performing the molecular dynamics simulation indicate the diffusivity for the reactive flow system.

In some aspects, the diffusivity is an ion diffusivity. In some aspects, the ion diffusivity is ion diffusivity of copper $(Cu)^{2+}$.

In some aspects, the plurality of reaction parameters for the reactive flow system are defined using input data. In some aspects, the input data is obtained from at least one of: simulation results and a database.

In some aspects, the determining the behavior of the reactive flow system by using the determined velocity field, the determined diffusivity, and the defined plurality of reaction parameters as inputs to the reactive transport solver comprises: solving an advection-diffusion-reaction equation using the reactive transport solver with the inputs. In such an embodiment, results of the solving indicate the behavior of the reactive flow system.

In some aspects, the behavior of the reactive flow system is a concentration profile. In some aspects, the concentration profile is a concentration profile of copper $(Cu)^{2+}$.

In some aspects, the method further comprises: updating the first model and the second model based on the determined behavior of the reactive slow system; determining an updated velocity field for the reactive flow system using the updated first model; determining an updated diffusivity for the reactive flow system using the updated second model; and determining an updated behavior of the reactive flow system by using the updated determined velocity field, the updated determined diffusivity, and the defined plurality of reaction parameters as inputs to the reactive transport solver. In some aspects, the method further comprises: iterating: (i) the updating, (ii) the determining an updated velocity field, (iii)

the determining an updated diffusivity, and (iv) the determining an updated behavior of the reactive flow system until the determined updated behavior of the reactive flow system reaches a steady state.

Embodiments described herein also provide computer-implemented methods and systems for determining component concentrations of reactive flow systems.

Yet another embodiment is directed to a system that includes a processor and a memory with computer code instructions stored thereon. In such an embodiment, the processor and the memory, with the computer code instructions, are configured to cause the system to implement any embodiments or combination of embodiments described herein.

Another embodiment is directed to a cloud computing implementation for determining behavior of a reactive flow system. Such an embodiment is directed to a computer program product executed by a server in communication across a network with one or more clients. The computer program product comprises program instructions which, when executed by a processor, causes the processor to implement any embodiments or combination of embodiments described herein.

It is noted that embodiments of the method, system, and computer program product may be configured to implement any embodiments, or combination of embodiments, described herein.

Yet another embodiment provides a multiscale reactive flow model to simulate *in-situ* leaching of copper in heterogeneous porous microstructures. In such an embodiment, a workflow is utilized that combines fluid flow simulations with advection-diffusion-reaction simulations, both of which are employed to model reactive flow. Such a workflow may include flow in resolved and unresolved pore structures and can also utilize parameters from molecular simulation (ionic diffusivity) and reaction databases (reaction rate parameters). Embodiments have also been validated by comparing embodiment determined results with other open-source codes for a model calcite dissolution on acid injection. A molecular dynamics model of clay is also presented to estimate ionic diffusivity in nano-porous media and a salt dissolved in water model is implemented to estimate ionic diffusivity in an open fracture. This model is applied to copper mining by leaching to analyze the reactive flow through a fractured digital rock model of a subsurface sample. The results were analyzed by tracking the concentration distribution along the pore space structure and calculating the outlet concentration of copper to confirm the leaching path. Several sensitivity studies described herein below show the robustness of embodiments as well as illustrate the importance of the acid inlet flow conditions and different reaction types and scales on copper production. An embodiment systematically increases the complexity of the model from a single scale surface reaction model to also include competitive bulk solution reactions, and flow through porous media to model multiscale reactive flow. Results from embodiments show that a multi-scale flow model with homogeneous bulk and heterogeneous surface reactions accurately models copper leaching.

## BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.

FIG. 1 is a flowchart depicting a method for determining behavior of a reactive flow system according to an embodiment.

FIG. 2 is a schematic illustrating copper leaching by acid injection at pore scale.

FIG. 3 is an example embodiment of a workflow for determining characteristics of a reactive flow system according to an embodiment.

FIG. 4 illustrates steps of an example sequential workflow used to obtain a velocity field from a Lattice Boltzmann method (LBM) simulation with and without a multiscale option of including a flow in clay nano-pores.

FIG. 5A is an illustration showing dissolution and precipitation surface reactions on mineral grains in an example embodiment.

FIG. 5B is a diagram showing an example mechanism combining surface reactions and bulk reactions to obtain concentrations of example species.

FIG. 5C is an example reactive flow method to update concentration of example species in voxels of a microstructure model according to an embodiment.

FIG. 6A is a plot of $H^+$ concentration as a function of time in two-dimensional (2D) geometry of an example calcite pellet placed in a rectangular channel.

FIG. 6B is a comparison plot of $H^+$ concentration (in M) as a function of the length of geometry between an example simulation and GeoChemFoam.

FIG. 6C is an example simulation of spatial $H^+$ concentration using GeoChemFoam.

FIG. 7A is an example digital rock microstructure used to model copper leaching by acid injection according to an embodiment.

FIG. 7B is a plot of steady state copper concentration as a function of time as determined by an embodiment.

FIG. 8A shows an example molecular dynamic (MD) simulation structure of copper chloride in bulk water.

FIG. 8B shows an example MD structure of clay with copper chloride in a hydrated nano-pore according to an embodiment.

FIG. 8C and 8D shows plots of copper and chloride ion diffusivities, respectively, in bulk water presented at different salt concentrations and temperatures.

FIG. 8E is a plot of copper and chloride ion diffusivities in a nano-porous clay model presented at different temperatures and 0.1 M of chloride ions.

FIG. 9 shows a three-dimensional (3D) microstructure of an example rock with cupperoferrite mineral (white regions) with fractures.

FIG. 10A is a surface plot of copper outlet concentration as a function of velocity (Peclet number) and reaction rate (Damkohler number).

FIG. 10B is a contour plot of copper outlet concentration as a function of velocity (Peclet number) and reaction rate (Damkohler number).

FIG. 11A is a plot of outlet concentration of copper as a function of time at a Peclet number of 10 and varying Damkohler numbers.

FIG. 11B is a plot of outlet concentration of copper as a function of time at a Damkohler number of 2.5 and varying Peclet numbers.

FIG. 11C is a plot of steady state copper concentration as a function of Damkohler number.

FIG. 11D is a plot of steady state copper concentration as a function of Peclet number.

FIG. 12A is a plot of copper species concentrations as a function of inlet pH.

FIG. 12B is a plot of iron species concentrations as a function of inlet pH.

FIG. 13A is a plot of $Cu^{2+}$ outlet concentration as a function of time for an example model using only heterogeneous surface reactions.

FIG. 13B is a plot of $Cu^{2+}$ outlet concentration as a function of time for an example model using both homogenous bulk reactions and heterogeneous surface reactions.

FIG. 13C is a plot of steady state $Cu^{2+}$ outlet concentration as a function of inlet acid concentration.

FIG. 14A is a cross-section image of an example digital rock visualizing copper concentration through a fracture for a single scale reactive flow system.

FIG. 14B is a cross-section image of an example digital rock visualizing copper concentration through a fracture for a multiscale reactive flow system.

FIG. 14C is a plot of copper outlet concentration as a function of time for each of the reactive flow systems shown in FIGs. 14A-B.

FIG. 15 is an example embodiment of a multiscale reactive flow simulation of a leached metal through a pore space.

FIG. 16 is a simplified block diagram of a computer system for determining behavior of a reactive flow system according to an embodiment.

FIG. 17 is a simplified block diagram of a computer network environment in which an embodiment of the present invention may be implemented.

DETAILED DESCRIPTION

A description of example embodiments follows.

**Introduction**

Reactive flow modeling is a tool to analyze any process that involves fluid flow and chemical reactions, such as geological carbon capture,[14,23] groundwater remediation,[43] enhanced oil recovery (EOR) with $CO_2$ injection,[26] corrosion and fouling of structural materials[41] and redox flow batteries.[11] One such application that is of interest to this current study is *in-situ* leaching of copper.[38] *In-situ* leaching involves circulation of acid to dissolve a target mineral to produce or extract a metal of interest. With the increase in world copper demand, *in-situ* leaching is proving to be a low-cost method of extracting copper.[32,38] Many challenges still exist to efficiently extract copper from *in-situ* leaching, such as determining optimal composition of the lixiviant, achieving uniform contact of injection fluid with the mineral, and changing permeability and porosity of the rock due to mineral dissolution and precipitation. Hence, modeling the real-time change in composition of fluid and fluid-rock interface through reactive flow modeling is beneficial to optimize copper production through in-situ leaching and to predict probable outcomes of alternate design and operating conditions. Thus, reactive flow modeling can be used to optimize real-world *in-situ* leaching of copper. Further, it is noted that while embodiments are described herein as being used to simulate and optimize *in-situ* leaching of copper, embodiments are not limited to such systems and embodiments can be used to model and optimize any reactive flow systems.

Even though reactive flow has applications in many industries, the majority of the advancement in the field has been related to geochemical modeling in subsurface systems.[2,27,28,31,49] In reactive flow modeling, two broad categories

of models exist: continuum level models[42] and pore scale models.[5,21] The use of continuum level models and pore scale models typically depends on the scale of the physics of interest. At pore scale, the microstructure is fully resolved, effective species transport coupled with surface reactions per volume are directly simulated within the microstructure, and the inputs are the materials properties. At continuum scale, the microstructure is unresolved and effective transport and reaction rates restricted by the microstructure connectivity need to be provided as inputs. Often, the inputs are provided using simplified microstructure property relationships, like porosity correlations for permeability and surface to volume ratio for a pack of spheres of parallel tubes. A strategy used by existing methods is to first model reactive transport at pore scale for a given microstructure and, then, follow the study with upscaling the property relationships and the model to continuum scale to handle a large scale.[25] The importance of this multiscale strategy to better understand the pore scale physics of reactive flow has been shown in the last few decades by advancements made in both laboratory experiments[30] and reactive flow modelling[2].

Two key properties of interest in reactive flow are reaction rates and diffusion coefficients. Reaction parameters like rate constants, equilibrium constants, and thermodynamic parameters are available in open-source thermodynamic databases such as LLNL database, PHREEQC database, MINTEQ database. Several open-source reactive flow simulators like PHREEQC,[35] Reaktoro,[13] TOUGHREACT[47] utilize these databases to perform chemical reactions and thermodynamic calculations to update the composition of the fluid phase. Each database is targeted to certain applications and depending on the application one is modeling; it is recommended to choose the right database.

Diffusion of solutes in water in nano-porous material like clay, and in bulk fluid are often modeled using molecular dynamic simulations.[8,22,39,40,45] Clay has unique properties including swelling in presence of water, which is a key reason to facilitate ionic mobility within the clay.[10] Several researchers have managed these problems in the past, and robust forcefields (molecular models[12]) exist for atomic interactions for ions and clay. However, these studies are limited to simple salts such as alkali metal and alkali earth metals at room temperatures. Since many reactive flow applications involve simple species like $Na^+$ and $Ca^{2+}$, it is possible to find diffusion parameters in the literature and, thus, researchers do not focus on explicitly modeling diffusion in general. However, there is limited literature and data available on less common ionic species, and $Cu^{2+}$ is one such case. Hence, in this document, incorporating molecular simulations into reactive flow workflows is shown to provide a general framework to any application, where diffusivity is one of the parameters to choose.

The fluid flow numerical simulation approach described herein is the Lattice Boltzmann method (LBM). LBM has been extensively used as a direct simulation approach for resolvable pore-scale events and to determine physical properties such as wettability, capillary effects, and viscous fingering phenomena.[16,20,44,48] LBM is an explicit method that often operates on a cubic lattice that solves a discretized version of the Boltzmann equation and has its origins in kinetic theory. Although multiphase capability is available to simulate immiscible multiphase scenarios, like oil and water, the example described herein uses the single-phase flow LBM. Here single-phase flow simulations are performed on the 3D microstructure model, extracted from direct x-ray micro-tomography imaging (microCT) of a representative sample, with the goal being to compute the flow velocity field in the interconnected pore space of the 3D microstructure. Moreover, a multiscale extension to the LBM[17,18] allows for partial flow through some semi-permeable materials, or porous media (PM) regions, with pore connections not resolved explicitly at the resolution of the 3D microstructure model. Nano-porous clays are examples of such material, and the multiscale extension described herein can compute effective flow velocity fields in the regions filled with these types of semipermeable materials.

Herein, a model is presented that combines the above components of multiscale reactive flow, e.g., microCT image to flow velocity fields, homogeneous and heterogeneous reactions, ion diffusion, and multiscale transport including resolved pore structures and unresolved porous material regions. Embodiments include a multiscale multispecies reactive flow workflow that has been implemented and validated. Hereinbelow an application of an embodiment to mineral dissolution in a 3D digital rock image is presented to illustrate the influence of reaction rate, velocity, reaction types, pH, and multiscale flow through porous media. A methodology used to model *in-situ* copper leaching is also discussed. Reactive flow workflows are introduced, and different components of reactive flow are explained. Example embodiments begin with validation cases of diffusivity of water in bulk using molecular simulations. Then, validation of the reactive flow methodology is performed by modeling a test case of a calcite grain dissolution and comparing results with published results of other pore scale reactive flow simulations. This validation is followed by presentation of a model for copper leaching. To get diffusivities for the reactive flow model, a molecular model of bulk copper ions in aqueous solution was used for diffusion in micro-pores and in clay nano-pore for porous media diffusivities. The reactive flow model was used to investigate the influence of pH, fluid velocity, and surface reactivity on copper production by conducting a series of sensitivity studies. The homogeneous bulk reactions were then incorporated into the model to understand its influence on copper production. The model was extended to include transport through both open fractures and nano-porous clays and show its influence on copper production.

Models, as described above, can be utilized in methods described herein to determine behavior of reactive flow systems. FIG. 1 illustrates one such example method 110 for determining behavior of a reactive flow system. The method 110 is computer-implemented and may be performed via any combination of hardware and software as is known in the art. For example, the method 110 may be implemented via one or more processors with associated memory storing computer

code that causes the processor to implement steps 111, 112, 113, 114, and 115 of the method 110. Further, it is noted that embodiments are described herein as being capable of being implemented in software provided by Applicant, however, embodiments are not limited to being implemented into existing software and, instead, embodiments can be performed using any combination of hardware and software as is known in the art.

Returning to FIG. 1, the method 110 begins at step 111 by defining, e.g., in computer memory, a plurality of models of a reactive flow system, wherein each defined model represents the reactive flow system at a respective scale. According to an embodiment of the method 110, example scales include microscale, molecular scale, and sub-surface scale, amongst others. In an example embodiment, models at different scales are defined at step 111 using different techniques. For instance, 3D imaging by x-ray tomography, 3D molecular modeling, and/or 3D seismic imaging, amongst other examples, may be utilized at step 111 to generate the computer models at respective scales. According to an embodiment, at least one model of the plurality of models defined at step 111 is a geometric model indicating properties of the reactive flow system. In another embodiment of the method 110, at step 111, a given model is defined as a model of one or more heterogeneous surface reactions. In such an embodiment, reaction rate laws are modelled for the defined model of the one or more heterogeneous surface reactions as functions of mineral dissolution and precipitation. In turn, the given model is defined based upon the modeled reaction rate laws and a model of one or more homogeneous bulk reactions. According to an embodiment, a heterogeneous surface reaction refers to a reaction between species dissolved in a fluid and a mineral surface that is in contact with the fluid. Further, in an embodiment, a homogeneous surface reaction refers to a reaction between species dissolved in a fluid. Reaction rate laws are provided for both types of reactions in embodiments.

Returning to FIG. 1, at step 112 the method 110 continues by determining a velocity field for the reactive flow system using a first model, at a first respective scale, of the defined plurality of models. According to an embodiment, the determined velocity field is a multiphase velocity field. In an example embodiment, the velocity field is determined for the reactive flow system using the first model at step 112 by, receiving an image of a material in the reactive flow system and segmenting (i.e., bisecting or classifying) the image into a plurality of phases, e.g., phases or segments representing materials, solids, and fluids. In turn, the velocity field is determined based on the plurality of phases of the image using the first model, wherein the first model is a single-phase fluid flow model. According to an embodiment, a computational fluid dynamics (CFD) simulation is implemented at step 112 to determine the velocity field. In an example embodiment, the first model is a microCT model and this model is utilized at step 112 in a Lattice Boltzmann (LBM) flow simulation to determine the velocity field. According to one such embodiment, the LBM flow simulation technique implemented at step 112 allows for sub-resolution flow and accurate multiphase flow. For instance, in such an embodiment, the functionality described in U.S. Patent Publication No. 2022/0207219 A1 may be used at step 112.

According to an embodiment, the material (e.g., the material shown in the received image) is porous. According to another embodiment, the material may further comprise one or more fractures. In another embodiment, the material is a nano-porous clay material. According to yet another embodiment, the material is Wyoming type montmorillonite having a formula of $Cu_{0.66}[Al_{3.33}Mg_{0.66}]\ [Si_8]\ O_{20}[OH]_4$.

The method 110 continues at step 113 by determining a diffusivity for the reactive flow system using a second model, at a second respective scale, of the defined plurality of models. In an embodiment, the diffusivity is determined at step 113 using a known technique. In one such embodiment, the diffusivity is determined using the Forcite module of the BIOVIA Material Studio® software application. According to an embodiment of the method 100, the diffusivity for the reactive flow system is determined using the second model by first, providing parameters of a bulk salt solution and parameters of a salt solution in a clay interlayer nano-pore as inputs for a molecular dynamics simulation. Second, the inputs are used to perform the molecular dynamics simulation as a function of temperature and salt concentration. Results of performing the molecular dynamics simulation indicate the diffusivity for the reactive flow system. In an example embodiment, the second model, i.e., the model used at step 113, is a chemical composition model. In such an embodiment, the chemical composition model is used in a molecular dynamics simulation at step 113 to determine the diffusivity. According to an embodiment, the molecular dynamics simulation method may be implemented in BIOVIA Materials Studio® using a Forcite module. In another embodiment, the diffusivity is an ion diffusivity. According to an embodiment, the ion diffusivity is ion diffusivity of copper $(Cu)^{2+}$.

At step 114, the method 110 continues by defining a plurality of reaction parameters for the reactive flow system. According to an embodiment, the plurality of reaction parameters for the reactive flow system are defined using input data. According to an embodiment, the input data is obtained from at least one of simulation results and a database.

To continue, at step 115 the method 110 automatically determines the behavior of the reactive flow system by using the determined velocity field, the determined diffusivity, and the defined plurality of reaction parameters as inputs to a reactive transport solver. According to an embodiment, the behavior is determined at step 115 using Equation 5 described below. In an example embodiment, a reactive transport equation is solved at step 115 to determine the behavior of the reactive flow system. According to an embodiment, determining the behavior of the reactive flow system by using the determined velocity field, the determined diffusivity, and the defined plurality of reaction parameters as inputs to the reactive transport solver includes solving an advection-diffusion-reaction equation using the reactive transport solver with the inputs. In such an embodiment, results of the solving indicate the behavior of the reactive flow system. In embodiments

of the method 110, the determined behavior of the reactive flow system is a concentration profile, e.g., a species concentration profile. According to another embodiment, the concentration profile is a concentration profile of copper $(Cu)^{2+}$. In another example embodiment, the behavior determined at step 115 is an overall production rate for the chemical species of interest (products).

The method 110 may further comprise updating the first model and the second model based on the determined behavior of the reactive flow system, determining an updated velocity field for the reactive flow system using the updated first model, determining an updated diffusivity for the reactive flow system using the updated second model, and determining an updated behavior of the reactive flow system by using the updated velocity field, updated determined diffusivity, and the defined plurality of reaction parameters as inputs to the reactive transport solver. Further, such an embodiment of the method 110 may further comprise iterating: (i) the updating, (ii) the determining an updated velocity field, (iii) the determining an updated diffusivity, and (iv) the determining an updated behavior of the reactive flow system until the determined updated behavior of the reactive flow system reaches a steady state.

## Exemplification

An example embodiment provides a workflow to solve multiscale reactive flow in porous microstructures. To model reactive flow, an embodiment models chemical interactions (with molecular models, heterogeneous and homogeneous reactions) combined with flow through microstructure. The resulting learning can be extended from microscale simulation to field scale simulation. Traditionally, reactive flow has been studied either at microscale or at field scale simulation; with the example workflow embodiments described herein, the gap to simulate reactive flow at multiscale is bridged.

The reactive flow model workflow, according to an embodiment, combines the various components described herein, such as multiphase multiscale reactive flow, *e.g.,* microCT image to flow velocity fields, multiphase flow, homogeneous and heterogeneous reactions, ion diffusion, and multiscale transport modeling including resolved pore structures and unresolved porous material regions. A demonstration of this multiphase, multiscale, and multispecies reactive flow workflow is presented with an application to mineral dissolution in a 3D digital rock image to understand the influence of reaction rate, velocity, types of reactions, pH, and multiscale flow through porous media.

Embodiments have multiple components that enable a unique opportunity for global optimization in design and operation, by working together in a simulation ecosystem such as Dassult Systemes 3DS Experience Platform that allow multiscale and 14 multiphysics modeling, simulation and process optimization.

## Example Methodology

## Example: Copper Leaching

An example application of the method 110 of FIG. 1 is provided below.

At field scale *in-situ* leaching is performed with an array of wells for injecting acid into a sub-surface formation where fluid flows in an interconnected fractured rock bearing the target mineral and the production solution after the mineral dissolution reaction is extracted through wells and passed through hydrometallurgy post-processing to extract copper from the solution. FIG. 2 is a schematic illustrating copper leaching by acid injection at pore scale for a fractured rock model 225. At field scale, acid is injected 221 through a parent well into fractured subsurface rock and a copper containing solution 224 is collected for further processing through a child well. At pore scale, acid is injected into the fractured rock to produce copper at the outlet. The digital rock model 225 (i.e., computer-based model) has four segmented phases: reactive mineral 226a, non-reactive mineral 226b, nano-porous mineral 226c, and pore 226d. FIG. 2 illustrates copper production at pore scale that can be modeled using embodiment by solving flow, mass transport, and chemical reactions. In the model 225 illustrated in FIG. 2, acid is injected at one end of the rock (inlet) 221 and, then, the acid moves due to advection 222a-b through the fracture network (black region 226d). In the process, acid diffuses 223a-b through nano-porous clay (dark gray region 226c) and reacts with the mineral (white region 226a) and copper is collected at the far end of the rock (outlet) 224. The different physics for the copper leaching illustrated in FIG. 2 can be systematically and discretely handled in a multiscale reactive flow workflow according to an embodiment.

## Example: Reactive Flow Workflow

An example implementation of the application of the method 110 of FIG. 1 to copper leaching is described below.

Embodiments can couple different parts of a reactive flow process using the workflow 330 illustrated in FIG. 3. In the workflow 330 of FIG. 3, at step 331, a microCT image, e.g., 337 is segmented into different regions and fed into an LBM single-phase fluid flow simulator to determine a flow velocity field (e.g., 338) at step 332. At step 333, a reaction database, such as PHREEQC, is used to get rate parameters to make reaction calculations, e.g., calculations shown in table

339.Further, at step 334, molecular simulations are performed using a solver, such as BIOVIA Materials Studio® version 2021, to determine ionic diffusivity coefficients (as illustrated in the model 340). In turn, at step 335, the velocity field (from step 332), rate parameters (from step 333), and diffusivity (from step 334) are used as inputs to a reactive transport simulation solver, which determines an advection-diffusion-reaction equation which can be used at step 336 to determine different species concentration profiles (such as those illustrated in the plot 341) in the 3D pore space of a digital rock model (e.g., the model 342).

**Example: Fluid Flow Modeling**

An example implementation of step 112 of the method 110 in a copper leaching example is described below.

Digital rock (e.g., computer-based model, computer-aided design (CAD) model, etc.) applications of LBM can be used in embodiments to predict fluid flow permeability of porous rocks (represented by the models). Embodiments may employ 3D imaging techniques, such as micro-CT imaging, suitable for the pore-scale description of porous rocks[16.20,44,48] so as to utilize LBM. LBM is based on the kinetic equation of fluid particles, and represents a statistical description of molecular behavior of the fluid particles. The LBM can be used to simulate the dynamic behavior of fluid flow without directly solving the continuum fluid mechanics equations. Moreover, the LBM based fluid solvers are considered competitive alternatives to traditional Navier-Stokes PDE-based numerical methods, particularly in applications involving complex geometries, like porous media flow in digital rock (models).

A computer-based model geometry used in an LBM solver of an embodiment can include 3D volume elements corresponding to voxels from microCT scanned images. In addition, a computer-based model used in embodiments can include pore/solid surface elements, called surfels. Using surfels enables high fidelity representation of the pore/solid geometry interface with an effective sub voxel resolution accuracy. No slip boundary conditions can be applied between fluid and surfels. The usage of surfels is a unique feature of the specific LBM applied by such an embodiment and makes computational costs manageable by allowing for a practical number of grid cells to be used in the rock volume.

Here, a multiscale framework can also be considered. Direct simulation can be performed on the resolved pore space while partial flow is allowed through unresolved porous media (PM). For the rock sample used in such an embodiment, the unresolved PM regions can be associated with clay nano-porous material. Transport through the PM regions can be achieved by applying a LBM multiscale extension[17,18] to the regions within the 3D model identified as clay nano-porous material during segmentation. An effective PM permeability can be independently estimated and inputted to control the strength of transport within the PM regions.

FIG. 4 shows a sequence of images from left to right illustrating steps of an example sequential workflow used to determine a velocity field from an LBM simulation with and without a multiscale option of including a flow in clay nano-pores. Image 441 is an input microCT image where grey values represent the X-ray absorption coefficient. The image 442 illustrates a segmented rock model (generated based on the microCT image 441) where phases of the rock model are depicted by shading. Specifically, shading 445a depicts reactive mineral, shading 445b illustrates non-reactive mineral, shading 445c illustrates micro-porous mineral, and shading 445d illustrates porosity. The model 442 can, in turn, be used in an LBM according to an embodiment to determine a flow velocity field. Image 443 illustrates flow velocity field results from the LBM simulation that considers only the flow through the resolved pore space. Image 444 illustrates flow velocity field results from the LBM multiscale simulation that considers the flow through both the resolved pore space and the PM marked regions. It is noteworthy that the computed fluid flow permeability value is the same for both flow simulations, which indicates that the PM regions' contribution to the flow are negligible when compared to the flow contribution from the larger fracture network. While this is true, it may still be expected that a non-negligible contribution to the copper production rate will be reachable since more regions are marked as reactive-mineral when PM regions are added to the contacted flow path.

**Example: Reaction Modeling**

An example implementation of step 114 in the method 110 for an illustrative copper leaching example is provided below.

In an embodiment, there are two categories of reactions in a reactive transport model, namely a heterogeneous surface reaction category and a homogeneous bulk reaction category. In an embodiment, first, reaction rate laws for surface reactions are modeled as functions of mineral dissolution and precipitation to concentrations of aqueous species involved in the reactions (based on transition state theory).

$$r = k \prod_{s} \{s\}^{n_s} (1 - \Omega)^{\alpha} \tag{1}$$

In equation (1) above, r is the reaction rate (mol $cm^{-2}s^{-1}$), k is the reaction rate constant, {s} is activity of the chemical species in the system that has a catalytic effect on the reaction, $n_s$ is the degree of rate dependence on s, and Ω is the saturation state defined by

$$\Omega = {}^{\mathbf{IAP}}/_{K_{eq}} \qquad (2)$$

In equation (2) IAP is the ion activity product, *i.e.,* ratio of products to reactant activity based on law of mass action, and $K_{eq}$ is equilibrium constant of the reaction. The saturation index, SI = log Ω , is used to quantify whether this reaction is dissolution or precipitation. If Ω < 1 the net reaction is dissolution, when Ω = 1 the net reaction is at equilibrium, otherwise the net reaction is precipitation.

It can be observed from the above two equations that a mineral surface reaction is driven by multiple parallel mechanisms. To illustrate, cuperoferrite (reactive mineral in the model) can be taken as an example to look at the final rate equation and parameters. A cuperoferrite reaction with acid is as follows:

$$\boldsymbol{CuFe_2O_4(s) + 8H^+ \overset{K_{eq,cup}}{\Longleftrightarrow} Cu^{+2} + 2Fe^{+3} + 4H_2O} \qquad (3)$$

It is assumed in this document that the rate of reaction depends on proton activity. Hence, the reaction rate equation for a cuperoferrite surface reaction can be written as follows:

$$\boldsymbol{rate = k[H^+]^{n_H}(1 - \frac{[Cu^{+2}][Fe^{+3}]^2}{[H^+]^8 K_{eq,cup}})^{\alpha}} \qquad (4)$$

The parameters used in Eq. (4) are listed in Table 1.

Table 1: Surface reaction parameters for cuproferrite used in this work.

| **K** | $n_H$ | **Log ($K_{eq,cup}$)** | $\alpha$* |
|---|---|---|---|
| Varied using Pe # | 1 | 22 | 2 |
| * Value of $\alpha$ is varied later in this work to find it has a very small effect on copper production. | | | |

For bulk homogeneous species, since the reaction rates are fast, they are assumed, in an embodiment, to reach equilibrium instantaneously.[34] Therefore, the relationships between aqueous species are found using the law of mass action. For example, the bulk reactions considering the species in Equation (3) are listed in Table 2.

Table 2: Homogeneous bulk reaction associated with Eq. 3.

| **Reactions** | **Equilibrium constant (log Keq)** |
|---|---|
| $H_2O \Leftrightarrow H^+ + OH^-$ | -14 |
| $Cu^{2+} + H_2O \Leftrightarrow Cu(OH)^+ + H^+$ | -8 |
| $Fe^{3+} + H_2O \Leftrightarrow Fe(OH)^{2+} + H^+$ | -2.19 |
| $2Fe^{3+} + 2H_2O \Leftrightarrow Fe_2(OH)_2^{4+} + 2H^+$ | -2.95 |
| $Fe^{3+} + 2H_2O \Leftrightarrow Fe(OH)_2^+ + 2H^+$ | -5.67 |

**Example: Reactive Transport Modeling**

An example implementation of step 115 of the method 110 for an illustrative copper leaching example is provided below.

In an embodiment the advection-diffusion-reaction equation is solved to model the transport of each species:

$$\frac{\partial C_i}{\partial t} = -\nabla.\left(vC_i - D_i\left(\nabla C_i + \frac{z_i F C_i}{RT}\nabla\psi\right)\right) + r_i \quad (5)$$

In equation (5) $C_i$ is the concentration of species i, $v$ is the velocity field, $z_i$ is the valency of species i, F is the Faraday's constant, R is the gas constant, T is the temperature, $\psi$ is the potential, and $r_i$ is the total reaction rate for reactions involving species i. In an embodiment, change in potential is neglected, as a first approximation, like most reactive flow simulators, but to account for accurate charge balance in the system, the Nerst-Plank equation is included together with Equation. (5). The flow velocity field in such an embodiment comes from the LBM single phase flow simulation, the rate of reactions, and the ionic diffusivities are from molecular simulations as discussed herein.

FIG. 5A illustrates a surface reaction where the reaction of acid ($H^+$) 562 flows over the mineral surface 553 and releases $Cu^{2+}$ 563a and/or $Fe^{3+}$ 563b and based on the saturation index (SI), the geometry of the mineral can be changed. In an embodiment, saturation index, SI, is used to define whether the reaction is a dissolution 551 or precipitation 552 reaction.

An embodiment combines the surface reaction illustrated in FIG. 5A with bulk homogeneous reactions. FIG. 5B illustrates the chemical reactions of this combination at the voxel level in a digital rock 3D model 554 where shading indicates grain 555a, pore 555b, and reactive mineral 555c. In such an embodiment, when the acid meets a reactive mineral 555c voxel, instead of a grain 555a voxel, visualized in FIG. 5B, the acid generates a certain amount of copper total (a mathematical construct used to make the concentration calculations easier) in the neighboring pore 555b voxels. Since bulk reactions are assumed to be in equilibrium in such an embodiment, the bulk reactions can be assumed to be a well-mixed batch reactor where copper total instantaneously transforms into copper species with different amounts based on equilibrium constant. To solve the transport and reaction equations, a global implicit scheme is used, in an embodiment, by converting non-linear equations to a system of linear equations using total concentrations 556, including primary and secondary species. The set of partial differential equations (PDEs) for mass transport (advection-diffusion-reaction) and algebraic relationships used in such an embodiment are listed herein in a further example.

FIG. 5C is a flowchart of a method 557 that summarizes a numeric implementation, according to an embodiment, of the reactive flow modeling described hereinabove in relation to FIGs. 5A-B. The workflow 557 of FIG. 5C begins at step 558 with receiving a segmented image, i.e., segmentation, of a 3D microCT image of a material of interest, e.g., porous rock. At step 559, the received image segmentation is used to determine a velocity field. To continue, at step 560 an advection-diffusion-reaction equation is solved for pore voxels next to the reactive mineral (e.g., as described above in relation to FIG. 5B) and the advection-diffusion equation is solved in the rest of the pore voxels, i.e., the pore voxels not next to the reactive mineral. Since a change in geometry due to dissolution and precipitation is not considered in such an embodiment, and short time profiles are simulated, the reaction rate for the dissolution part of the mineral reaction is considered. Further, in the workflow 557 of FIG. 5C, precipitation is suppressed by equating the precipitation rate to zero when concentration of the products starts to build up. After the transport equations are solved at step 560, the method 557 moves to step 561 where total concentrations are obtained and linear transformations as shown in algebraic speciation equations (a4 - a9) are used to get concentrations of the secondary species. Further, the method 557 may iterate 563 (i.e., repeat steps 560 and 561) until concentration of copper reaches a steady state.

According to an embodiment of the method 557, the transport equations are solved at step 560 using a finite difference method. An embodiment uses a structured uniform grid which is obtained from voxelized micro-CT images of the pore space, and the time derivative of Eq. (5) is discretized using a first-order implicit method, and advection term using an Upwind scheme.

**Example: Diffusion Modeling**

In an embodiment, MD simulations are carried out using the Forcite module in Material Studio based on a theory as detailed in Ref. 6. In such an embodiment, Forcefield parameters for atoms on Clay are modeled using CLAYFF forcefield (Ref. 12), and COMPASIII forcefield (updated with flexible water model) for water and ions (Refs. 4 and 37). The clay model in this embodiment is the generic Wyoming type montmorillonite with the unit cell formula of $Cu_{0.66}$ $[Al_{3.33}Mg_{0.66}]$ $[Si_8]$ $O_{20}[OH]_4$ having isomorphic substitution of $Al^{3+}$ with $Mg^{2+}$ in the octahedral sheet. The partial charge on the clay surface is taken from previous literature.[19,22] The simulation model of clay, according to an embodiment, includes a periodically replicated simulation cell with two montmorillonite parallel surfaces, each consisting of 45-unit cells with $Cu^{2+}$ counterions and with a separation and waters in the nano-pore like 2W waters from previous studies.[19] Further, in such an embodiment, the bulk copper chloride simulation is performed at different salt concentrations in a cubic box filled with ions and water at different concentrations and temperatures.

The clay simulation is equilibrated with NVT for 1 ns followed by 1 ns of NPT at 1 bar to relax the montmorillonite layers. Production runs for the ionic diffusion in the nano-pore clay simulation can be carried out in NVT ensemble at 298, 398, and 498 K for a 5 ns simulation and 1 fs time step with Nose barostat.[33] A leap frog algorithm can be used as an

integrator. An embodiment calculates the long-range electrostatic interactions using the particle mesh Ewald method.[15] The cutoff for Lennard-Jones (LJ) and electrostatic interactions are set to 12 Å, according to an embodiment.

An embodiment calculates self-diffusion coefficients for simple ions for reactive transport simulations from the slope of the Mean square displacement (MSD).[19] To obtain the diffusivity of a proton ($H^+$), which is known to have a more complex mechanism involving several water molecules and quantum tunneling effects called the Grotthuss mechanism,[3] a kinetically corrected MC-MD simulation can be modeled in Materials Studio,[1] or other such simulation tool known to those of skill in the art.

**Example: Partial Differential Equations (PDEs) Used In Embodiments**

$$\frac{\partial [Cu_T](r)}{\partial t} = -\nabla.\left(v(r)[Cu_T] - D_{Cu_T}(\nabla[Cu_T])\right) + rate \quad (a1)$$

$$\frac{\partial [Fe_T](r)}{\partial t} = -\nabla.\left(v(r)[Fe_T] - D_{Fe_T}(\nabla[Fe_T])\right) + 2rate \quad (a2)$$

$$\frac{\partial [H^+](r)}{\partial t} = -\nabla.\left(v(r)[H^+] - D_{H^+}(\nabla[H^+])\right) - 8rate \quad (a3)$$

while the set of algebraic speciation equations are:

$$[Cu^{2+}] = \frac{Cu_T}{(1 + \frac{k_c}{[H^+]})} \quad (a4)$$

$$[Cu(OH)^+] = \frac{\frac{k_c}{[H^+]} Cu_T}{(1 + \frac{k_c}{[H^+]})} \quad (a5)$$

$$Fe_T = [Fe^{3+}] + \frac{k_1[Fe^{3+}]}{[H^+]} + 0.5\ \frac{k_2[Fe^{3+}]^2}{[H^+]^2} + \frac{k_3[Fe^{3+}]}{[H^+]^2} \quad (a6)$$

$$[Fe(OH)^{2+}] = \frac{k_1[Fe^{3+}]}{[H^+]} \quad (a7)$$

$$[Fe(OH)_2^{4+}] = \frac{k_2[Fe^{3+}]^2}{[H^+]^2} \quad (a8)$$

$$[Fe(OH)_2^{+}] = \frac{k_3[Fe^{3+}]}{[H^+]^2} \quad (a9)$$

**Validation and Application of Embodiments**

In this section, the reactive flow methods (described herein) are applied and discussed in fundamental validation cases, followed by a discussion of applications of the methods to a case of flow through fractured rock. Each of the

following sections is based on different studied components of the reactive flow workflow with results from nano-scale molecular simulations to microscale fluid flow simulations. The results from these different sections can be combined to model copper leaching through a fracture rock or other such reactive flow process/system.

## Fundamental Validation

### Water Diffusivity

The diffusivity of water that was obtained from molecular simulations according to embodiments, was first validated. These diffusivities are listed in Table 3. The diffusivity of water was calculated using the Forcite module in BIOVIA Materials Studio® for different forcefield water models and was compared to experimental values of water diffusivity. The 4-point water model (TIP4P/2005) and flexible 3-point water model (SPC/Fw, now incorporated into COMPASIII forcefield model) were found to be more accurate than the other water forcefield models. Furthermore SPC/Fw has been shown to better predict hydrogen bonding structures and thermodynamic properties necessary to accurately model clay interfaces and, therefore, the flexible 3-point water forcefield model was used to simulate ion diffusion in clay nano-pores herein.

**Table 3.** Comparison of water diffusivity from experiments and values calculated using different water models.

| Type | D($10^{-5}$ $cm^2/s$) |
|---|---|
| Experimental value [a] | 2.3 |
| 3-point water models (SPC, TIP3P) | 4.2, 5.67 |
| 4-point water model (TIP4P/2005) | 2.1 ± 0.12 |
| COMPASIII [b] | 5.01 ± 0.49 |
| Universal [c] | 4.65 ± 0.25 |
| COMPASIII (2022 update) [d] | 2.33 ± 0.01 |

*a* Ref. 24, *b* Ref. 37, *c* Ref. 9, *d* Ref. 46.

### Calcite Dissolution

The work described herein was validated with a benchmark model for single-phase reactive flow at pore-scale.[29] This benchmark case did not include any multicomponent reaction or geometry evolution. This was intended to validate the implementation of reactive flow and its boundary conditions. A simple 2D geometry of a calcite pellet placed in a rectangular channel was simulated as presented in Ref. 29 with simulation parameters taken from the same reference. The concentration profile 662 with a diffusive boundary layer around the calcite mineral is shown in FIG. 6A. In the concentration profile 662, concentration is illustrated in accordance with the key 669. Plot 661 shows average outlet acid concentration (in mol/cc) 666b as a function of time (in seconds) 666a for multiple reactive flow simulation methods 665a-f. The plot 661 compares the concentration profile of $H^+$ from the simulation results 665f of an embodiment to results obtained using other reactive flow pore scale simulations 665a-e, namely, Vortex 665a, Dissolvefoam 665b, Lattice Boltzmann 665c, OpenFOAM DBS 665d, and Chombo-crunch 665e, on the same geometry. Since the geometry was not evolving with reaction in this test, only results from short simulation times (time to reach steady state is of the order of 3 seconds[29]) were compared. Short time average outlet concentration profile from the simulation matched well with the results in Ref. 29 which validates the reactive flow embodiments used herein. As shown in FIG. 6A the steady state concentration profile 662 around the calcite pellet had a boundary layer in the form of a tail with concentration of $H^+$ lower at the surface. The lines for Dissolvefoam 665b and OpenFOAM DBS 665d approximately overlap.

FIG. 6B is a plot 663 that illustrates the boundary effects of reactions on surfaces. Plot 663 illustrates this effect by showing average spatial concentration of $H^+$ 667b versus width 667a of the geometry. The plot 663 shows results from GeoChemFoam code 668a and results 668b from embodiments. The spatial concentration profile 664 from the simulation was compared to results from reactiveTransportALEfoam (as shown in FIG. 6C), an openFOAM library shared as part of GeoChemFoam code with the concentration differences 670a-c observed.[28] Agreement between codes was good, especially the thickness of the diffuse layer and the rate of $H^+$ consumption.

### Application To Copper Leaching

Embodiments have been validated for modeling copper leaching in fractured rock microstructures where the rock

is modeled as a cube with side length 300 μm. An example model 770 is shown in FIG. 7A. In the model 770 the white region 771 in the rock is the reactive mineral, the lighter gray region 772 in the rock is the non-reactive mineral, the darker gray region 773 in the rock is the micro-porous mineral, and the black region 774 in the rock is the pore. To validate copper leaching, a simulation was implemented using the model 770 where acid 775 is injected at one end of the rock 770 and a copper solution 776 is extracted at the outlet of the rock 770. The rock 770 was segmented into four regions, where cuperoferrite was used as the reactive mineral 771, smectite was considered for the nano-porous mineral 773, solid rock was considered for the non-reactive mineral 772, and void is considered for the pore 774. Molecular simulations are described further herein to obtain diffusivities inside the clay nano-porous mineral 773 and pores (fractures) 774 of the digital rock 770, followed by results from reactive flow simulations to show the influence of inlet flow 775 conditions and reaction types on copper production/extraction 776. FIG. 7B depicts a comparison of the outlet concentration 778a of the copper production/extraction 776 from FIG. 7A over the course of time (seconds) 778b. The outlet concentration 778a is computed for a given value of pH = 2 779 and plotted 777 as shown in FIG. 7B, where after about 0.2 seconds the concentration plateaued.

## Ion Diffusivity In Clay Nano-Pore

To get the diffusivity values needed as part of reactive flow calculations through the open fracture and the nano-porous clay material, MD simulations of a bulk salt solution (represented by the model 880 shown in FIG. 8A) were employed, as were salt solutions in the clay interlayer nano-pore (represented by the model 884 shown in FIG. 8B). The bulk salt solution model 880 includes Copper ions 881, Chloride ions 882, and water molecules 883 that are represented by tightly packed spheres in the bulk salt solution. Clay nano-pores 885a-b shown in FIG. 8B are generic Wyoming type montmorillonite taken from the literature.[19] In FIG. 8B, Copper ions 886, Chloride ions 887, and water molecules 888 are all indicated by spheres in between the montmorillonite layers 885a-b. FIG. 8B also includes atomic density profiles 889 along the clay pore for water oxygen 893a, copper 893c, and chloride 893b. The density of water oxygen 893a, copper 893c, and chloride 893b inside the clay nano-pores as shown in the plot 889 oscillates. The oscillations in density are a standard feature arising from finite size effects near the nano-pore walls.[7] Simulations were performed as a function of temperature and salt concentration to understand the dependence of diffusivity on these external factors as done in many previous studies on clay nano-pores.[19,22,36]

FIG. 8C is a plot 890 showing diffusivity values 896a of copper versus concentration 895a as a function of temperature, namely 498 K 894a, 398 K 894b, and 298 894c. FIG. 8D is a plot 891 showing diffusivity values 896b of chloride in bulk liquid water versus salt concentration 895b as a function of temperature, namely 498 K 894d, 398 K 894e, and 298 894f. The plots 890 and 891 show that diffusivity 896a-b of both ions increased with temperature, which was expected, but reduced with increased salt concentration 895a-b. This reduction in diffusivity with increased concentration change was due to increased ion-ion clustering at high salt concentrations, which reduced the mobility of each ion.

Similarly, FIG. 8E is a plot 892 showing diffusivity values 897 of copper 899a and chloride 899b inside a clay nano-pore model at different temperatures 898. Several previous studies have reported diffusivity of simple ions like $Na^+$ inside clay nano-pores and the work described herein is an extension of that to other ions of importance for copper leaching. FIG. 8E shows that the diffusivity 897 of copper ions 899a in clay is two orders of magnitude slower than the values in bulk water which was expected because of increased interaction with the charged clay surface.

## Copper Leaching By Acid Injection In A Fractured Rock Model

FIG. 9 depicts views 990a-c of a 3D microstructure model of an example rock with cupperoferrite mineral and fractures. The view 990a depicts the entire 3D rock model 994, while the view 990b illustrates an upper portion 995 of the model 994 and the view 990c illustrates a lower portion 996 of the model 994. FIG. 9 also includes a scale illustrating copper concentration 993a-c as depicted in the views 990a-c. Using the model 994, the injection of acid in a rock microstructure was simulated to demonstrate the capability of embodiments to handle multispecies reactive transport in a complex 3D microstructure. In this section, the reactive flow simulation case is described along with several sensitivity studies, that utilized different flow and reaction conditions, using dimensionless numbers. The effects of having only heterogeneous surface reactions and adding multispecies homogeneous reactions are also discussed. Finally, flow through porous media is introduced to model the multiscale reactive flow through both fracture and porous media and compare it to flow through only the fracture. Simulation results are discussed by examining concentration profiles in the 3D rock to present outlet concentrations of acid, and composition of the outlet solution.

To visualize the flow pattern and reaction, the 3D steady state concentration profile of copper in the fracture 992 is utilized. Copper plumes arise on the white mineral because it is created by acid reacting with the cupperoferrite mineral 991a (represented by white mineral in the 3D model). The copper then advects and diffuses to the rest of the fracture 992 due to flow and concentration gradients of copper. High concentrations 993a are shown close to reactive mineral and low concentrations 993c in the rest of the geometry flowing over the light gray 991c and dark gray 991b minerals.

FIGs. 10A-B illustrate plots 1010 and 1011, respectively, showing results from sensitivity studies. Specifically, the plot 1010 illustrates a sensitivity study that utilized different Peclet 1012a (Pe, shown on x-axis) and Damkohler 1012b (Da, shown on y-axis) numbers to understand the effect of inlet velocity and surface reactivity of mineral on copper production 1012c (outlet concentration, shown on z-axis). The plot 1010 in FIG. 10A shows that the effect of Damkohler number 1012b on copper production 1012c was larger than Peclet 1012a, since Damkohler 1012b represented the effect of increasing the rate of the forward reaction to produce more copper as illustrated by the gradient 1013 in the plot 1010.

The contour plot 1011 of FIG. 10B was used to determine values for Damkohler 1014b and Peclet 1014a that would be held constant in future experiments as shown in FIG. 11A and FIG. 11B. The contour plot 1011 plots Damkohler 1014b versus Peclet 1014a where each series corresponds to a respective concentration of copper. For the results described in the following sections a Peclet 1014a number of 10 and a Damkohler 1014b number of 2.5 were used as constants. The dashed lines 1015a-b are the values chosen for reaction rate and velocity for further analysis. The dashed lines 1015a-b shown in FIG. 10B were used to further visualize the steady state concentration profiles of copper in FIGs. 11A-D, while varying only one parameter at the time.

FIGs. 11A-D are plots 1110, 1111, 1112, and 1113, respectively, showing sensitivity study results as a function of time in order to capture transient behavior to steady state.

FIG. 11A is a plot 1110 of outlet concentration 1114a versus time 1115a. The plot 1110 includes a profile 1117a-g for respective Damkohler numbers 10, 2.5, 0.5, 0.125, 0.025, 0.005, and 0.001. The plot 1110 illustrates that each concentration 1114a profile 1117a-g reached steady state at the same time based on the dashed line 1116 representing a Peclet number of 10, but the steady state value for concentration of copper 1114a increased with increasing Damkohler number 1117a-g. The lines representing 0.005 Da 1117f and 0.001 Da 1117g are approximately overlapping.

FIG. 11B is a plot 1111 of outlet concentration 1114b versus time 1115b. The plot 1111 includes series 1118a-e for respective Peclet numbers 500, 250, 50, 10, and 2.5. The lines representing 500 Pe 1118a and 250 Pe 1118b are approximately overlapping.

FIG. 11C is a plot 1112 of steady state concentration 1120 versus Damkohler number 1121. The plot 1112 shows that at a given inlet velocity, when the surface reactivity was increased, more copper was produced. This can be seen in FIG. 11C, which shows the steady state concentration of copper 1120 at the outlet compared to the Damkohler number. When the Peclet number 1118a-e was changed at a Damkohler number of 2.5 as shown by the dashed line 1119 of FIG. 11B, the time to reach steady state for the concentration profile changed as shown in FIG. 11B.When the Peclet number 1118a-e was changed at a Damkohler number of 2.5 as shown by the dashed line 1119 of FIG. 11B, the time to reach steady state for the concentration profile changed in the comparison plot 1111, as shown in FIG. 11B.

FIG. 11D is a plot 1113 of steady state concentration 1122 versus Peclet number 1123. The plot 1113 shows that the steady state value 1122 for copper at the outlet seems to have a dependency on Peclet number 1123 such that low velocities corresponds to low concentrations, while at high velocities an asymptotic value for concentration is approached. Moving beyond the sensitivity to each individual parameter shown in FIGs. 11A-D, which correspond to the dashed lines 1015a and 1015b in FIG. 10B, it can also be observed that while changing reactivity increased overall copper production, there also seemed to be some limitation imposed by the microstructure that restricts additional copper production if there is still a high copper concentration around the cuproferrite locations. This meant that unless the velocity is also increased for removing copper from the system, increasing the reactivity is not beneficial. This interesting observation shows the role of the microstructure connectivity in the balance between reactivity and transport.

## Incorporation Of Homogeneous Bulk Reaction

In this section, the interplay between competitive homogeneous bulk reactions and surface reactions on the mineral surface in a 3D pore space of the fractured rock are examined. The homogeneous bulk reactions used in this model are shown in Table 2. When these bulk reactions are added to the system of equations, the effect of speciation and pH on the outlet copper production can be understood.

FIG. 12A is a bar chart 1220 showing outlet concentrations 1222 of copper species 1224 and 1225 as a function of inlet acidity 1223a-f, specifically, at pH of 0, 1, 2, 3, 4, and 8. Similarly, FIG. 12B is a bar chart 1221 showing outlet concentrations 1226 of iron species 1228a-d as a function of inlet acidity 1227a-f, specifically, at pHs of 0, 1, 2, 3, 4, and 8.. One observation from FIGs. 12A-B is that the product concentrations at the outlet decreased with increasing pH, which is due to the fact that the production of copper 1224 and 1225, and iron 1228a-d is mainly controlled by the surface reactions, which need high acid concentration (low pH) for better reactivity. Competitive reactions in the bulk produce secondary copper and iron species, *e.g.,* the dominant species for copper and iron change with inlet pH conditions. This is an effect that needs to be properly captured in any reactive flow simulator, otherwise biased estimations of primary species concentrations could be obtained. This embodiment contains a simple subset of homogeneous bulk reactions, but in a real-world application, with several competitive surface and bulk reactions combined with a large size of the porous rock, this issue of speciation will have a significant influence on the product concentrations. Therefore, the selected value for the inlet acidic concentration and species considered in the model of the aqueous solution are very important to optimize any

leaching application.

To continue, the effect of competitive bulk reactions on the primary species of copper ion ($Cu^{2+}$) production was examined and results are shown in FIGs. 13A-C. FIG. 13A is a plot 1330 showing outlet concentration 1331, for heterogeneous reactions, versus time 1332 for multiple different pHs 1333a-f, specifically, at pHs of 0, 1, 2, 3, 4, and 8. FIG. 13B is a plot 1334 showing outlet concentration 1335, for homogenous and heterogeneous reactions, versus time 1336 for multiple different pHs 1337a-f, specifically, at pHs of 0, 1, 2, 3, 4, and 8. FIG. 13C is a plot 1340 of steady state concentration 1341 versus acid concentration 1342. The plot 1340 includes a series for heterogeneous and homogeneous reactions 1343 and a series for only heterogeneous reactions 1344.

Comparing the plots 1330 (FIG. 13A) and 1334 (FIG. 13B) shows that concentration of copper ions at the outlet (1331 and 1335, FIGs. 13A and 13B, respectively) decreased as the inlet pH conditions (1333a-f and 1337a-f, FIGs. 13A and 13B, respectively) increased due to the reduction in surface reactivity for only heterogeneous reactions 1330 compared to homogeneous and heterogeneous reactions 1331. Interestingly, when competitive bulk reactions were added, the copper ion production was higher than when only surface reactions were present (as shown in plot 1340 of FIG. 13C). Plot 1340 comparing heterogeneous and homogenous reactions 1343 to only heterogeneous reactions 1344 based on the steady state concentration 1341 and the acid concentration 1342 shows that the competitive bulk reactions consume products from the surface reaction ($Cu^{2+}$) and hence move the reaction forward. Thus, having multispecies bulk reaction has an important effect on the composition and quantities of the products at the outlet, and is helpful to accurately model the copper leaching problem.

## Copper Leaching By Acid Injection In Fractured Rock And Clay - Multiscale

The possibility of flow through fracture and nano-porous clay, *e.g.,* the multiscale reactive transport problem, was also studied. Transport through nano-porous materials (clay, in this embodiment) happened mostly due to diffusion of species through the nano-porous regions segmented in the 3D microstructure model. These diffusivities were estimated using MD simulations as presented herein.

FIG. 14A shows a cross-section of a digital rock fractured model 1440 with flow only through fracture 1444. The cross section 1440 indicates material phases using shading where shading 1442a, 1442b, and 1442c represent reactive mineral, non-reactive mineral, and micro-porous mineral, respectively. FIG. 14A also includes a legend 1443 whereby copper concentration is indicated based on shading. FIG. 14A shows that copper concentration was high next to the reactive mineral 1442a. The copper concentration is lower next to the non-reactive mineral 1442b and the micro-porous mineral 1442c.

FIG. 14B shows a cross-section of a digital rock fractured model 1441 with flow through fracture 1445 and nano-porous clay 1446c. The cross section 1441 indicates material phases using shading where shading 1446a, 1446b, and 1446c represent reactive mineral, non-reactive mineral, and nano-porous clay, respectively. FIG. 14B also includes a legend 1448 whereby copper concentration is indicated based on shading. Multiscale flow through the fracture 1445 and the nano-porous clay regions 1446c shows that copper started to penetrate through the dark gray region 1446c, e.g., at location 1447, which represents the nano-porous clay in the model. Incorporating flow through nano-porous clay helped reach other reactive minerals 1446a that were not in direct contact with the fracture 1445, which led to an increased estimation in copper production. The copper concentration around the non-reactive mineral 1446b still remains relatively lower compared to the nano-porous clay and reactive mineral.

FIG. 14C shows a plot 1449 of the outlet copper concentration 1450 as a function of time 1451. The plot 1449 includes a series 1452 for single scale reactive flow (e.g., as shown in FIG. 14A) and a series 1453 for multi-scale reactive flow (e.g., as shown in FIG. 14B). It can be observed in plot 1449 that it takes relatively longer for multiscale flow 1453 to reach a steady state because the transport through porous media is relatively slower. An interesting observation from FIG. 14C was that over time, more copper was produced than when a single scale reactive flow 1452 model was used. This means that incorporating a multiscale flow model through nano-porous materials, as in embodiments, plays an important role in improving the accuracy of estimations of reactive flow production models of copper leaching.

An example digital rock model 1550 that may be utilized in embodiments is depicted in FIG. 15. FIG. 15 includes zoomed in view 1551 showing copper leaching 1551 in the fractured middle portion of the rock 1552 through a pore space (fractures in a clay material), surrounded by the reactive mineral 1553a, micro-porous mineral 1552c, and non-reactive mineral 1553b. FIG. 15 also includes a zoomed in view 1554 of clay that is modeled with Wyoming type montmorillonite above and below the digital rock as a two layers 1557a-b, with copper ions 1558, chloride ions 1559, and water molecules 1560 between the two layers 1557a-b. Further still, FIG. 15 includes a view 1555 of a fluid in a fracture that is modeled as bulk molecular fluid containing copper ions 1561, chloride ions 1562, and water molecules 1563.

## Example Method Workflow

Described herein are computer-implemented methods of determining behavior of reactive flow systems.

Embodiments provide a workflow for reactive transport simulation with single scale and multiscale flow with multispecies reactions. In an embodiment, the flow velocity field for both single scale and multiscale flow is taken from LBM single phase flow simulations and combined with molecular dynamics simulation for ion diffusivity and reactions from available databases in a multiscale reactive flow simulation. The reactive transport model in embodiments was validated with a short time simulation of calcite dissolution and results were compared to published results of other reactive flow codes. The molecular simulation part of embodiments was also validated with the estimation of water diffusivity and compared to other available experimental values.

The multiscale reactive flow workflow was systematically used to explore the importance of surface reactions, competitive bulk reactions, pH, and flow through porous media combined with sensitivity on both transport and reaction parameters. In all these results, the outlet copper ions concentration was analyzed, which is the product from copper leaching, and the composition of the product stream was explored when homogeneous bulk reactions were included in addition to the heterogeneous surface dissolution reactions. Together with the outlet concentration, the 3D concentration profile in the digital rock model was validated to illustrate the impact of both transport and reactivity as well as the microstructure connectivity.

Through these simulation results, it was observed that it is important to include homogeneous bulk reactions (as they have a significant effect on the composition of outlet stream) together with surface reactions to accurately model the concentration of copper ions produced from copper leaching. It was also observed that pH has a significant impact on the chemical equilibrium of the aqueous solution and leads to different relative fractions on species composition of the outlet stream. Finally, a multiscale flow through porous materials was included, which modeled the nano-porous clay in the rock. This multiscale extension had an important effect on the outlet copper ion concentration since more reactive minerals could be reached that were not originally in direct contact with the fracture. Incorporating multiscale flow through fracture and nano-porous clay was shown to have a significant effect on the prediction of copper ion production from copper leaching.

Embodiments provide a robust workflow for multiscale reactive flow in 3D microstructure models where reactions and fluid transport are combined using molecular dynamics simulations, LBM fluid flow simulations, and reactive transport simulations. Further, embodiments provide a simple and effective way to include bulk homogeneous reactions in addition to surface heterogeneous reactions into a reactive flow simulation. This has an important effect on copper ion production in the outlet stream. Further still, embodiments can simulate a multiscale flow through fracture and nano-porous materials combined with homogeneous and heterogeneous reactions to accurately model the reactive flow through a digital rock model.

The accurate results of embodiments can be used for various reactive flow real-world applications. For example, embodiments can be used to operate/control reactive flow systems, such as copper leaching applications, amongst other examples. In one such example implementation, embodiments can be utilized to determine optimized operating parameters for a copper leaching application and the real-world system can, in turn, be controlled to operate in accordance with the determined optimized parameters. Another example is the reactive flow behavior of CO2 dissolved in water flowing through concrete, a porous material, and reacting with the mineral cement solid component.

The workflows for reactive flow modeling described herein combine the below capabilities to accurately model reactive flow simulations: (1) Accurate microstructure models based on 3D imaging (microCT), (2) Multiscale flow through resolvable and unresolvable pore(s), (3) Multiphase flow simulator to solve combined gas and liquid simulation, (4) Heterogeneous and homogeneous reactions into reactive flow model, (5) Upscaling from pore scale flow simulation to model field scale reactive flow simulation, (6) Molecular simulations to estimate material, reaction and diffusion properties required to model reactive flow, (7) A robust workflow for multiphase, multiscale and multispecies reactive flow in 3D microstructure models where reactions and fluid transport are combined using molecular dynamics simulations, LBM fluid flow simulations, and reactive transport simulations, (8) An effective way to include bulk homogeneous reactions in addition to surface heterogeneous reactions into a reactive flow simulation. This has an important effect on the determination of species production in the outlet stream, as shown in the embodiments, (9) A multiphase flow through resolvable pore and unresolved-pore materials combined with homogeneous and heterogeneous reactions to accurately model the reactive flow through a digital rock model, (10) Implementing embodiments in the Dassault Systemes' 3DEXPERIENCE® platform enables optimization of multiphase, multiscale, and multispecies reactive transport models, so that the models can be scaled up for field scale simulations in industries such as mining, oil and gas, energy storage technologies, chemical industry, and material corrosion, *etc.*

<u>**Computer Support**</u>

FIG. 16 is a simplified block diagram of a computer-based system 2020 that may be used to determine behavior of a reactive flow system according to any variety of embodiments described herein. The system 2020 comprises a bus 2023. The bus 2023 serves as an interconnect between the various components of the system 2020. Connected to the bus 2023 is an input/output device interface 2026 for connecting various input and output devices such as a keyboard, mouse,

display, speakers, etc. to the system 2020. A central processing unit (CPU) 2022 is connected to the bus 2023 and provides for the execution of computer instructions implementing embodiments. Memory 2025 provides volatile storage for data used for conducting computer instructions implementing embodiments described herein, e.g., method 110. Storage 2024 provides non-volatile storage for software instructions, such as an operating system (not shown) and embodiment configurations, etc. The system 2020 also comprises a network interface 2021 for connecting to any variety of networks known in the art, including wide area networks (WANs) and local area networks (LANs).

It should be understood that the example embodiments described herein may be implemented in many different ways. In some instances, the various methods and machines described herein may each be implemented by a physical, virtual, or hybrid general purpose computer, such as the computer system 2020, or a computer network environment such as the computer environment 2120, described herein below in relation to FIG. 17. The computer system 2020 may be transformed into the machines that execute the methods described herein, for example, by loading software instructions into either memory 2025 or non-volatile storage 2024 for execution by the CPU 2022. One of ordinary skill in the art should further understand that the system 2020 and its various components may be configured to carry out any embodiments or combination of embodiments described herein. Further, the system 2020 may implement the various embodiments described herein utilizing any combination of hardware, software, and firmware modules operatively coupled, internally, or externally, to the system 2020.

FIG. 17 illustrates a computer network environment 2120 in which an embodiment of the present invention may be implemented. In the computer network environment 2120, the server 2121 is linked through the communications network 2122 to the clients 2123a-n. The environment 2120 may be used to allow the clients 2123a-n, alone or in combination with the server 2121, to execute any of the methods described herein. For nonlimiting example, computer network environment 2120 provides cloud computing embodiments, software as a service (SAAS) embodiments, and the like.

Embodiments or aspects thereof may be implemented in the form of hardware, firmware, or software. If implemented in software, the software may be stored on any non-transient computer readable medium that is configured to enable a processor to load the software or subsets of instructions thereof. The processor then executes the instructions and is configured to operate or cause an apparatus to operate in a manner as described herein.

Further, firmware, software, routines, or instructions may be described herein as performing certain actions and/or functions of the data processors. However, it should be appreciated that such descriptions contained herein are merely for convenience and that such actions in fact result from computing devices, processors, controllers, or other devices executing the firmware, software, routines, instructions, etc.

It should be understood that the flow diagrams, block diagrams, and network diagrams may include more or fewer elements, be arranged differently, or be represented differently. But it further should be understood that certain implementations may dictate the block and network diagrams and the number of block and network diagrams illustrating the execution of the embodiments be implemented in a particular way.

Accordingly, further embodiments may also be implemented in a variety of computer architectures, physical, virtual, cloud computers, and/or some combination thereof, and thus, the data processors described herein are intended for purposes of illustration only and not as a limitation of the embodiments.

While example embodiments have been particularly shown and described, it will be understood by those skilled in the art that various changes in form and details may be made therein without departing from the scope of the embodiments encompassed by the appended claims.

For example, the foregoing description and details of embodiments in the figures reference Applicant-Assignee (Dassault Systemes Americas Corporation) and Dassault Systemes, tools and platforms, for purposes of illustration and not limitation. Other similar tools and platforms are suitable.

## References

1. Abbott, J.W., Hanke, F., 2022. Kinetically Corrected Monte Carlo-Molecular Dynamics Simulations of Solid Electrolyte Interphase Growth. J. Chem. Theory Comput. 18, 925-934. https://doi.org/10.1021/acs.jctc.1c00921
2. Abd, A.S., Abushaikha, A.S., 2021. Reactive transport in porous media: a review of recent mathematical efforts in modeling geochemical reactions in petroleum subsurface reservoirs. SN Appl. Sci. 3, 1-28. https://doi.org/10.1007/s42452-021-04396-9
3. Agmon, N., 1995. CHEMICAL PHYSICS The Grotthuss mechanism. Chem. Phys. Lett. 50, 456-462.
4. Akkermans, R. L. C., Spenley, N. A., Robertson, S. H. 2021. COMPASS III: automated fitting workflows and extension to ionic liquids. Molecular Simulation, 47, 540-551
5. Al-Khulaifi, Y., Lin, Q., Blunt, M.J., Bijeljic, B., 2017. Reaction Rates in Chemically Heterogeneous Rock: Coupled Impact of Structure and Flow Properties Studied by X-ray Microtomography. Environ. Sci. Technol. 51, 4108-4116.

https://doi.org/10.1021/acs.est.6b06224

6. Allen, M. P., Tildesley, D. J. 1987. Computer Simulation of Liquids; Clarendon Press, Oxford Science Publications

7. Beckstein, O., Sansom, M.S.P., 2003. Liquid-vapor oscillations of water in hydrophobic nanopores. Proc. Natl. Acad. Sci. U. S. A. 100, 7063-7068. https://doi.org/10.1073/pnas.1136844100

8. Boek, E.S., 2014. Molecular dynamics simulations of interlayer structure and mobility in hydrated Li-, Na- and K-montmorillonite clays. Mol. Phys. 112, 1472-1483. https://doi.org/10.1080/00268976.2014.907630

9. Casewit, C.J., Colwell, K.S., Rappé, A.K., 1992. Application of a Universal Force Field to Main Group Compounds. J. Am. Chem. Soc. 114, 10046-10053. https://doi.org/10.1021/ja00051a042

10. Chatterjee A., Ebina T., Mizukami F., 2005. Effects of Water on the Structure and Bonding of Resorcinol in the Interlayer of Montmorillonite Nanocomposite: A Periodic First Principle Study. Phys. Chem. B, 109, 15, 7306-7313 https://doi.org/10.1021/jp045775z

11. Chen, L., He, Y.L., Tao, W.Q., Zelenay, P., Mukundan, R., Kang, Q., 2017. Pore-scale study of multiphase reactive transport in fibrous electrodes of vanadium redox flow batteries. Electrochim. Acta 248, 425-439.https://doi.org/10.1016/j.electacta.2017.07.086

12. Cygan, R.T., Liang, J.J., Kalinichev, A.G., 2004. Molecular models of hydroxide, oxyhydroxide, and clay phases and the development of a general force field. J. Phys. Chem. B 108, 1255-1266. https://doi.org/10.1021/jp0363287

13. Damiani, L.H., Kosakowski, G., Glaus, M.A., Churakov, S. V., 2020. A framework for reactive transport modeling using FEniCS-Reaktoro: governing equations and benchmarking results. Comput. Geosci. 24, 1071-1085. https://doi.org/10.1007/s10596-019-09919-3

14. Dooley, J.J., Dahowski, R.T., Davidson, C.L., Wise, M.A., Gupta, N., Kim, S.H., Malone, E.L., 2006. Carbon dioxide capture and geologic storage-A core element of a global energy technology strategy to address climate change. Jt. Glob. Chang. Res. Inst. 1-37.

15. Essmann, U., Perera, L., Berkowitz, M.L., Darden, T., Lee, H., Pedersen, L.G., 1995. A smooth particle mesh Ewald method. J. Chem. Phys. 103, 8577-8593. https://doi.org/10.1063/1.470117

16. Fager A., Crouse B., Sun G., Xu R., Freed D., 2019. " Evaluation of Directly Simulated WAG Hysteresis at Pore Scale and its Effect on Injectivity Index" SPE 195734, SPE Offshore Europe Conf. & Exhib., Aberdeen, UK

17. Fager A., Otomo H., Salazar-Tio R., Balasubramanian G., Crouse B., Zhang R., Chen H., Schembre-McCabe J., 2021. "Multi-scale Digital Rock: Application of a multi-scale multi-phase workflow to a Carbonate reservoir rock", Int. Symp. Soc. Core Analysts

18. Freed, D. 1998. "Lattice-Boltzmann Method for Macroscopic Porous Media Modeling." International Journal of Modern Physics C 09 (08): 1491-1503. https://doi.org/10.1142/S0129183198001357

19. Holmboe, M., Bourg, I.C., 2014. Molecular dynamics simulations of water and sodium diffusion in smectite interlayer nanopores as a function of pore size and temperature. J. Phys. Chem. C 118, 1001-1013. https://doi.org/10.1021/jp408884g

20. Jerauld, G., Fredrich J., Lane N., Sheng Q., Crouse B., Freed D., Fager A., and Xu R.. 2017. "Validation of a Workflow for Digitally Measuring Relative Permeability." In. Society of Petroleum Engineers. https://doi.org/10.2118/188688-MS

21. Kang, Q., Lichtner, P.C., Zhang, D., 2007. An improved lattice Boltzmann model for multicomponent reactive transport in porous media at the pore scale. Water Resour. Res. 43, 1-12. https://doi.org/10.1029/2006WR005551

22. Kosakowski, G., Churakov, S. V., Thoenen, T., 2008. Diffusion of Na and Cs in montmorillonite. Clays Clay Miner. 56, 190-206.https://doi.org/10.1346/CCMN.2008.0560205

23. Krevor, S., Blunt, M.J., Benson, S.M., Pentland, C.H., Reynolds, C., Al-Menhali, A., Niu, B., 2015. Capillary trapping for geologic carbon dioxide storage - From pore scale physics to field scale implications. Int. J. Greenh. Gas Control 40, 221-237. https://doi.org/10.1016/j.ijggc.2015.04.006

24. Krynicki, K., Green, C.D., Sawyer, D.W., 1978. Pressure and temperature dependence of self-diffusion in water. Faraday Discuss. Chem. Soc. 66, 199-208. https://doi.org/10.1039/DC9786600199

25. Li, L., Peters, C.A., Celia, M.A., 2006. Upscaling geochemical reaction rates using pore-scale network modeling. Adv. Water Resour. 29, 1351-1370. https://doi.org/10.1016/j.advwatres.2005.10.011

26. Mackay, E.J., 2013. Modelling the injectivity, migration and trapping of CO2 in carbon capture and storage (CCS), Geological Storage of Carbon Dioxide (co2): Geoscience, Technologies, Environmental Aspects and Legal Frameworks. Woodhead Publishing Limited. https://doi.org/10.1533/9780857097279.1.45

27. MacQuarrie, K.T.B., Mayer, K.U., 2005. Reactive transport modeling in fractured rock: A state-of-the-science review. Earth-Science Rev. 72, 189-227. https://doi.org/10.1016/j.earscirev.2005.07.003

28. Maes, J., Menke, H.P., 2021. GeoChemFoam: Direct Modelling of Multiphase Reactive Transport in Real Pore Geometries with Equilibrium Reactions. Transp. Porous Media 139, 271-299. https://doi.org/10.1007/s11242-021-01661-8

29. Molins, S., Soulaine, C., Prasianakis, N.I., Abbasi, A., Poncet, P., Ladd, A.J.C., Starchenko, V., Roman, S., Trebotich, D., Tchelepi, H.A., Steefel, C.I., 2021. Simulation of mineral dissolution at the pore scale with evolving fluid-solid interfaces: review of approaches and benchmark problem set. Comput. Geosci. 25, 1285-1318. https://doi.org/10.1007/s10596-019-09903-x

30. Noiriel, C., 2015. Resolving Time-dependent Evolution of Pore-Scale Structure, Permeability and Reactivity using X-ray Microtomography. Rev. Mineral. Geochemistry 80, 247-285.

31. Noiriel, C., Daval, D., 2017. Pore-Scale Geochemical Reactivity Associated with CO2 Storage: New Frontiers at the Fluid-Solid Interface. Acc. Chem. Res. 50, 759-768. https://doi.org/10.1021/acs.accounts.7b00019

32. Norgate, T.E., Jahanshahi, S., Rankin, W.J., 2007. Assessing the environmental impact of metal production processes. J. Clean. Prod. 15, 838-848. https://doi.org/10.1016/j.jclepro.2006.06.018

33. Nosé, S. 1984. A unified formulation of the constant temperature molecular-dynamics methods. Journal of Chemical Physics. 81 (1): 511-519.Bibcode:1984JChPh..81..511N. doi:10.1063/1.447334.)

34. Oliveira, T.D.S., Blunt, M.J., Bijeljic, B., 2019. Modelling of multispecies reactive transport on pore-space images. Adv. Water Resour. 127, 192-208. https://doi.org/10.1016/j.advwatres.2019.03.012

35. Parkhurst, D.L., Wissmeier, L., 2015. PhreeqcRM: A reaction module for transport simulators based on the geochemical model PHREEQC. Adv. Water Resour. 83, 176-189. https://doi.org/10.1016/j.advwatres.2015.06.001

36. Rahromostaqim, M., Sahimi, M., 2020. Molecular Dynamics Study of the Effect of Layer Charge and Interlayer Cations on Swelling of Mixed-Layer Chlorite-Montmorillonite Clays. J. Phys. Chem. C 124, 2553-2561.https://doi.org/10.1021/acs.jpcc.9b10919

37. Rigby, D., 2004. Fluid density predictions using the COMPASS force field. Fluid Phase Equilib. 217, 77-87. https://doi.org/10.1016/j.fluid.2003.08.019

38. Sinclair, L., Thompson, J., 2015. In situ leaching of copper: Challenges and future prospects. Hydrometallurgy 306-324.

39. Smith, D.E., 1998. Molecular computer simulations of the swelling properties and interlayer structure of cesium montmorillonite. Langmuir 14, 5959-5967. https://doi.org/10.1021/la980015z

40. Sposito, G., Skipper, N.T., Sutton, R., Park, S.H., Soper, A.K., Greathouse, J.A., 1999. Surface geochemistry of the clay minerals. Proc. Natl. Acad. Sci. U. S. A. 96, 3358-3364. https://doi.org/10.1073/pnas.96.7.3358

41. Sridhar, N., 2017. Local corrosion chemistry - A review. Corrosion 73, 18-30. https://doi.org/10.5006/2246

42. Steefel, C.I., Appelo, C.A.J., Arora, B., Jacques, D., Kalbacher, T., Kolditz, O., Lagneau, V., Lichtner, P.C., Mayer, K.U., Meeussen, J.C.L., Molins, S., Moulton, D., Shao, H., Šimůnek, J., Spycher, N., Yabusaki, S.B., Yeh, G.T., 2015. Reactive transport codes for subsurface environmental simulation, Computational Geosciences. https://doi.org/10.1007/s10596-014-9443-x

43. Steefel, C.I., DePaolo, D.J., Lichtner, P.C., 2005. Reactive transport modeling: An essential tool and a new research approach for the Earth sciences. Earth Planet. Sci. Lett. 240, 539-558. https://doi.org/10.1016/j.epsl.2005.09.017

44. Sun G., Sun Z., Fager A., Crouse B., 2022. "Pore-scale Analysis of CO2-brine Displacement in Berea Sandstone and Its Implications to CO2 Injectivity", Int. Symp. Soc. Core Analysts

45. Teich-McGoldrick, S.L., Greathouse, J.A., Jové-Colón, C.F., Cygan, R.T., 2015. Swelling Properties of Montmorillonite and Beidellite Clay Minerals from Molecular Simulation: Comparison of Temperature, Interlayer Cation, and Charge Location Effects. J. Phys. Chem. C 119, 20880-20891. https://doi.org/10.1021/acs.jpcc.5b03253

46. Wu, Y., Tepper, H.L., Voth, G.A., 2006. Flexible simple point-charge water model with improved liquid-state properties. J. Chem. Phys. 124.https://doi.org/10.1063/1.2136877

47. Xu, T., Sonnenthal, E., Spycher, N., Pruess, K., 2006. TOUGHREACT - A simulation program for non-isothermal multiphase reactive geochemical transport in variably saturated geologic media: Applications to geothermal injectivity and CO2 geological sequestration. Comput. Geosci. 32, 145-165. https://doi.org/10.1016/j.cageo.2005.06.014

48. Xu, R., Crouse B., Freed D., Fager A., Jerauld G., Lane N., and Sheng Q.. 2018. "Continuous vs Discontinuous Capillary Desaturation and Implications for IOR/EOR", SCA 2018-066, Int. Symp. Soc. Core Analysts, Trondheim, Norway

49. Yoon, H., Kang, Q., Valocchi, A.J., 2015. Lattice Boltzmann-based approaches for pore-scale reactive transport. Pore Scale Geochemical Process. 80, 393-431. https://doi.org/10.2138/rmg.2015.80.12

## Claims

1. A computer-implemented method of determining behavior of a reactive flow system that includes liquid or liquid and gas, the method comprising:

   - defining a plurality of models of the reactive flow system, wherein each defined model represents the reactive flow system at a respective scale;
   - determining a velocity field for the reactive flow system using a first model, at a first respective scale, of the

defined plurality of models;
- determining a diffusivity for the reactive flow system using a second model, at a second respective scale different from the first respective scale, of the defined plurality of models, said determining a velocity field and determining a diffusivity being automatically performed by one or more digital processors;
- using input data, defining a plurality of reaction parameters for the reactive flow system;
- automatically determining the behavior of the reactive flow system by using the determined velocity field, the determined diffusivity, and the defined plurality of reaction parameters as inputs to a reactive transport solver; and
- determining optimized operating parameters of the reactive flow system to operate a real-world reactive flow system, the real-world reactive flow system being one among:

-- leaching of metals, such as copper;
-- geological carbon capture;
-- groundwater remediation;
-- enhanced oil recovery (EOR) with CO2 injection; or
-- corrosion and fouling of structural materials and redox flow batteries.

**2.** The method of Claim 1, wherein a given scale is a microscale, a molecular scale, or a sub-surface scale.

**3.** The method of Claim 1, wherein at least one model of the defined plurality of models is a geometric model indicating properties of the reactive flow system.

**4.** The method of Claim 1, wherein defining a given model of the plurality of models of the reactive flow system comprises:

- defining a model of one or more heterogeneous surface reactions;
- modeling rate laws, for the defined model of the one or more heterogeneous surface reactions, as functions of mineral dissolution and precipitation; and
- defining the given model based upon the modeled rate laws and a model of one or more homogeneous bulk reactions.

**5.** The method of Claim 1, wherein the determining the velocity field for the reactive flow system using the first model comprises:

- receiving an image of a material in the reactive flow system;
- segmenting the image into a plurality of phases, each phase representing a material, solid, or fluid;
- determining the velocity field of the reactive flow system based on the plurality of phases using the first model, wherein the first model is a single-phase fluid flow model.

**6.** The method of Claim 5, wherein the material is porous, or the material further comprises one or more fractures, or the material is a nano-porous clay material, or the material is Wyoming type montmorillonite having a formula of $Cu_{0.66}$ $[Al_{3.33}Mg_{0.66}]$ $[Si_8]$ $O_{20}[OH]_4$.

**7.** The method of Claim 1, wherein the determined velocity field is a multiphase velocity field.

**8.** The method of Claim 1, wherein the determining the diffusivity for the reactive flow system using the second model comprises:

providing parameters of a bulk salt solution and parameters of a salt solution in a clay interlayer nano-pore as inputs for a molecular dynamics simulation; and
using the inputs for the molecular dynamics simulation, performing the molecular dynamics simulation as a function of temperature and salt concentration, wherein results of performing the molecular dynamics simulation indicate the diffusivity for the reactive flow system.

**9.** The method of Claim 8, wherein the diffusivity is an ion diffusivity, preferably the ion diffusivity is ion diffusivity of copper $(Cu)^{2+}$.

**10.** The method of Claim 1, wherein the input data is obtained from at least one of: simulation results and a database.

**11.** The method of Claim 1, wherein the determining the behavior of the reactive flow system by using the determined

velocity field, the determined diffusivity, and the defined plurality of reaction parameters as inputs to the reactive transport solver comprises:

- solving an advection-diffusion-reaction equation using the reactive transport solver with the inputs, wherein results of the solving indicate the behavior of the reactive flow system.

**12.** The method of Claim 1, wherein the determined behavior of the reactive flow system is a concentration profile, preferably the concentration profile is a concentration profile of copper $(Cu)^{2+}$.

**13.** The method of Claim 1 further comprising:

- updating the first model and the second model based on the determined behavior of the reactive flow system;
- determining an updated velocity field for the reactive flow system using the updated first model;
- determining an updated diffusivity for the reactive flow system using the updated second model; and
- determining an updated behavior of the reactive flow system by using the updated determined velocity field, the updated determined diffusivity, and the defined plurality of reaction parameters as inputs to the reactive transport solver.

**14.** The method of Claim 13 further comprising:

- iterating: (i) the updating, (ii) the determining an updated velocity field, (iii) the determining an updated diffusivity, and (iv) the determining an updated behavior of the reactive flow system until the determined updated behavior of the reactive flow system reaches a steady state.

**15.** A computer program comprising instructions which, when the program is executed by a computer, cause the computer to carry out the method of any one of claims 1 to 14, preferably the computer program is executed by a server in communication across a network with one or more clients.

**16.** A computer-based system for determining behavior of a reactive flow system, the computer-based system comprising:

a processor; and
a memory with computer code instructions stored thereon, the processor and the memory, with the computer code instructions, being configured to cause the computer-based system to perform the method of anyone of claims 1 to 14.

## Patentansprüche

**1.** Rechnerimplementiertes Verfahren zum Bestimmen eines Verhaltens eines reaktiven Strömungssystems, das Flüssigkeit oder Flüssigkeit und Gas enthält, das Verfahren umfassend:

- Definieren einer Vielzahl von Modellen des reaktiven Strömungssystems, wobei jedes definierte Modell das reaktive Strömungssystem in einem entsprechenden Maßstab darstellt;
- Bestimmen eines Geschwindigkeitsfelds für das reaktive Strömungssystem unter Verwendung eines ersten Modells in einem ersten jeweiligen Maßstab anhand der definierten Vielzahl von Modellen;
- Bestimmen einer Diffusivität für das reaktive Strömungssystem unter Verwendung eines zweiten Modells in einem zweiten jeweiligen Maßstab, der sich von dem ersten jeweiligen Maßstab unterscheidet, anhand der definierten Vielzahl von Modellen, wobei ein Bestimmen eines Geschwindigkeitsfelds und ein Bestimmen einer Diffusivität automatisch von einem oder mehreren digitalen Prozessoren durchgeführt werden;
- Verwenden von Eingangsdaten, die eine Vielzahl von Reaktionsparametern für das reaktive Strömungssystem definieren;
- automatisches Bestimmen des Verhaltens des reaktiven Strömungssystems unter Verwendung des bestimmten Geschwindigkeitsfelds, der bestimmten Diffusivität und der definierten Vielzahl von Reaktionsparametern als Eingänge für einen reaktiven Transportlöser; und
- Bestimmen optimierter Betriebsparameter des reaktiven Strömungssystems für den Betrieb eines realen reaktiven Strömungssystems, wobei das reale reaktive Strömungssystem eines von Folgenden ist:

-- Auslaugen von Metallen, wie beispielsweise Kupfer;
-- geologische Kohlenstoffabscheidung;
-- Grundwassersanierung;
-- tertiäre Ölgewinnung (EOR) mit CO2-Injektion; oder
-- Korrosion und Verschmutzung von Strukturmaterialien und Redox-Flussbatterien.

**2.** Verfahren nach Anspruch 1, wobei eine bestimmte Skala eine Mikroskala, eine molekulare Skala oder eine unter der Oberfläche liegende Skala ist.

**3.** Verfahren nach Anspruch 1, wobei mindestens ein Modell der definierten Vielzahl von Modellen ein geometrisches Modell ist, das Eigenschaften des reaktiven Strömungssystems angibt.

**4.** Verfahren nach Anspruch 1, wobei ein Definieren eines gegebenen Modells der Vielzahl von Modellen des reaktiven Strömungssystems Folgendes umfasst:

- Definieren eines Modells für eine oder mehrere heterogene Oberflächenreaktionen;
- Modellieren von Geschwindigkeitsgesetzen für das definierte Modell der einen oder mehreren heterogenen Oberflächenreaktionen abhängig von der Mineralauflösung und -präzipitation; und
- Definieren des gegebenen Modells basierend auf den modellierten Ratengleichungen und einem Modell einer oder mehrerer homogener Massenreaktionen.

**5.** Verfahren nach Anspruch 1, wobei das Bestimmen des Geschwindigkeitsfelds für das reaktive Strömungssystem unter Verwendung des ersten Modells Folgendes umfasst:

- Empfangen eines Bilds eines Materials in dem reaktiven Strömungssystem;
- Segmentieren des Bilds in eine Vielzahl von Phasen, wobei jede Phase ein Material, einen Feststoff oder ein Fluid darstellt;
- Bestimmen des Geschwindigkeitsfeles des reaktiven Strömungssystems basierend auf der Vielzahl von Phasen unter Verwendung des ersten Modells, wobei das erste Modell ein einphasiges Fluidströmungsmodell ist.

**6.** Verfahren nach Anspruch 5, wobei das Material porös ist, oder das Material ferner einen oder mehrere Risse umfasst, oder das Material ein nanoporöses Tonmaterial ist, oder das Material Montmorillonit vom Typ Wyoming ist, das eines Formel $Cu_{0,66}[Al_{3,33}Mg_{0,66}]$ $[Si_8]$ $O_{20}[OH]_4$ aufweist.

**7.** Verfahren nach Anspruch 1, wobei das bestimmte Geschwindigkeitsfeld ein mehrphasiges Geschwindigkeitsfeld ist.

**8.** Verfahren nach Anspruch 1, wobei das Bestimmen der Diffusivität für das reaktive Strömungssystem unter Verwendung des zweiten Modells Folgendes umfasst:

Bereitstellen von Parametern einer Hauptsalzlösung und von Parametern einer Salzlösung in einer Tonzwischenschicht-Nanopore als Eingänge für eine Molekulardynamiksimulation; und
unter Verwendung der Eingänge für die Molekulardynamiksimulation, Durchführen der Molekulardynamiksimulation abhängig von Temperatur und Salzkonzentration, wobei Resultate eines Durchführens der Molekulardynamiksimulation die Diffusivität für das reaktive Strömungssystem angeben.

**9.** Verfahren nach Anspruch 8, wobei die Diffusivität eine Ionendiffusivität ist, vorzugsweise die Ionendiffusivität die Ionendiffusivität von Kupfer $(Cu)^{2+}$ ist.

**10.** Verfahren nach Anspruch 1, wobei die Eingangsdaten aus mindestens einem von Folgenden erlangt werden: Simulationsresultaten und einer Datenbank.

**11.** Verfahren nach Anspruch 1, wobei das Bestimmen des Verhaltens des reaktiven Strömungssystems unter Verwendung des bestimmten Geschwindigkeitsfelds, der bestimmten Diffusivität und der definierten Vielzahl von Reaktionsparametern als Eingänge für den reaktiven Transportlöser Folgendes umfasst:

- Lösen einer Advektions-Diffusions-Reaktions-Gleichung unter Verwendung des reaktiven Transportlösers mit den Eingängen, wobei Resultate des Lösens das Verhalten des reaktiven Strömungssystems angeben.

**12.** Verfahren nach Anspruch 1, wobei das bestimmte Verhalten des reaktiven Strömungssystems ein Konzentrationsprofil ist, vorzugsweise das Konzentrationsprofil ein Konzentrationsprofil von Kupfer $(Cu)^{2+}$ ist.

**13.** Verfahren nach Anspruch 1, ferner umfassend:

- Aktualisieren des ersten Modells und des zweiten Modells basierend auf dem bestimmten Verhalten des reaktiven Strömungssystems;
- Bestimmen eines aktualisierten Geschwindigkeitsfelds für das reaktive Strömungssystem unter Verwendung des aktualisierten ersten Modells;
- Bestimmen einer aktualisierten Diffusivität für das reaktive Strömungssystem unter Verwendung des aktualisierten zweiten Modells; und
- Bestimmen eines aktualisierten Verhaltens des reaktiven Strömungssystems unter Verwendung des aktualisierten bestimmten Geschwindigkeitsfelds, der aktualisierten bestimmten Diffusivität und der definierten Vielzahl von Reaktionsparametern als Eingänge für den reaktiven Transportlöser.

**14.** Verfahren nach Anspruch 13, ferner umfassend:

- iterierend: (i) das Aktualisieren, (ii) das Bestimmen eines aktualisierten Geschwindigkeitsfelds, (iii) das Bestimmen einer aktualisierten Diffusivität, und (iv) das Bestimmen eines aktualisierten Verhaltens des reaktiven Strömungssystems, bis das bestimmte aktualisierte Verhalten des reaktiven Strömungssystems einen stationären Zustand erreicht.

**15.** Rechnerprogramm, umfassend Anweisungen, die, wenn das Programm von einem Rechner ausgeführt wird, den Rechner veranlassen, das Verfahren nach einem der Ansprüche 1 bis 14 durchzuführen, wobei das Rechnerprogramm vorzugsweise von einem Server ausgeführt wird, der über ein Netzwerk mit einem oder mehreren Clients kommuniziert.

**16.** Rechnergestütztes System zum Bestimmen eines Verhaltens eines reaktiven Strömungssystems, das rechnergestützte System umfassend:

einen Prozessor; und
einen Speicher mit darauf gespeicherten Rechnercodeanweisungen, wobei der Prozessor und der Speicher mit den Rechnercodeanweisungen konfiguriert sind, um das rechnergestützte System zu veranlassen, das Verfahren nach einem der Ansprüche 1 bis 14 durchzuführen.

## Revendications

**1.** Procédé mis en œuvre par ordinateur pour déterminer le comportement d'un système d'écoulement réactif qui comprend un liquide ou un liquide et un gaz, le procédé comprenant le fait de :

- définir une pluralité de modèles du système d'écoulement réactif, dans lequel chaque modèle défini représente le système d'écoulement réactif à une échelle respective ;
- déterminer un champ de vitesse pour le système d'écoulement réactif à l'aide d'un premier modèle, à une première échelle respective, parmi la pluralité définie de modèles ;
- déterminer une diffusivité pour le système d'écoulement réactif à l'aide d'un deuxième modèle, à une deuxième échelle respective, différente de la première échelle respective, parmi la pluralité définie de modèles, ladite détermination d'un champ de vitesse et ladite détermination d'une diffusivité étant effectuées automatiquement par un ou plusieurs processeurs numériques ;
- à l'aide de données d'entrée, définir une pluralité de paramètres de réaction pour le système d'écoulement réactif ;
- déterminer automatiquement le comportement du système d'écoulement réactif en utilisant le champ de vitesse déterminé, la diffusivité déterminée et la pluralité définie de paramètres de réaction comme entrées d'un solveur de transport réactif ; et
- déterminer des paramètres de fonctionnement optimisés du système d'écoulement réactif pour faire fonctionner un système d'écoulement réactif concret, le système d'écoulement réactif concret étant l'un parmi :

  -- la lixiviation de métaux, tels que le cuivre ;

-- le captage géologique du carbone ;
-- la dépollution des eaux souterraines ;
-- la récupération assistée du pétrole (EOR) avec injection de COz ; ou
-- la corrosion et l'encrassement des matériaux structurels et des batteries à flux redox.

**2.** Procédé selon la revendication 1, dans lequel une échelle donnée est une échelle microscopique, une échelle moléculaire ou une échelle sous-superficielle.

**3.** Procédé selon la revendication 1, dans lequel au moins un modèle parmi la pluralité définie de modèles est un modèle géométrique indiquant les propriétés du système d'écoulement réactif.

**4.** Procédé selon la revendication 1, dans lequel la définition d'un modèle donné parmi la pluralité de modèles du système d'écoulement réactif comprend le fait de :

- définir un modèle d'une ou plusieurs réactions de surface hétérogènes ;
- modéliser des lois de vitesse, pour le modèle défini de la ou des réactions de surface hétérogènes, en tant que fonctions de la dissolution et de la précipitation des minéraux ; et
- définir le modèle donné sur la base des lois de vitesse modélisées et d'un modèle d'une ou plusieurs réactions homogènes en vrac.

**5.** Procédé selon la revendication 1, dans lequel la détermination du champ de vitesse pour le système d'écoulement réactif à l'aide du premier modèle comprend le fait de :

- recevoir une image d'un matériau dans le système d'écoulement réactif ;
- segmenter l'image en une pluralité de phases, chaque phase représentant un matériau, un solide ou un fluide ;
- déterminer le champ de vitesse du système d'écoulement réactif sur la base de la pluralité de phases à l'aide du premier modèle, dans lequel le premier modèle est un modèle d'écoulement de fluide à phase unique.

**6.** Procédé selon la revendication 5, dans lequel le matériau est poreux, ou le matériau comprend en outre une ou plusieurs fractures, ou le matériau est un matériau argileux nanoporeux, ou le matériau est une montmorillonite de type Wyoming ayant une formule $Cu_{0.66}[Al_{3.33}Mg_{0.66}]$ [Sis] $O_{20}[OH]_4$.

**7.** Procédé selon la revendication 1, dans lequel le champ de vitesse déterminé est un champ de vitesse multiphase.

**8.** Procédé selon la revendication 1, dans lequel la détermination de la diffusivité pour le système d'écoulement réactif à l'aide du deuxième modèle comprend le fait de :

fournir des paramètres d'une solution saline en vrac et de paramètres d'une solution saline dans un nanopore intercouche d'argile comme entrées pour une simulation de dynamique moléculaire ; et
à l'aide des entrées pour la simulation de dynamique moléculaire, effectuer la simulation de dynamique moléculaire en fonction de la température et de la concentration en sel, dans lequel les résultats de la réalisation de la simulation de dynamique moléculaire indiquent la diffusivité pour le système d'écoulement réactif.

**9.** Procédé selon la revendication 8, dans lequel la diffusivité est une diffusivité ionique, de préférence la diffusivité ionique est la diffusivité ionique du cuivre $(Cu)^{2+}$.

**10.** Procédé selon la revendication 1, dans lequel les données d'entrée sont obtenues à partir d'au moins l'un des éléments parmi : les résultats de simulation et une base de données.

**11.** Procédé selon la revendication 1, dans lequel la détermination du comportement du système d'écoulement réactif en utilisant le champ de vitesse déterminé, la diffusivité déterminée et la pluralité définie de paramètres de réaction comme entrées du solveur de transport réactif comprend le fait de :

- résoudre une équation d'advection-diffusion-réaction à l'aide du solveur de transport réactif avec les entrées, dans lequel les résultats de la résolution indiquent le comportement du système d'écoulement réactif.

**12.** Procédé selon la revendication 1, dans lequel le comportement déterminé du système d'écoulement réactif est un profil de concentration, de préférence le profil de concentration est un profil de concentration du cuivre $(Cu)^{2+}$.

**13.** Procédé selon la revendication 1 comprenant en outre le fait de :

- mettre à jour le premier modèle et le deuxième modèle sur la base du comportement déterminé du système d'écoulement réactif ;
- déterminer un champ de vitesse mis à jour pour le système d'écoulement réactif à l'aide du premier modèle mis à jour ;
- déterminer une diffusivité mise à jour pour le système d'écoulement réactif à l'aide du deuxième modèle mis à jour ; et
- déterminer un comportement mis à jour du système d'écoulement réactif en utilisant le champ de vitesse déterminé mis à jour, la diffusivité déterminée mise à jour et la pluralité définie de paramètres de réaction comme entrées du solveur de transport réactif.

**14.** Procédé selon la revendication 13, comprenant en outre le fait de :

- itérer : (i) la mise à jour, (ii) la détermination d'un champ de vitesse mis à jour, (iii) la détermination d'une diffusivité mise à jour, et (iv) la détermination d'un comportement mis à jour du système d'écoulement réactif jusqu'à ce que le comportement mis à jour déterminé du système d'écoulement réactif atteigne un état stable.

**15.** Programme informatique comprenant des instructions qui, lorsque le programme est exécuté par un ordinateur, amènent l'ordinateur à mettre en œuvre le procédé selon l'une quelconque des revendications 1 à 14, dans lequel, de préférence le programme informatique est exécuté par un serveur en communication à travers un réseau avec un ou plusieurs clients.

**16.** Système informatisé permettant de déterminer le comportement d'un système d'écoulement réactif, le système informatisé comprenant :

un processeur ; et
une mémoire sur laquelle sont stockées des instructions de code informatique, le processeur et la mémoire, avec les instructions de code informatique, étant configurés pour amener le système informatisé à mettre en œuvre le procédé selon l'une quelconque des revendications 1 à 14.

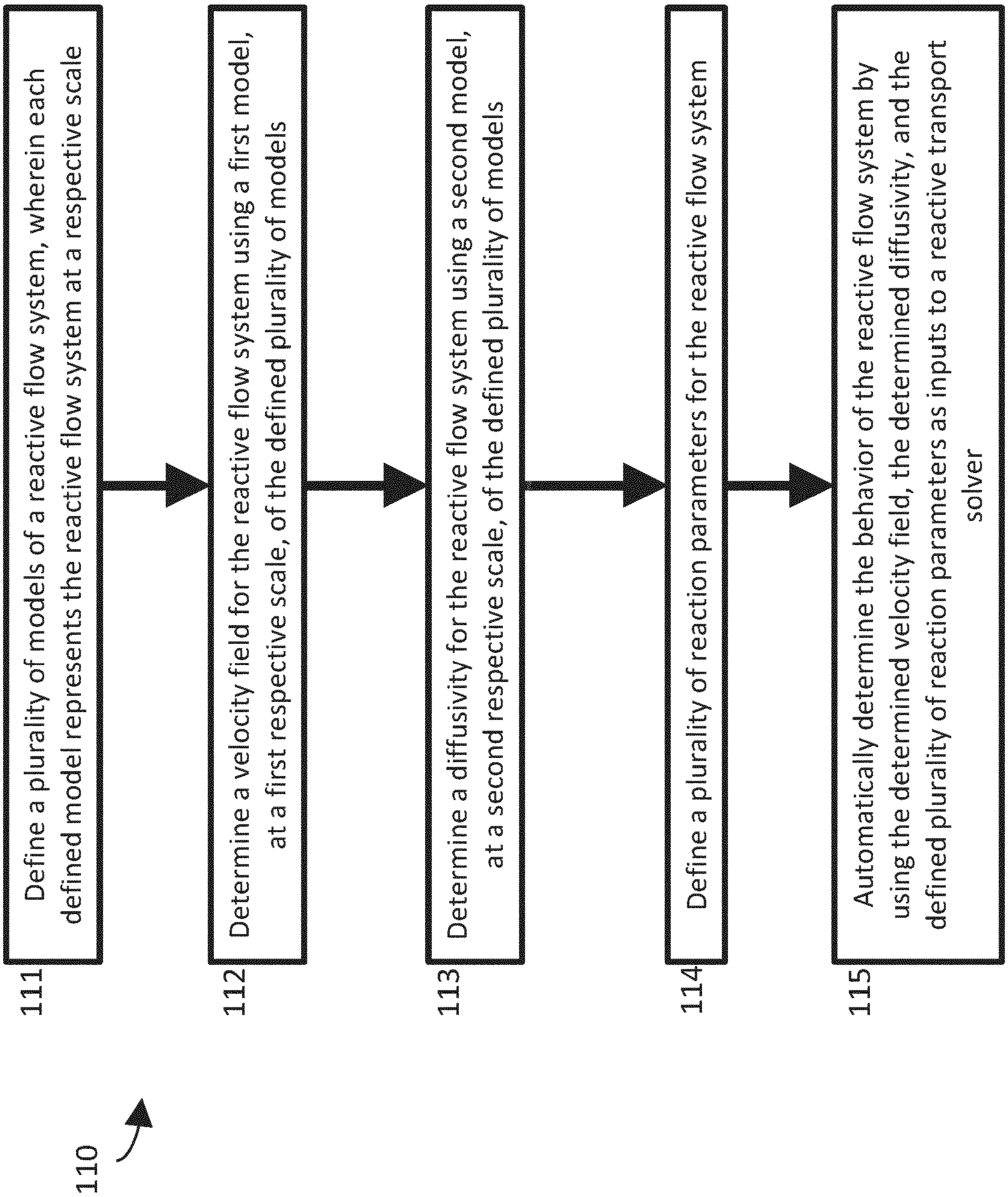
110
111
Define a plurality of models of a reactive flow system, wherein each defined model represents the reactive flow system at a respective scale
112
Determine a velocity field for the reactive flow system using a first model, at a first respective scale, of the defined plurality of models
113
Determine a diffusivity for the reactive flow system using a second model, at a second respective scale, of the defined plurality of models
114
Define a plurality of reaction parameters for the reactive flow system
115
Automatically determine the behavior of the reactive flow system by using the determined velocity field, the determined diffusivity, and the defined plurality of reaction parameters as inputs to a reactive transport solver

FIG. 1

Attorney Docket No.: 4412.1047-003

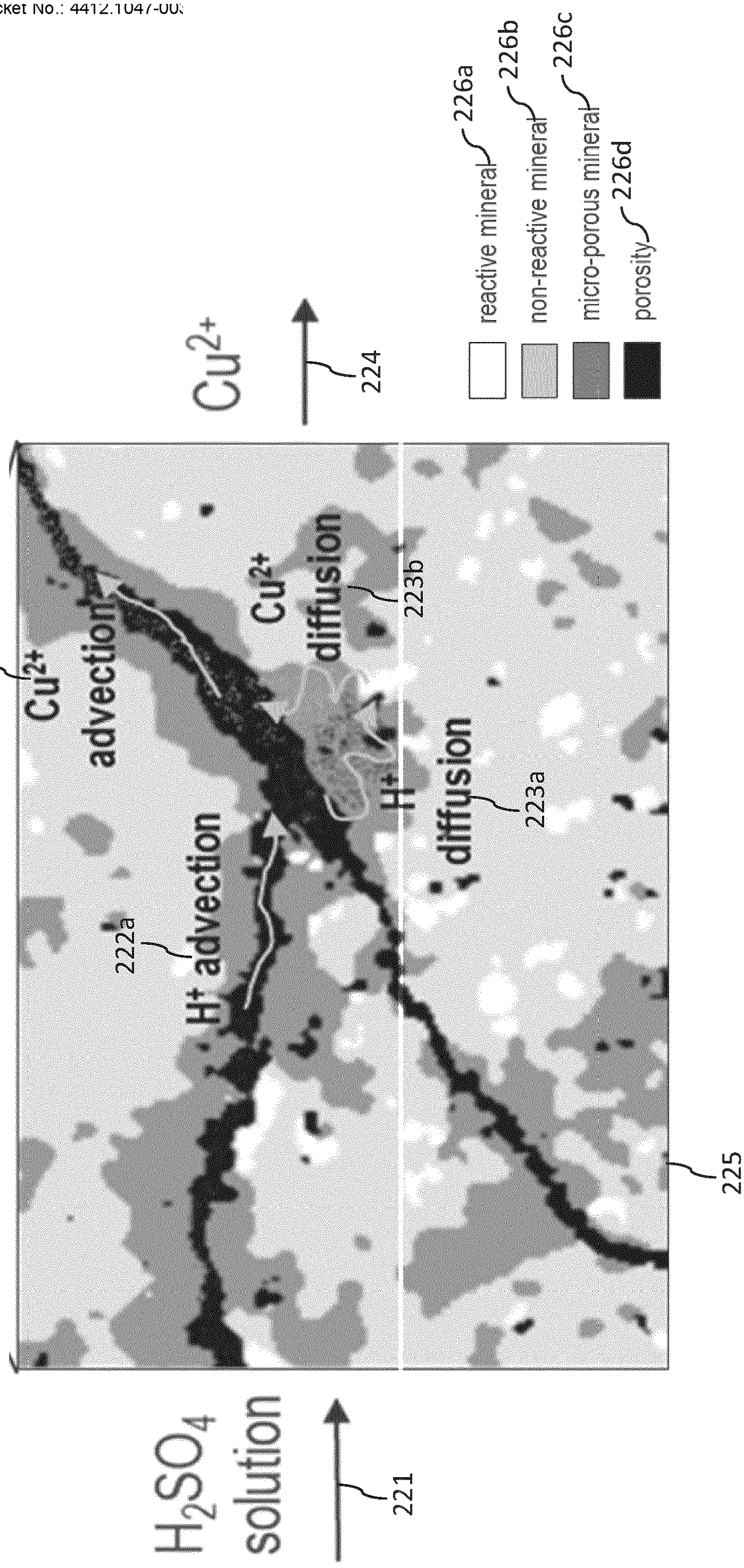


FIG. 2

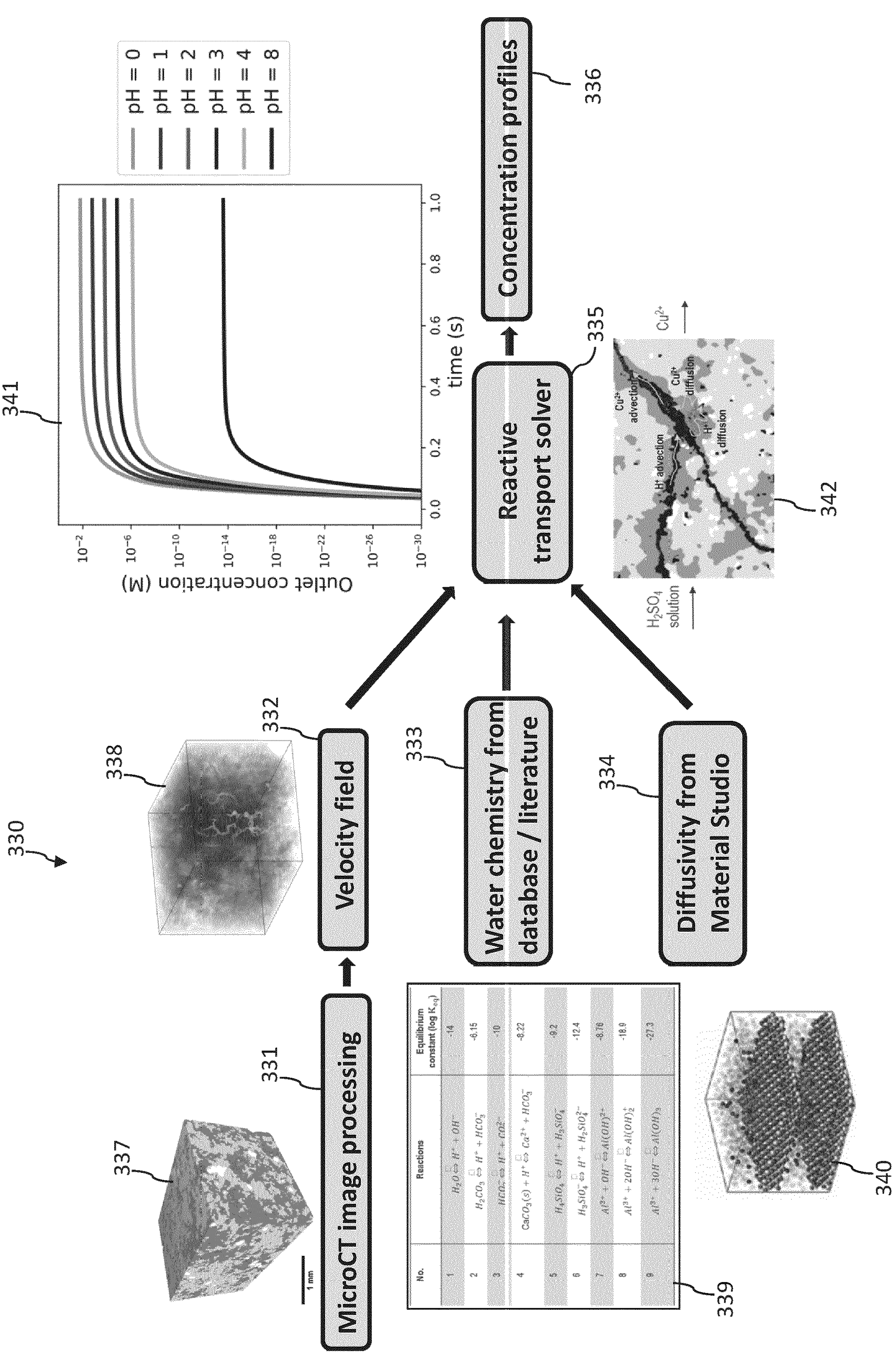
330
337
1 mm
331
MicroCT image processing
338
332
Velocity field
333
Water chemistry from database / literature
334
Diffusivity from Material Studio
339
No.
Reactions
Equilibrium constant (log K<sub>eq</sub>)
340
341
Outlet concentration (M)
time (s)
pH = 0
pH = 1
pH = 2
pH = 3
pH = 4
pH = 8
335
Reactive transport solver
336
Concentration profiles
342
H<sub>2</sub>SO<sub>4</sub> solution
Cu<sup>2+</sup>
H<sup>+</sup> advection
H<sup>+</sup> diffusion
Cu<sup>2+</sup> advection
Cu<sup>2+</sup> diffusion

FIG. 3

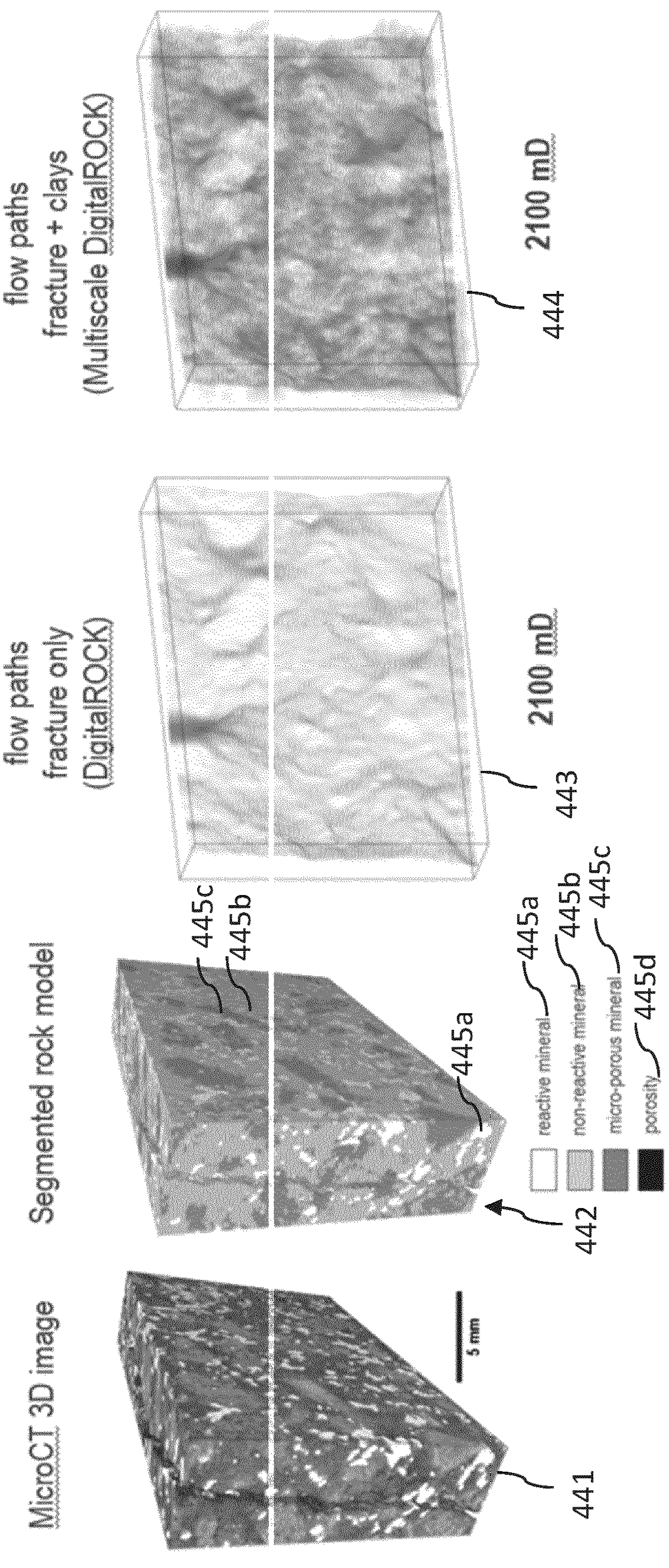

MicroCT 3D image
5 mm
441
Segmented rock model
442
445a
445b
445c
reactive mineral
non-reactive mineral
micro-porous mineral
porosity
445d
flow paths
fracture only
(DigitalROCK)
2100 mD
443
flow paths
fracture + clays
(Multiscale DigitalROCK)
2100 mD
444


FIG. 4

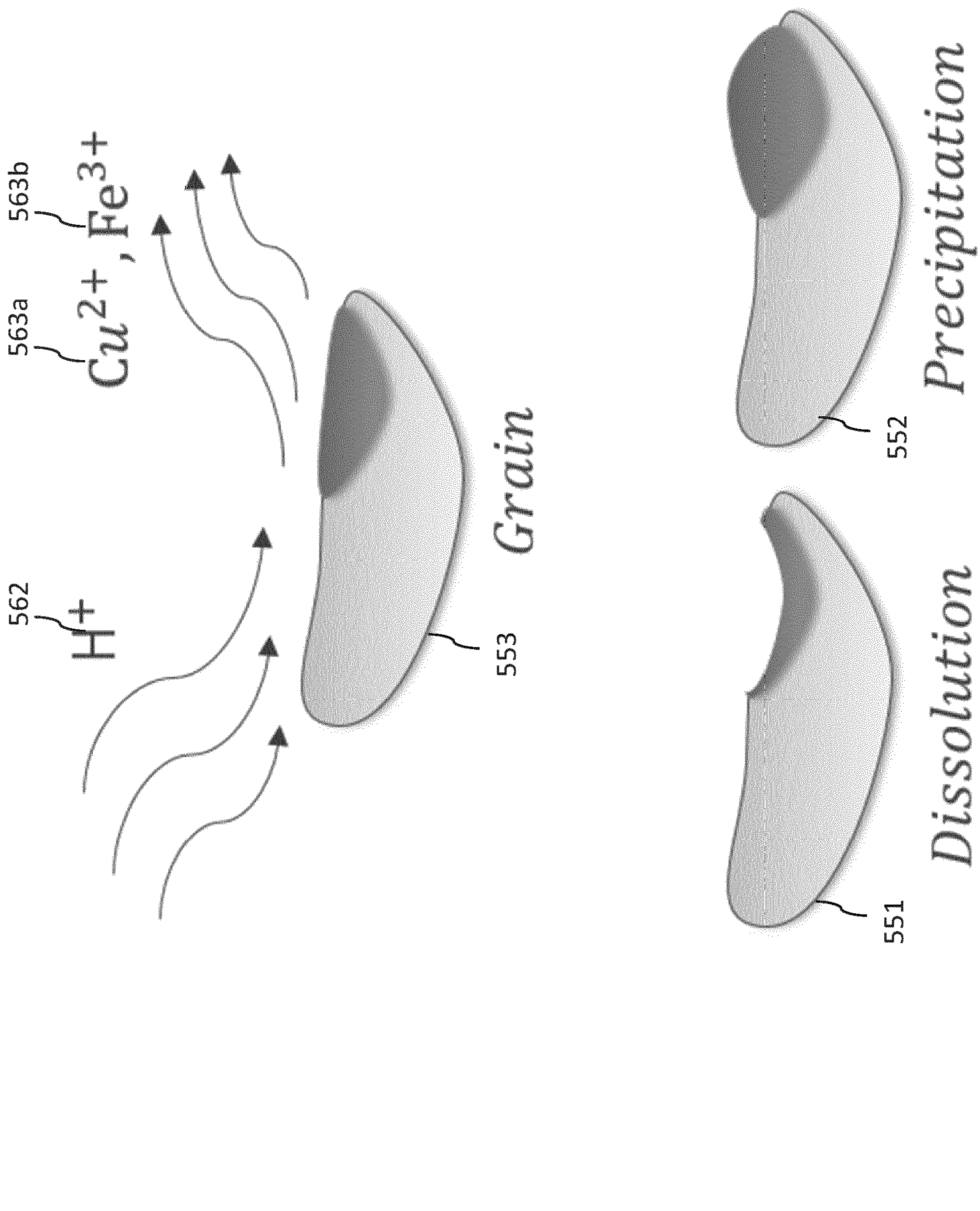
562
H+
563a
563b
$Cu^{2+}$, $Fe^{3+}$
553
Grain
551
Dissolution
552
Precipitation

FIG. 5A

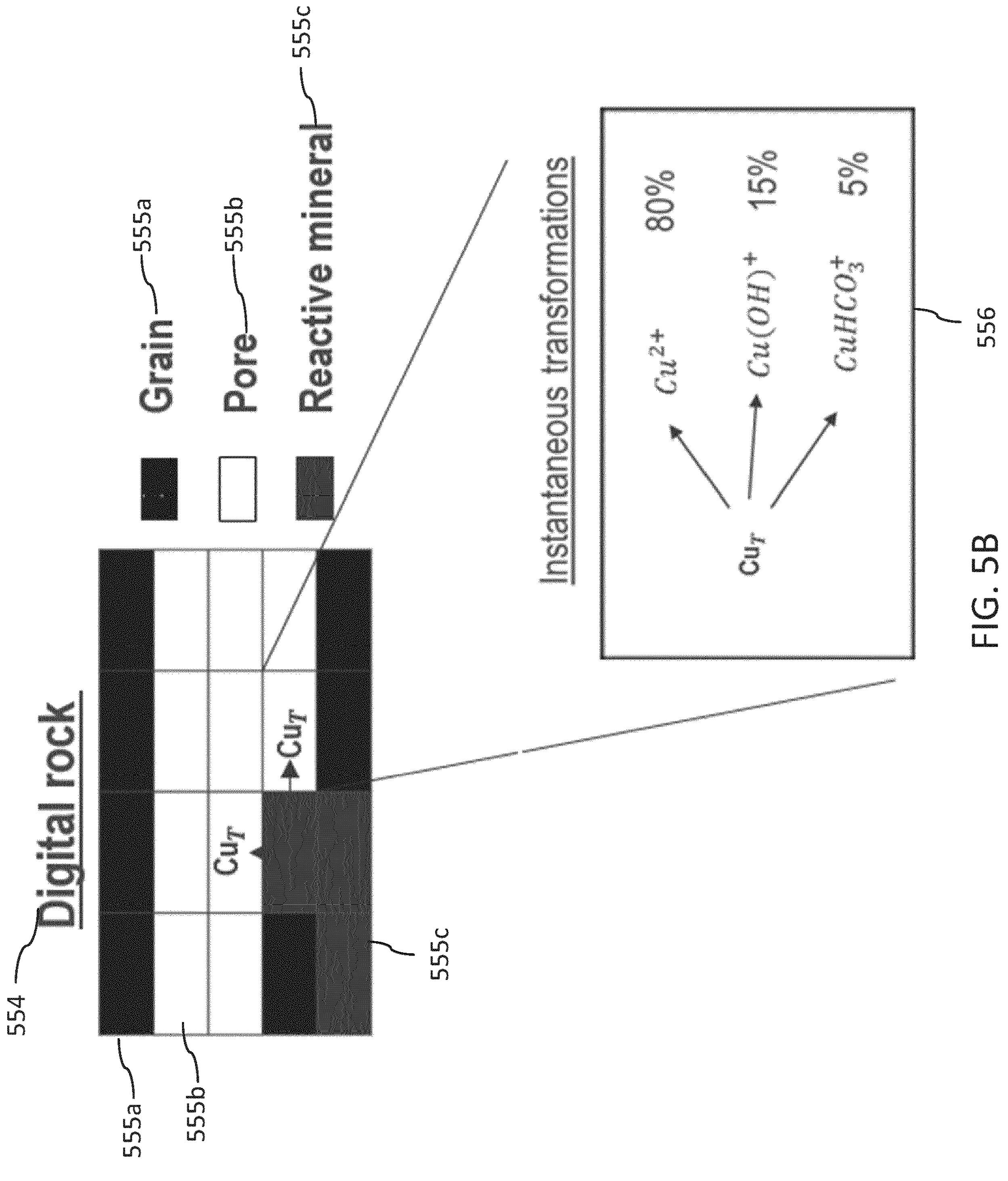
554
Digital rock
555a
555b
555c
$Cu_T$
$Cu_T$
Grain
Pore
Reactive mineral
Instantaneous transformations
$Cu_T$
$Cu^{2+}$ 80%
$Cu(OH)^+$ 15%
$CuHCO_3^+$ 5%
556

FIG. 5B

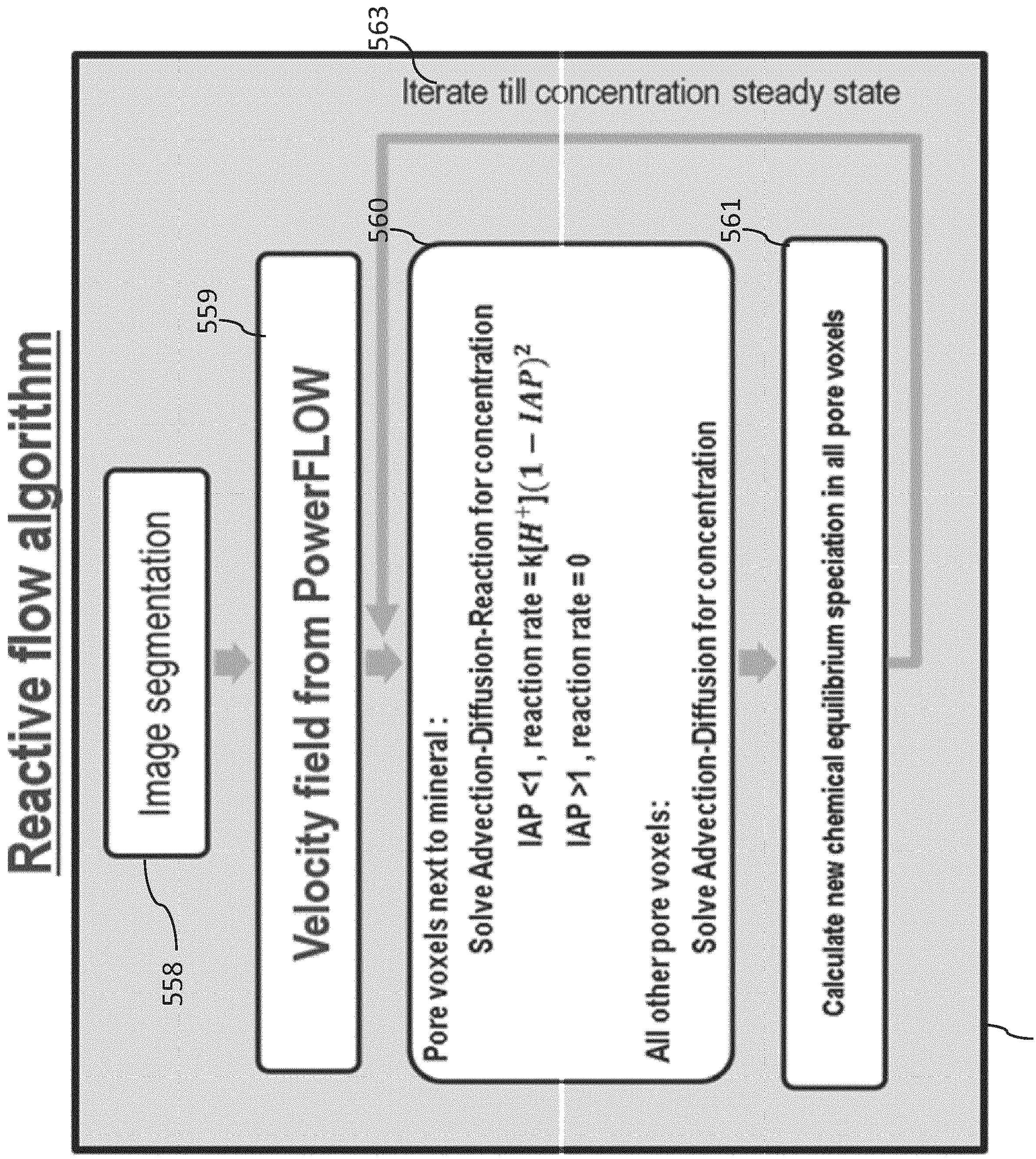
Reactive flow algorithm
Image segmentation
Velocity field from PowerFLOW
Pore voxels next to mineral :
Solve Advection-Diffusion-Reaction for concentration
IAP <1 , reaction rate = k[$H^+$](1 − IAP)$^2$
IAP >1 , reaction rate = 0
All other pore voxels:
Solve Advection-Diffusion for concentration
Calculate new chemical equilibrium speciation in all pore voxels
Iterate till concentration steady state
557
558
559
560
561
563

FIG. 5C

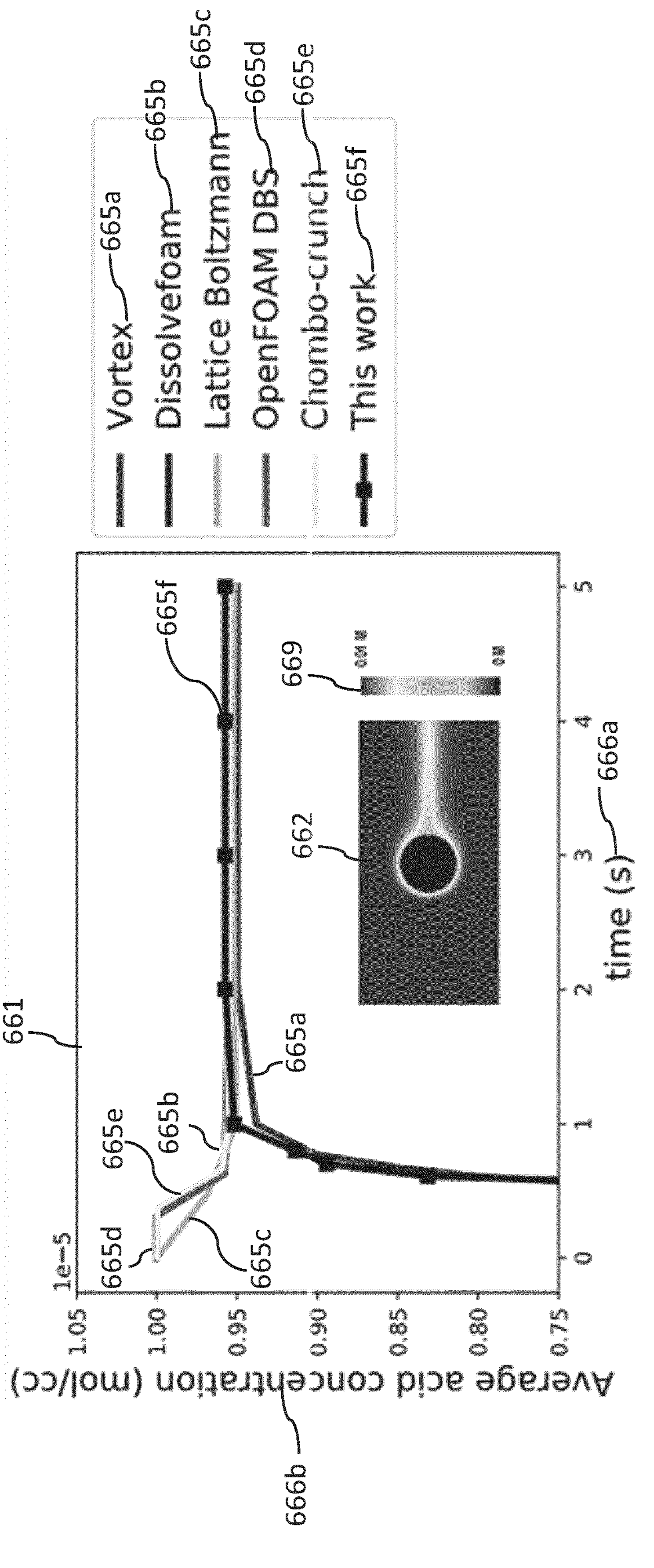

661
Vortex
Dissolvefoam
Lattice Boltzmann
OpenFOAM DBS
Chombo-crunch
This work
665a
665b
665c
665d
665e
665f
1e−5
Average acid concentration (mol/cc)
1.05
1.00
0.95
0.90
0.85
0.80
0.75
time (s)
0
1
2
3
4
5
662
669
666a
666b


FIG. 6A

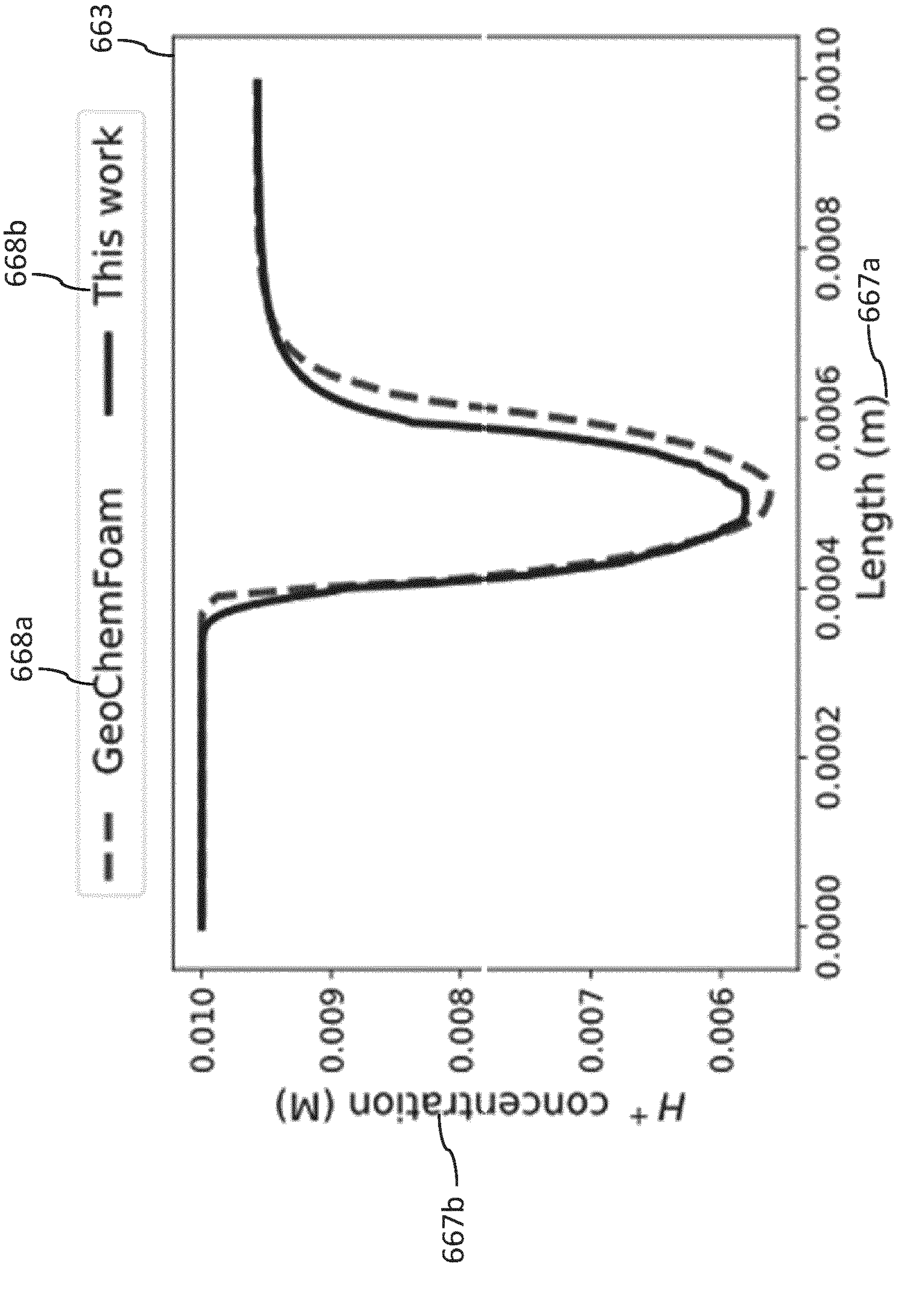
663
668a
668b
GeoChemFoam
This work
0.010
0.009
0.008
0.007
0.006
$H^+$ concentration (M)
667b
0.0000
0.0002
0.0004
0.0006
0.0008
0.0010
Length (m)
667a

FIG. 6B

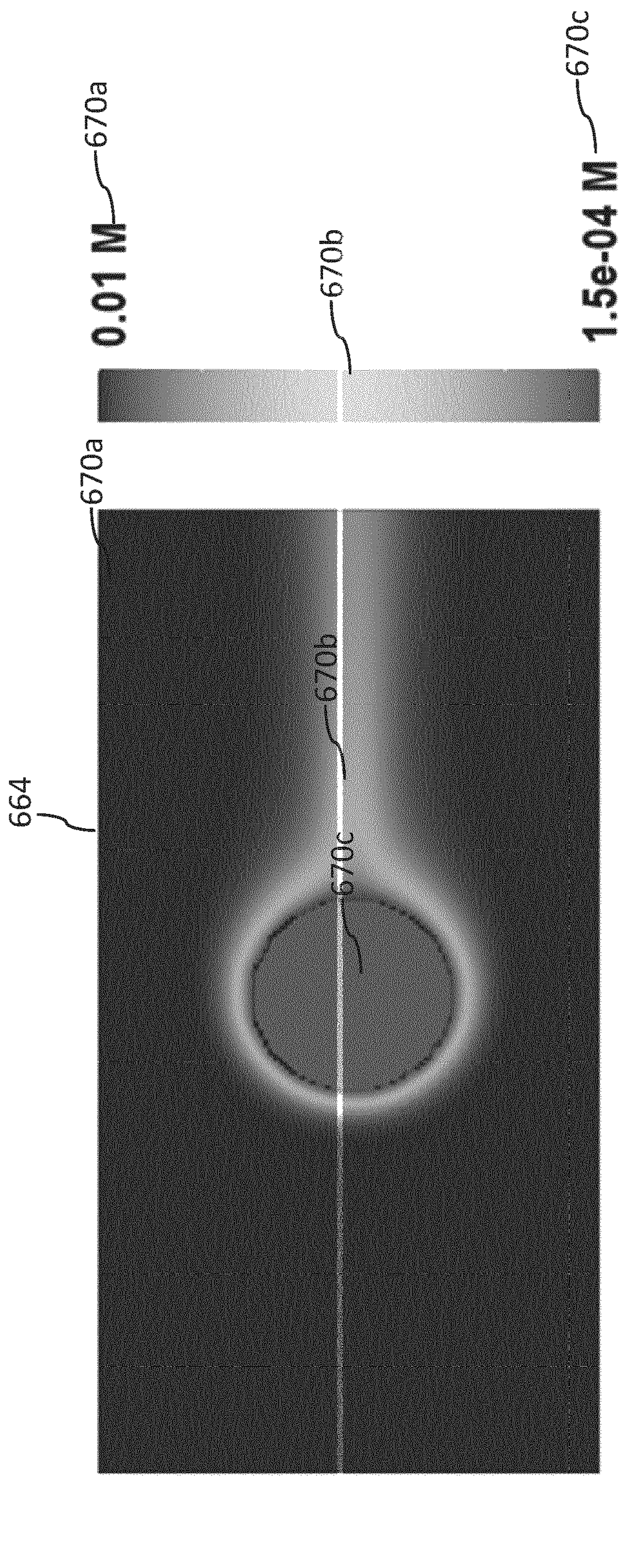


FIG. 6C

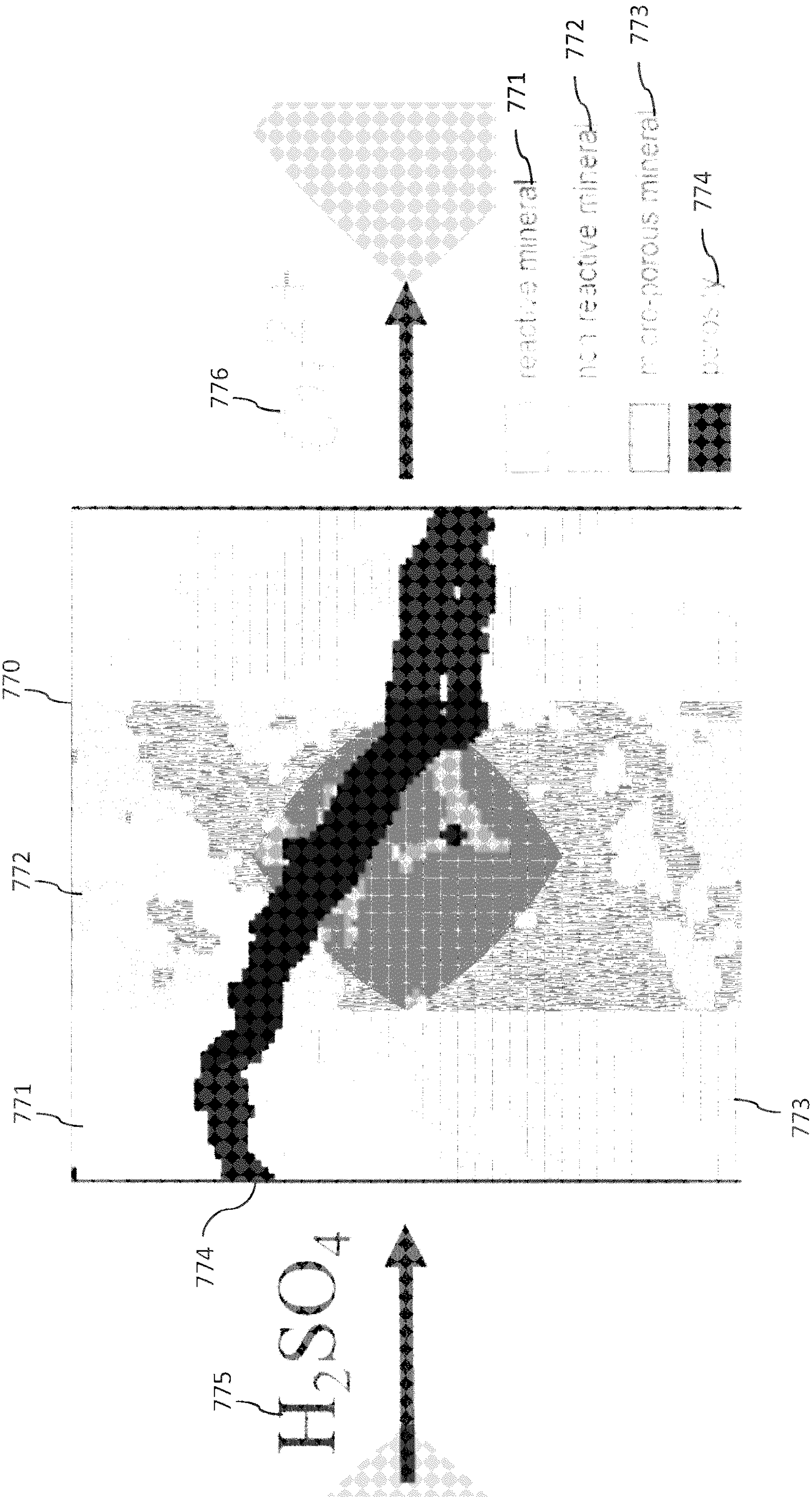
770
771
772
773
774
775
776
$H_2SO_4$
reactive mineral
non reactive mineral
micro-porous mineral
porosity

FIG. 7A

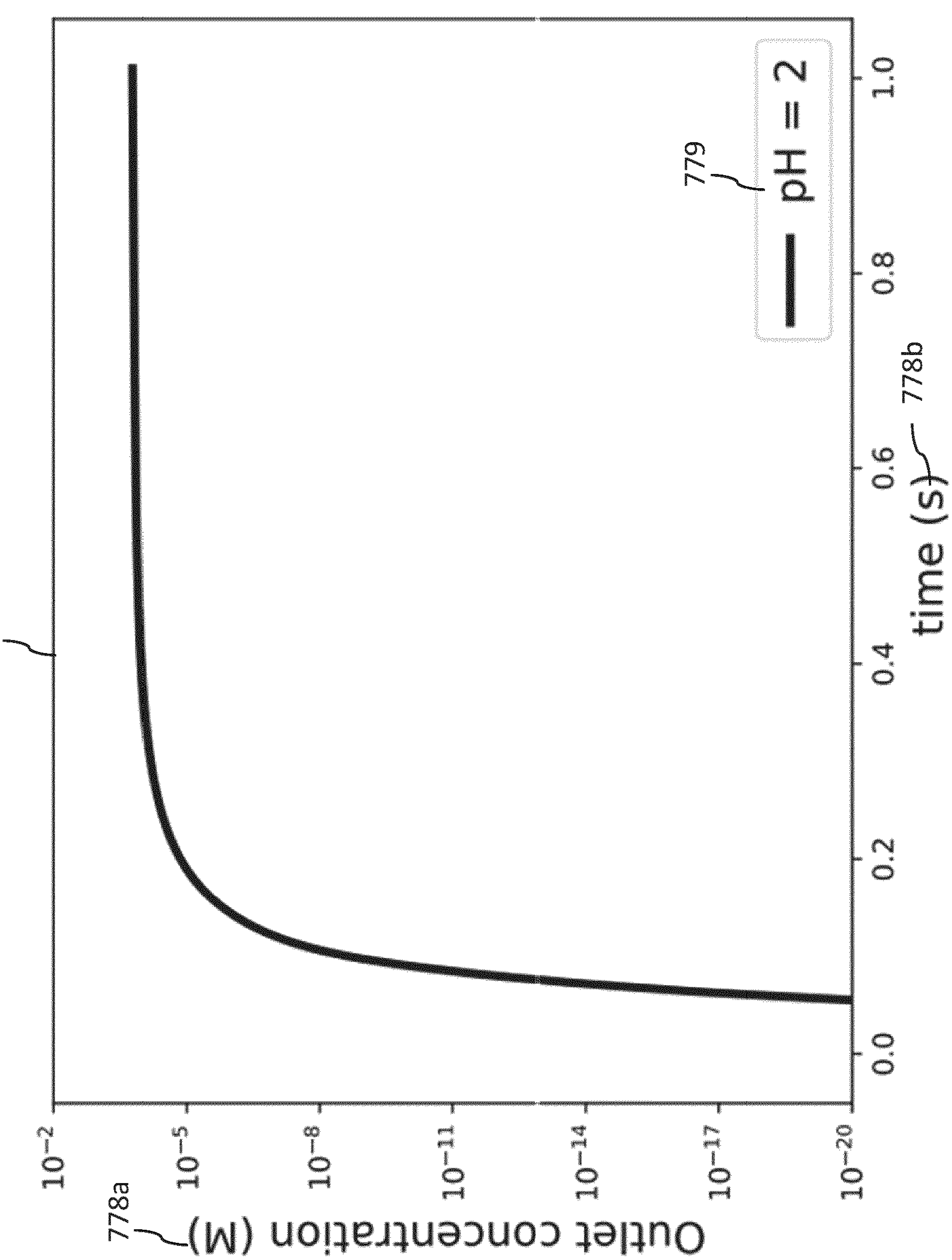
777
Outlet concentration (M)
778a
10−2
10−5
10−8
10−11
10−14
10−17
10−20
0.0
0.2
0.4
0.6
0.8
1.0
time (s)
778b
pH = 2
779

FIG. 7B

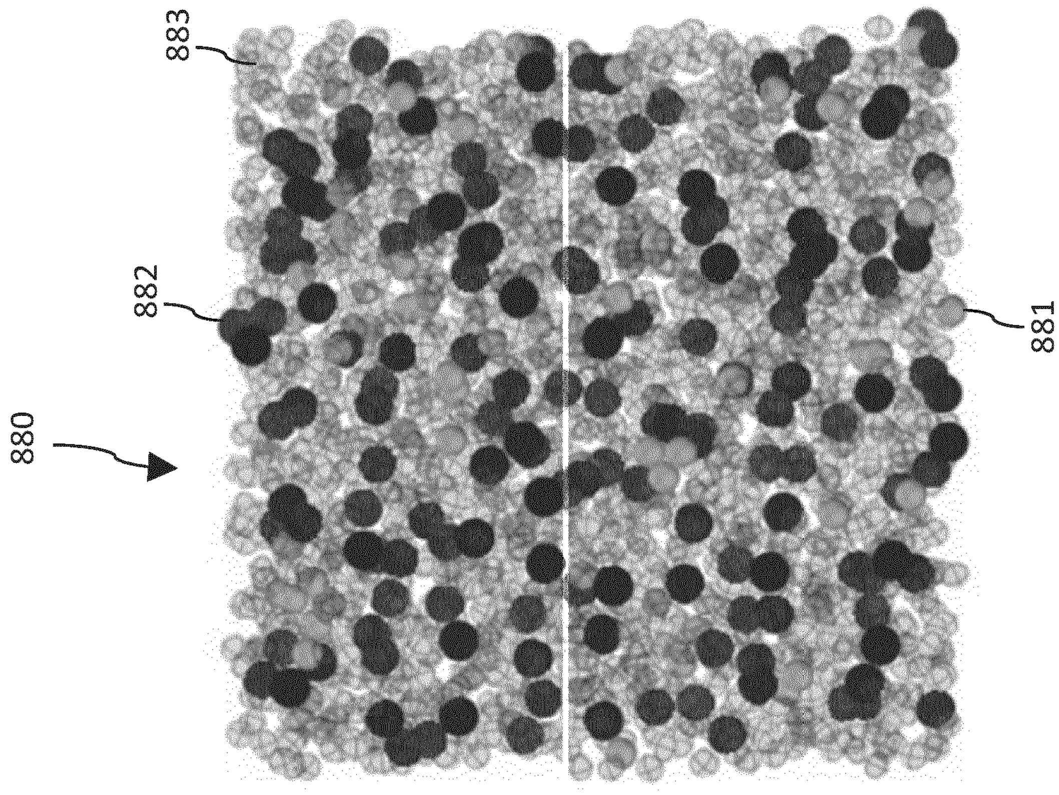


FIG. 8A

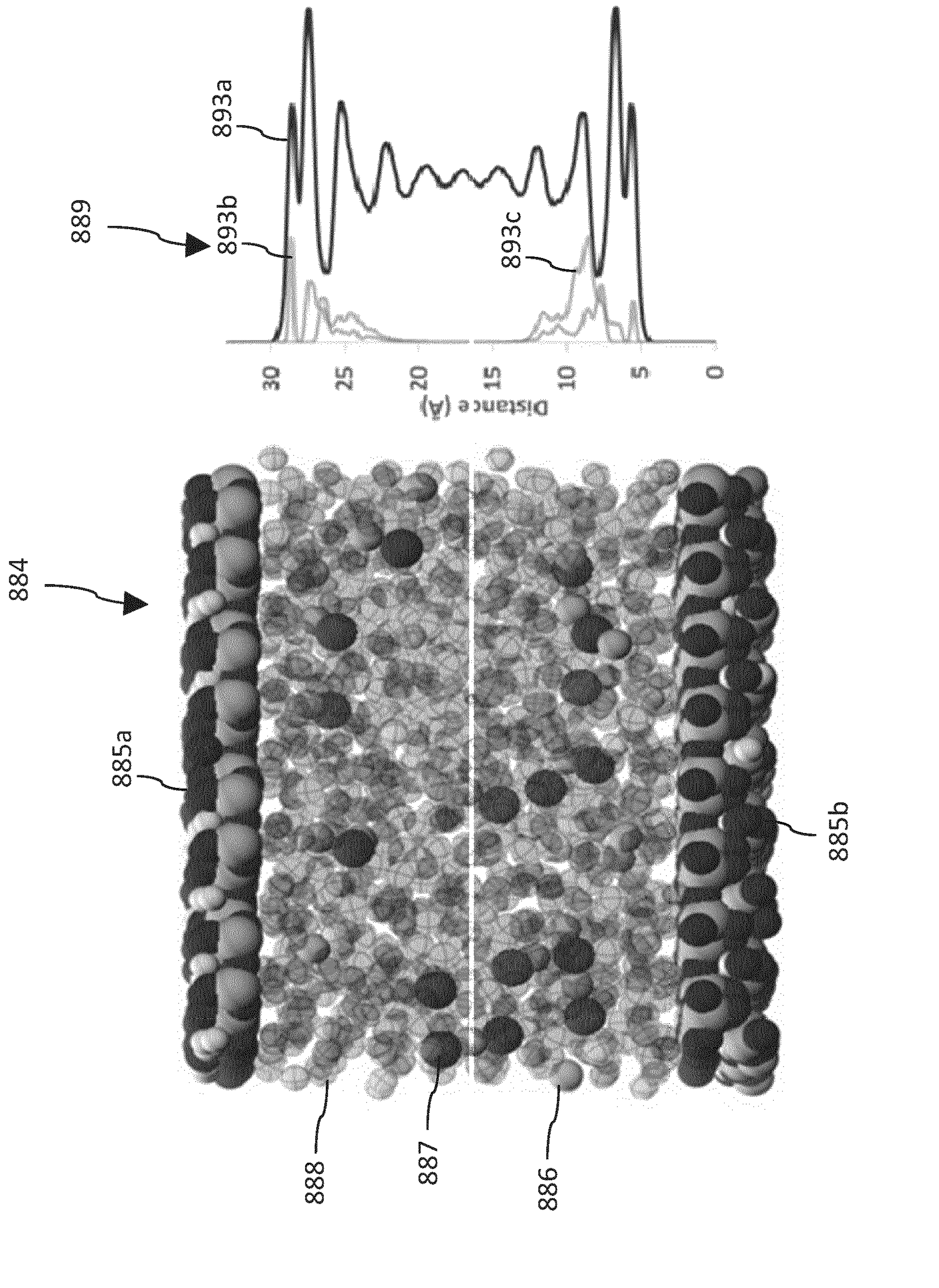
884
885a
885b
886
887
888
889
893a
893b
893c
Distance (Å)
30
25
20
15
10
5
0

FIG. 8B

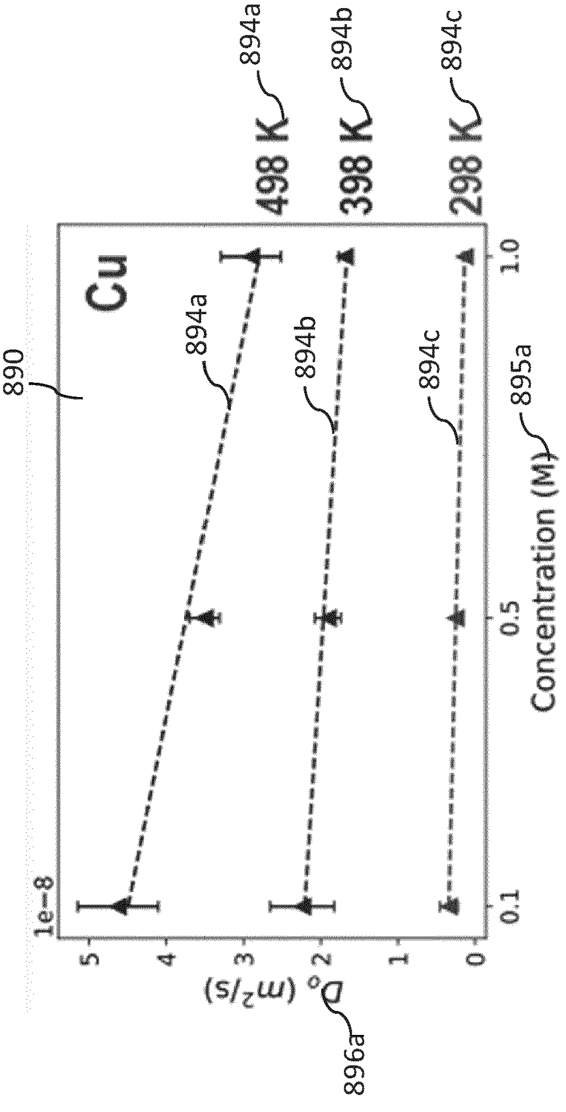

890
Cu
1e−8
5
4
3
2
1
0
$D_o$ ($m^2$/s)
896a
0.1
0.5
1.0
Concentration (M)
895a
894a
894b
894c
498 K
398 K
298 K


FIG. 8C

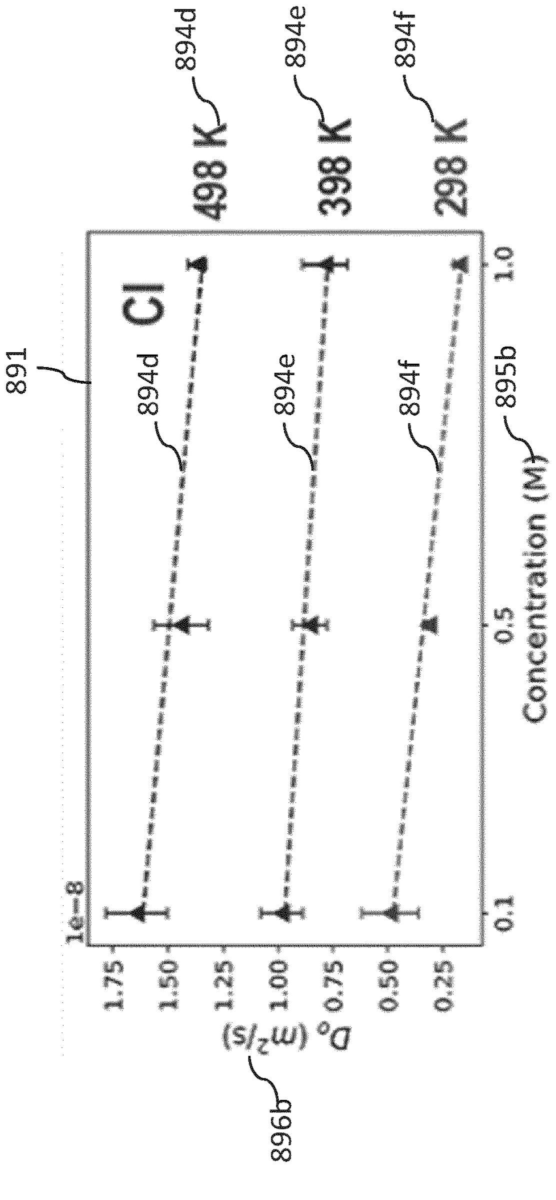

891
Cl
1e−8
1.75
1.50
1.25
1.00
0.75
0.50
0.25
$D_o$ ($m^2$/s)
896b
0.1
0.5
1.0
Concentration (M)
895b
894d
894e
894f
498 K
398 K
298 K


FIG. 8D

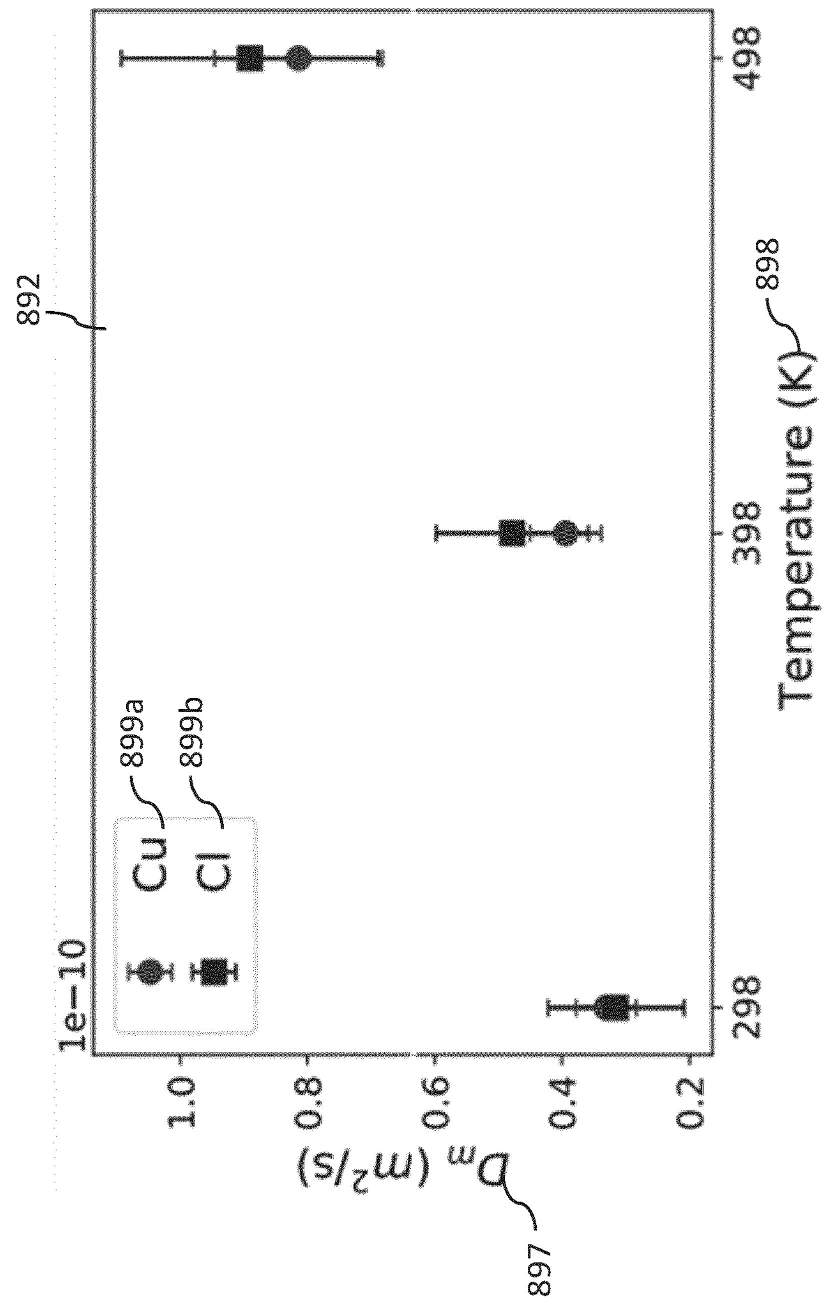
892
899a
899b
Cu
Cl
1e−10
1.0
0.8
0.6
0.4
0.2
$D_m$ ($m^2$/s)
897
298
398
498
Temperature (K)
898

FIG. 8E

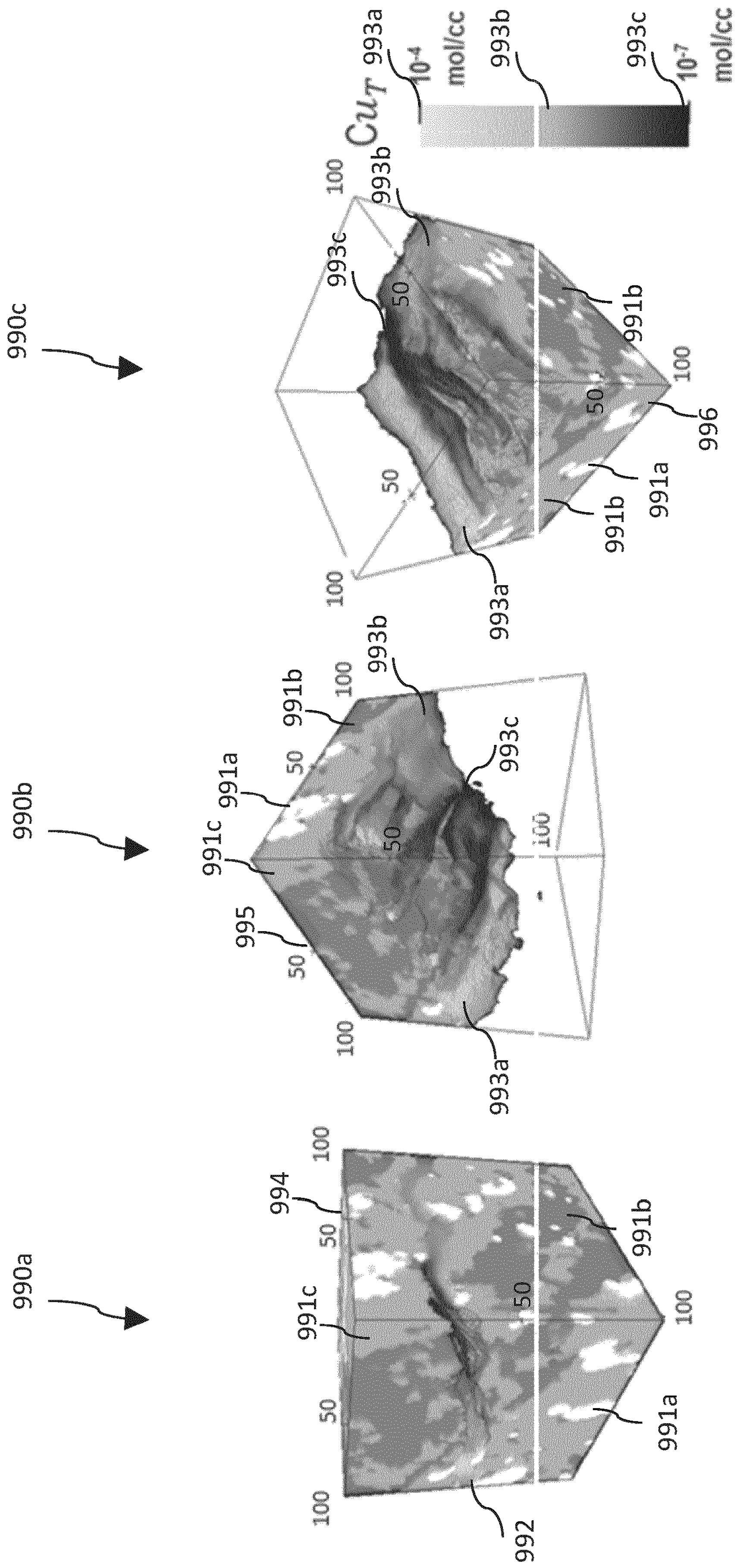
990a
990b
990c
991a
991b
991c
992
993a
993b
993c
994
995
996
$Cu_T$
$10^{-4}$ mol/cc
$10^{-7}$ mol/cc
50
100

FIG. 9

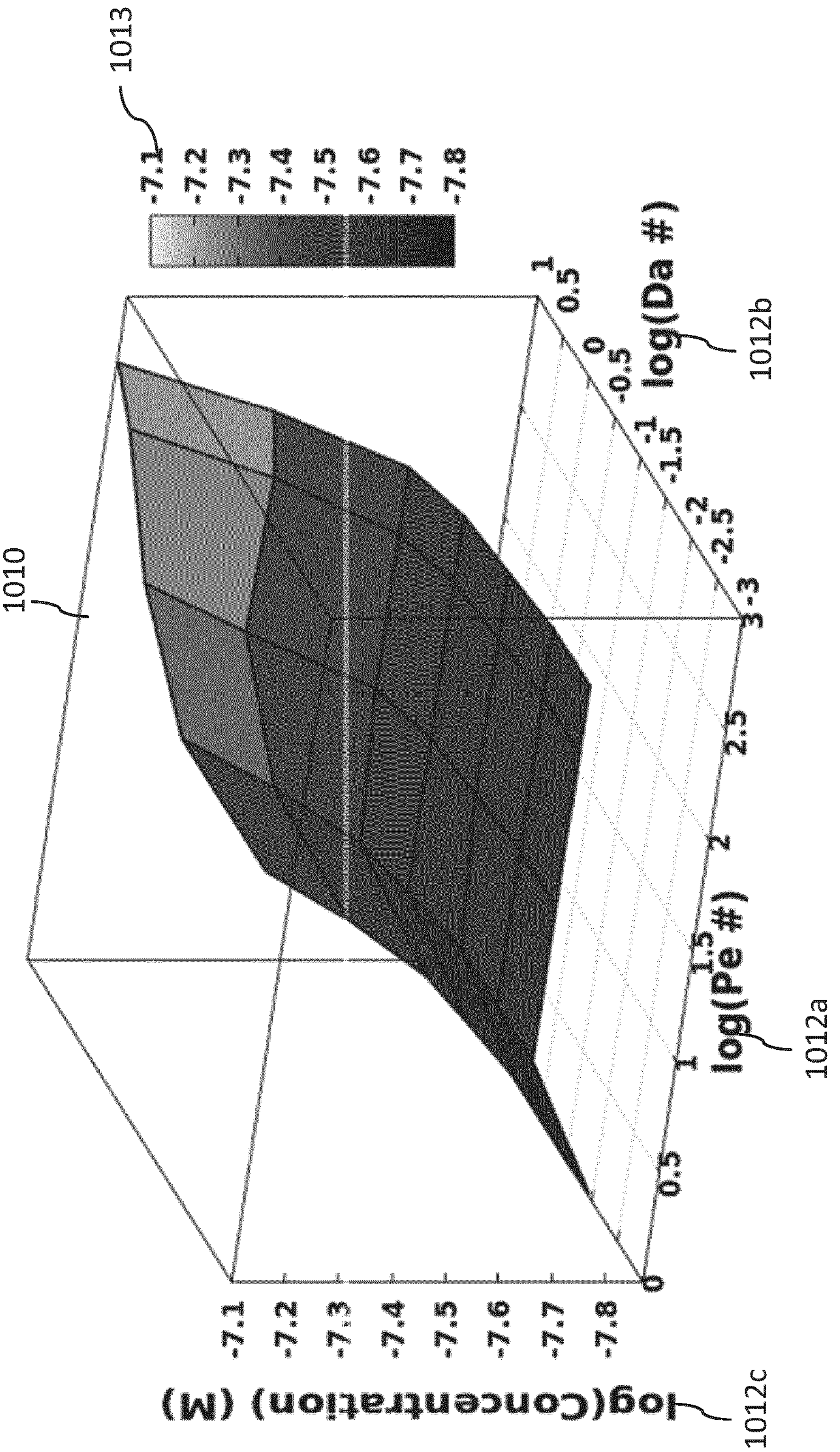
1013
-7.1
-7.2
-7.3
-7.4
-7.5
-7.6
-7.7
-7.8
1010
log(Da #)
1012b
1
0.5
0
-0.5
-1
-1.5
-2
-2.5
-3
log(Pe #)
1012a
0
0.5
1
1.5
2
2.5
3
log(Concentration) (M)
1012c
-7.1
-7.2
-7.3
-7.4
-7.5
-7.6
-7.7
-7.8

FIG. 10A

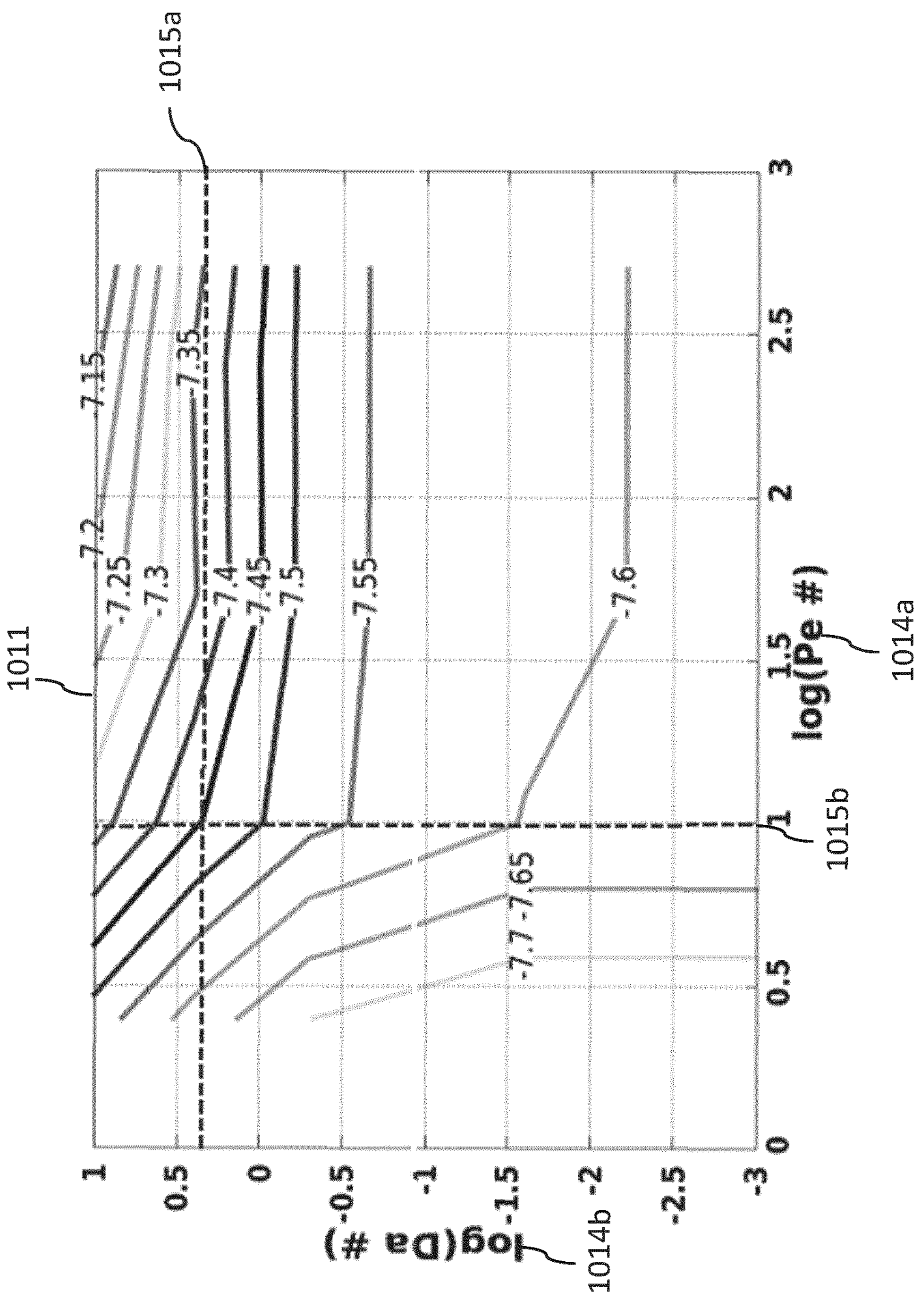
1015a
1011
1014a
1015b
1014b
log(Pe #)
log(Da #)
-7.15
-7.2
-7.25
-7.3
-7.35
-7.4
-7.45
-7.5
-7.55
-7.6
-7.65
-7.7
0
0.5
1
1.5
2
2.5
3
1
0.5
0
-0.5
-1
-1.5
-2
-2.5
-3

FIG. 10B

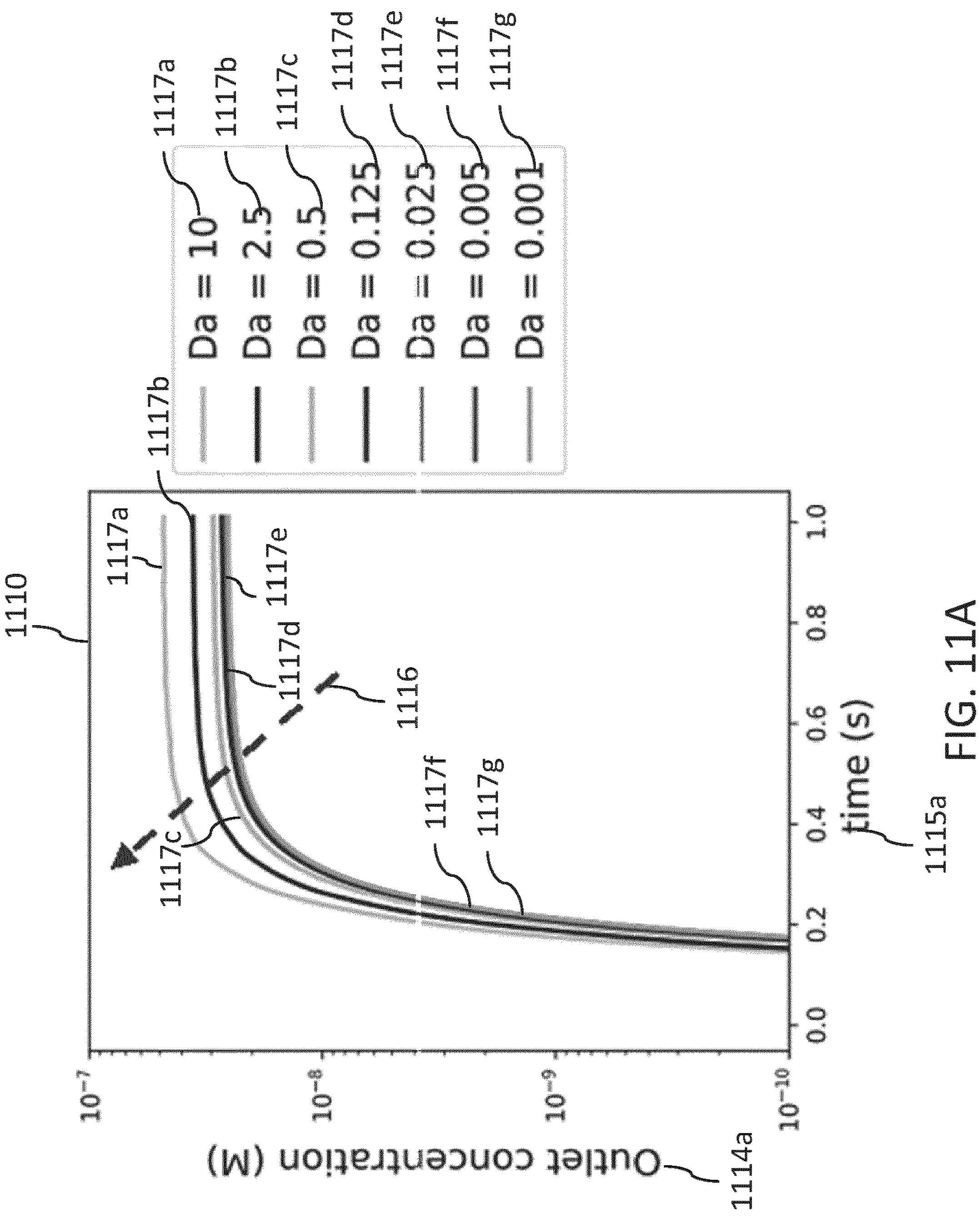
1110
1117a
1117b
1117c
1117d
1117e
1117f
1117g
1116
Da = 10
Da = 2.5
Da = 0.5
Da = 0.125
Da = 0.025
Da = 0.005
Da = 0.001
Outlet concentration (M)
1114a
time (s)
1115a
$10^{-7}$
$10^{-8}$
$10^{-9}$
$10^{-10}$
0.0
0.2
0.4
0.6
0.8
1.0

FIG. 11A

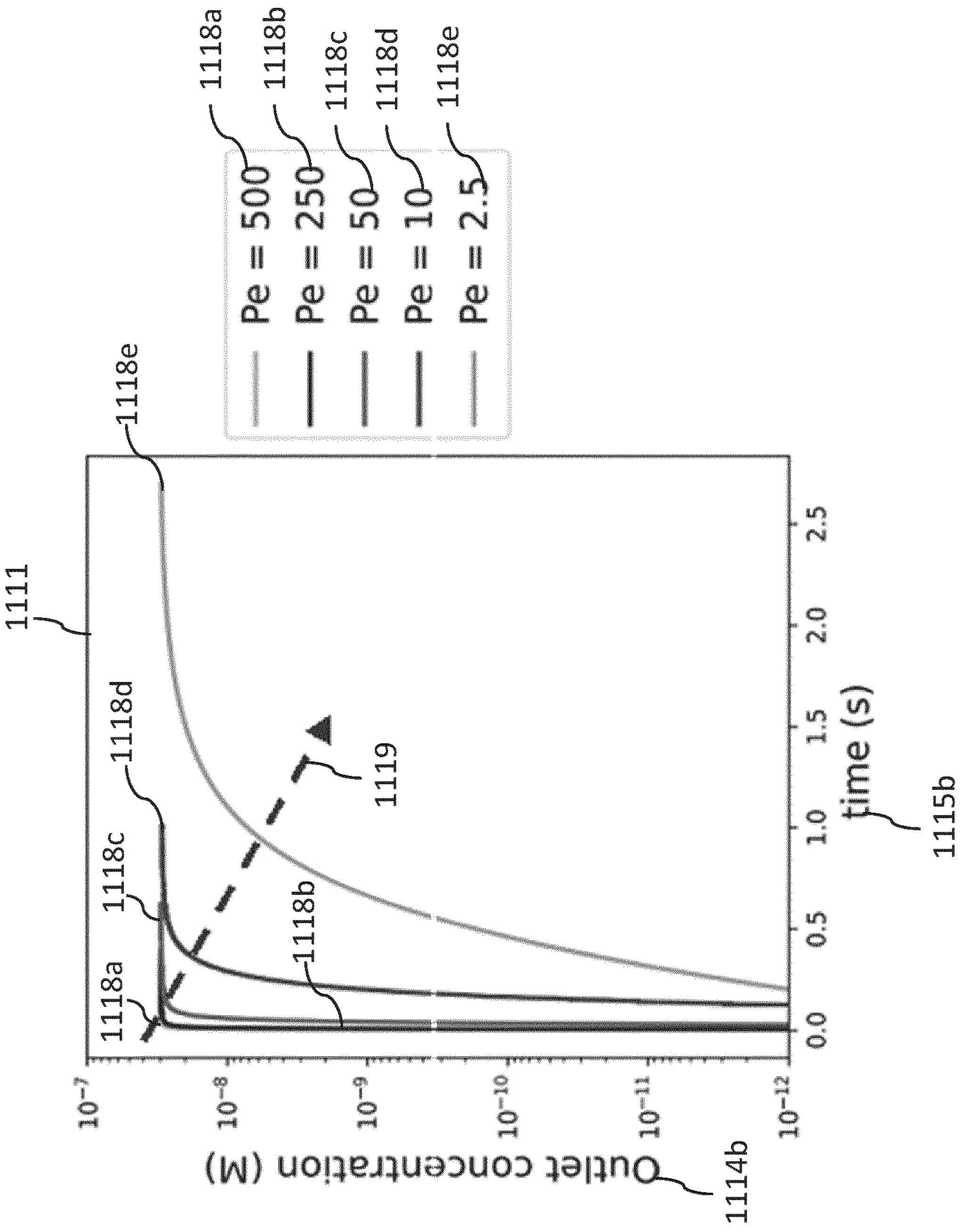
1111
1118a
1118b
1118c
1118d
1118e
Pe = 500
Pe = 250
Pe = 50
Pe = 10
Pe = 2.5
1119
Outlet concentration (M)
1114b
time (s)
1115b
$10^{-7}$
$10^{-8}$
$10^{-9}$
$10^{-10}$
$10^{-11}$
$10^{-12}$
0.0
0.5
1.0
1.5
2.0
2.5

FIG. 11B

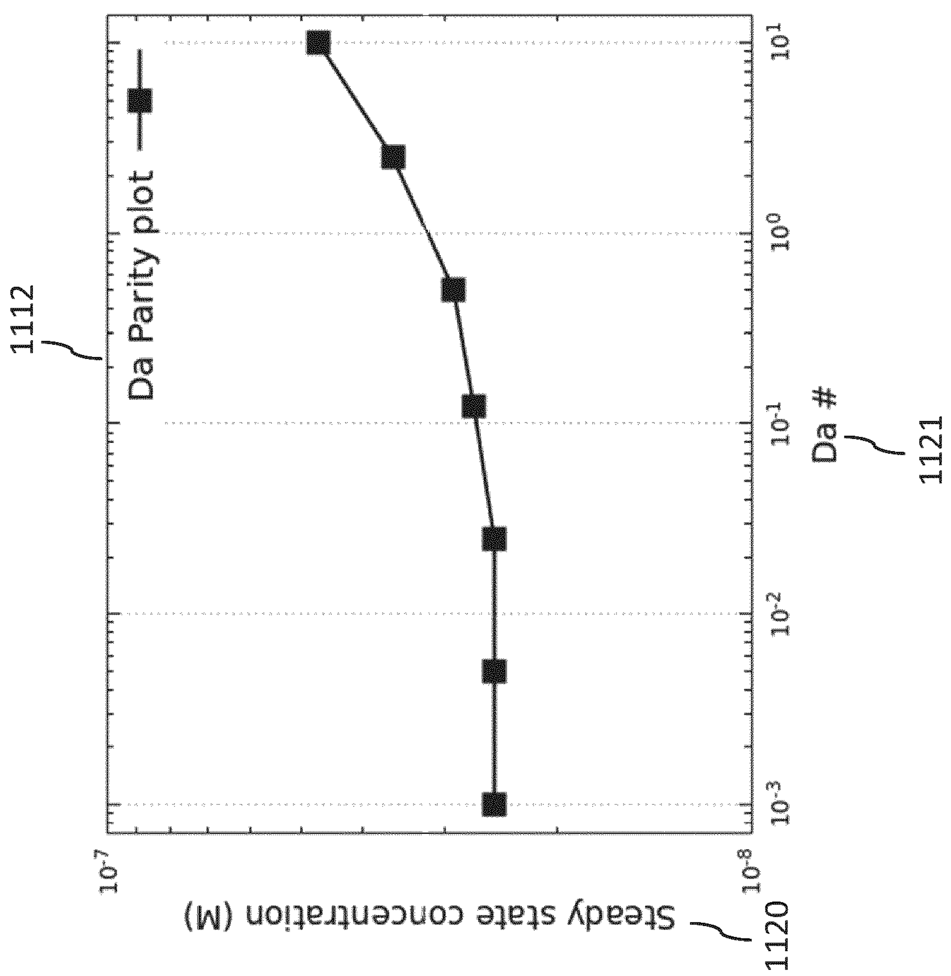

1112
Da Parity plot
Steady state concentration (M)
1120
$10^{-7}$
$10^{-8}$
$10^{-3}$
$10^{-2}$
$10^{-1}$
$10^{0}$
$10^{1}$
Da #
1121


FIG. 11C

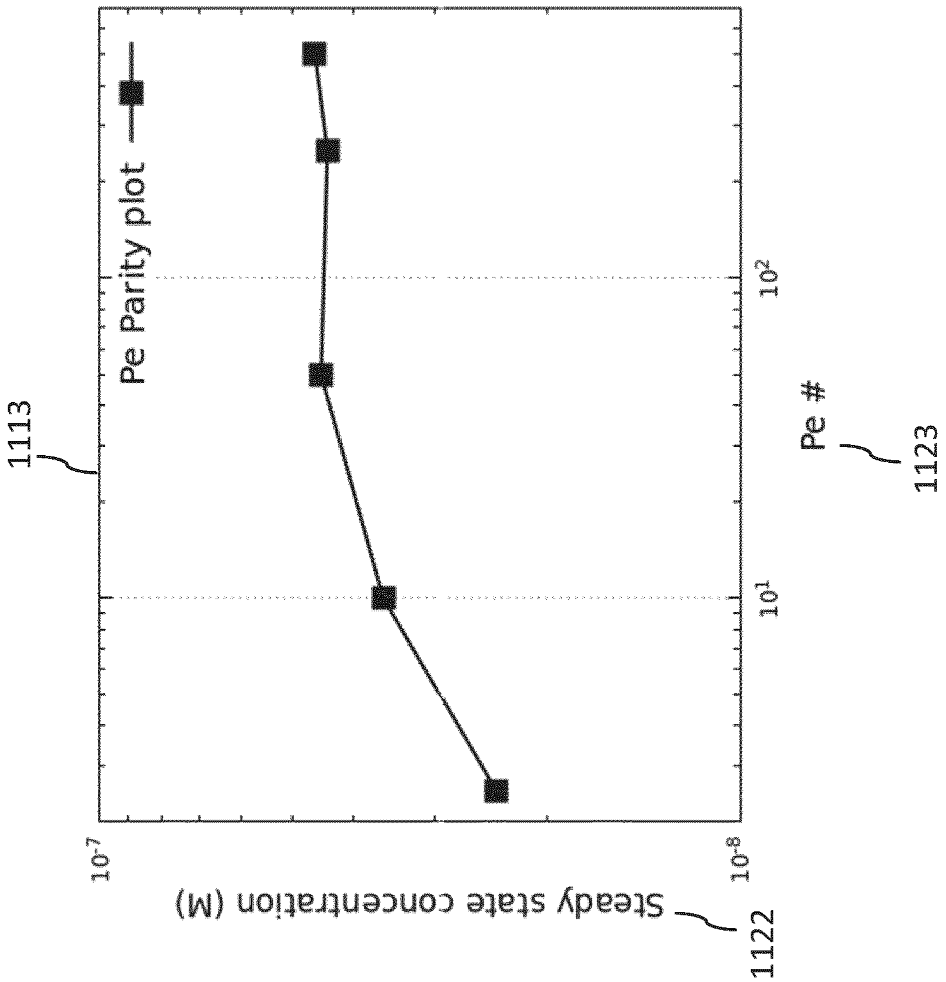
1113
Pe Parity plot
Steady state concentration (M)
1122
10-7
10-8
101
102
Pe #
1123

FIG. 11D

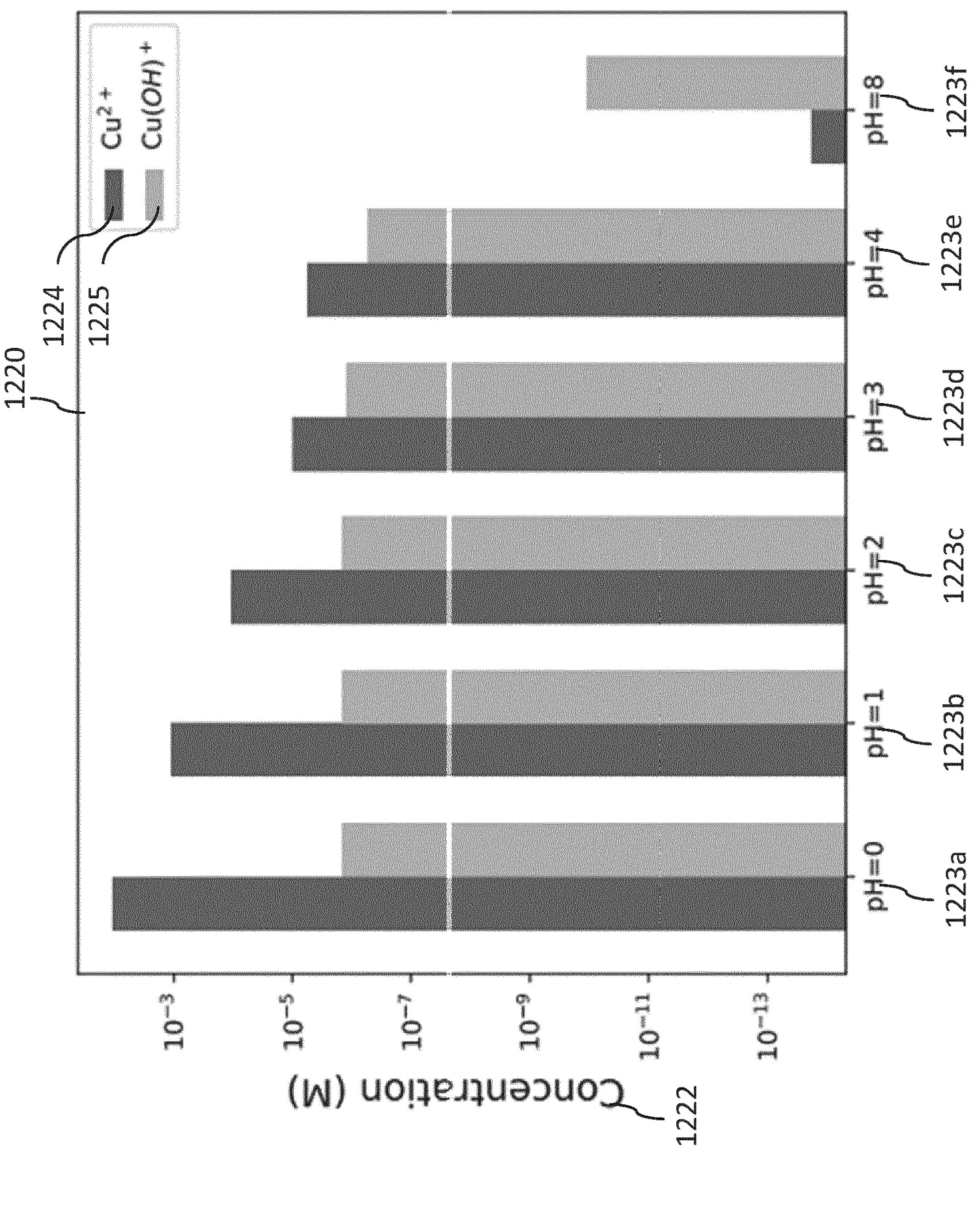
1220
1224
1225
Cu$^{2+}$
Cu(OH)$^{+}$
Concentration (M)
1222
$10^{-3}$
$10^{-5}$
$10^{-7}$
$10^{-9}$
$10^{-11}$
$10^{-13}$
pH=0
pH=1
pH=2
pH=3
pH=4
pH=8
1223a
1223b
1223c
1223d
1223e
1223f

FIG. 12A

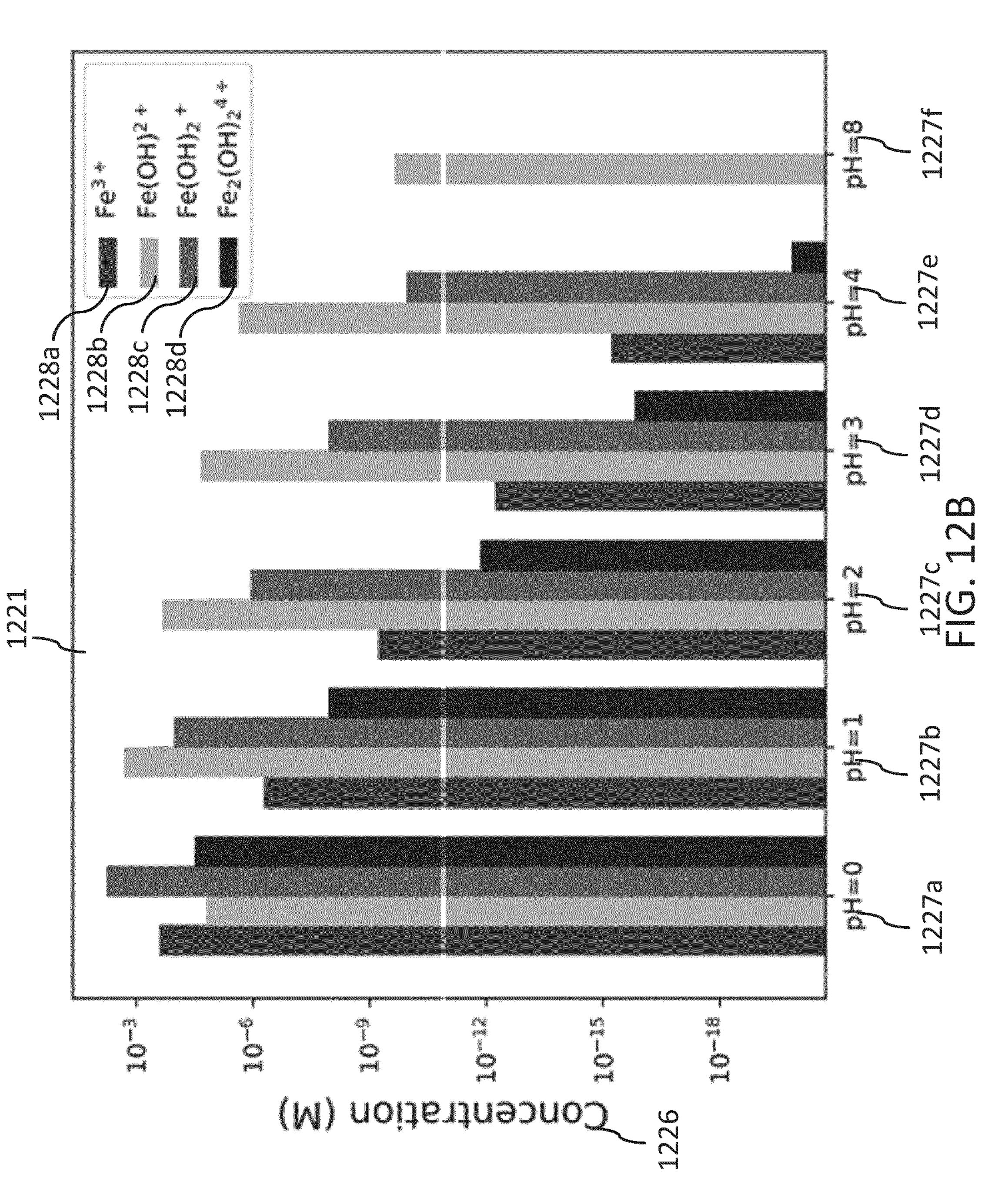
1221
1226
Concentration (M)
$10^{-3}$
$10^{-6}$
$10^{-9}$
$10^{-12}$
$10^{-15}$
$10^{-18}$
pH=0
pH=1
pH=2
pH=3
pH=4
pH=8
1227a
1227b
1227c
1227d
1227e
1227f
1228a
1228b
1228c
1228d
$Fe^{3+}$
$Fe(OH)^{2+}$
$Fe(OH)_2^{+}$
$Fe_2(OH)_2^{4+}$

FIG. 12B

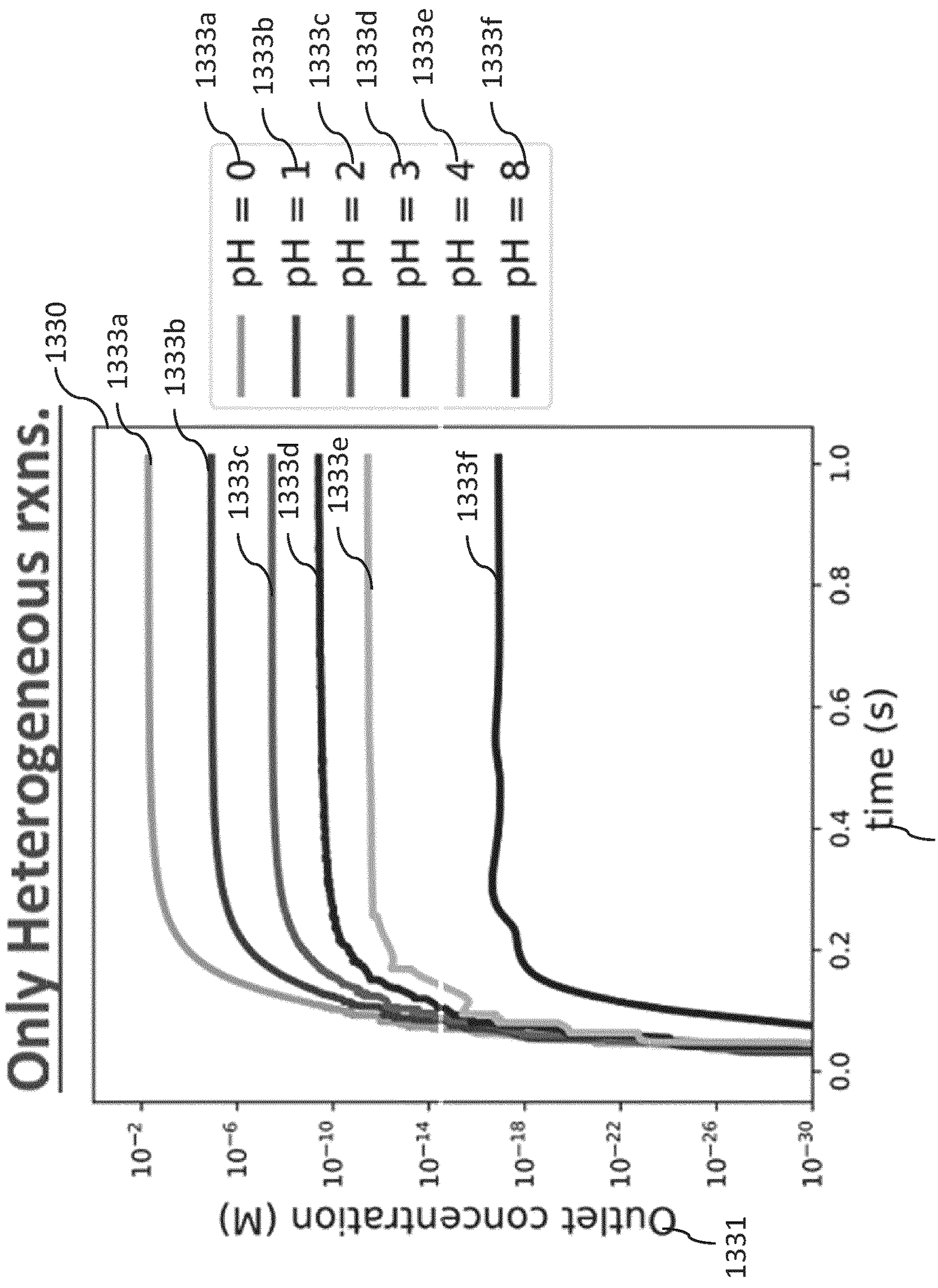
Only Heterogeneous rxns.
1330
1333a
1333b
1333c
1333d
1333e
1333f
pH = 0
pH = 1
pH = 2
pH = 3
pH = 4
pH = 8
Outlet concentration (M)
1331
$10^{-2}$
$10^{-6}$
$10^{-10}$
$10^{-14}$
$10^{-18}$
$10^{-22}$
$10^{-26}$
$10^{-30}$
0.0
0.2
0.4
0.6
0.8
1.0
time (s)
1332

FIG. 13A

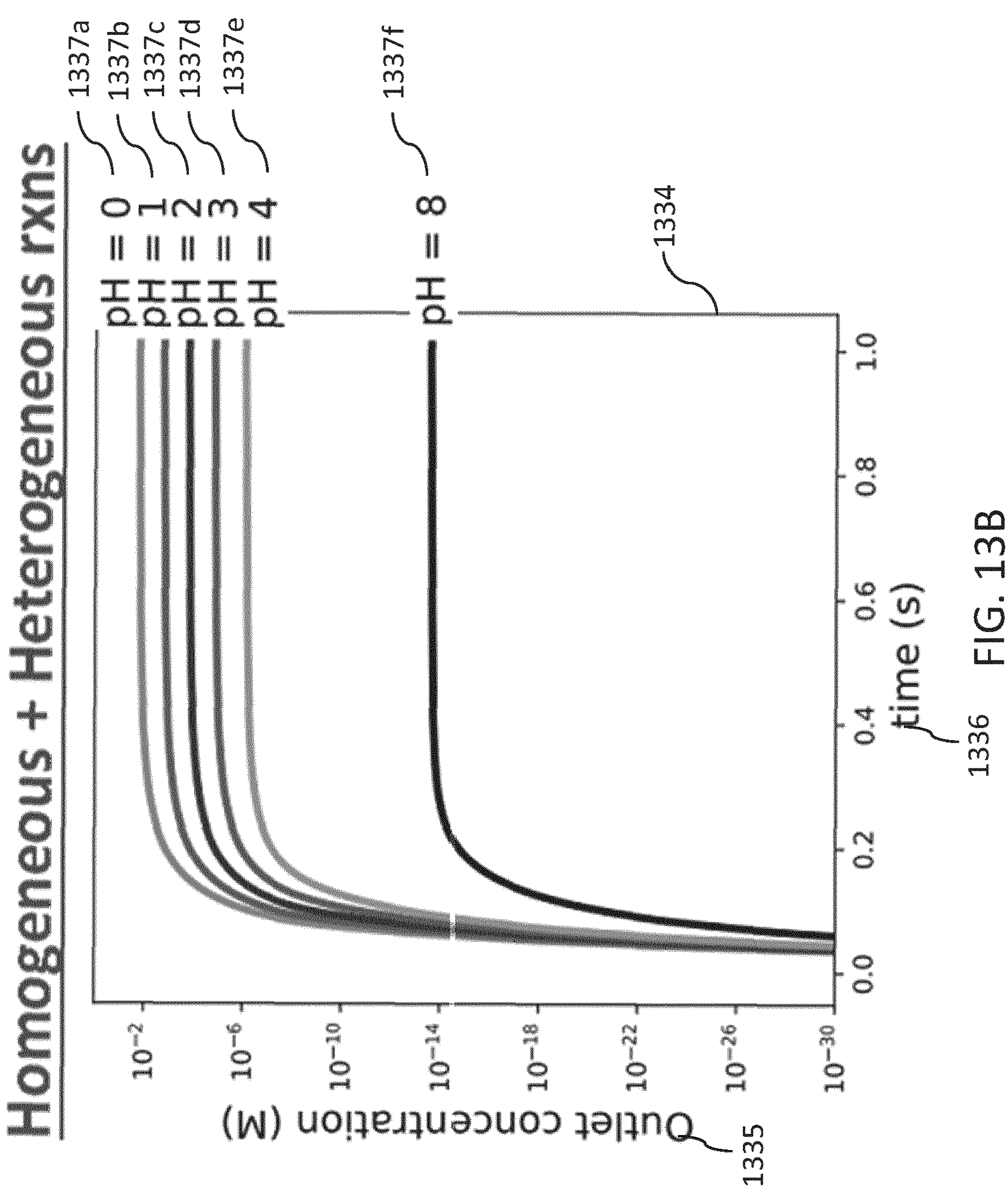
Homogeneous + Heterogeneous rxns
Outlet concentration (M)
1335
$10^{-2}$
$10^{-6}$
$10^{-10}$
$10^{-14}$
$10^{-18}$
$10^{-22}$
$10^{-26}$
$10^{-30}$
0.0
0.2
0.4
0.6
0.8
1.0
time (s)
1336
pH = 0
pH = 1
pH = 2
pH = 3
pH = 4
pH = 8
1337a
1337b
1337c
1337d
1337e
1337f
1334

FIG. 13B

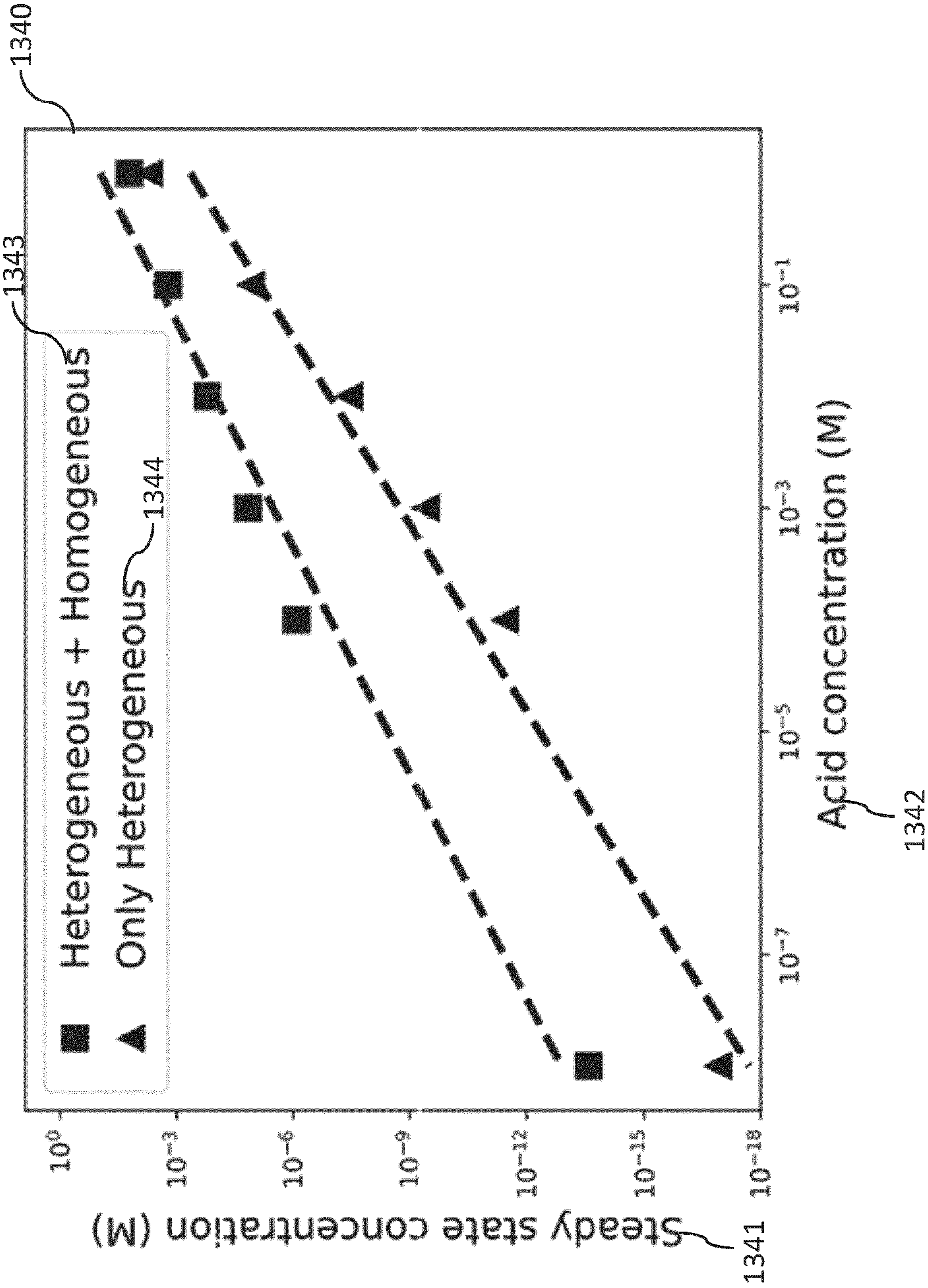
1340
1343
1344
Heterogeneous + Homogeneous
Only Heterogeneous
Steady state concentration (M)
1341
$10^{0}$
$10^{-3}$
$10^{-6}$
$10^{-9}$
$10^{-12}$
$10^{-15}$
$10^{-18}$
$10^{-7}$
$10^{-5}$
$10^{-3}$
$10^{-1}$
Acid concentration (M)
1342

FIG. 13C

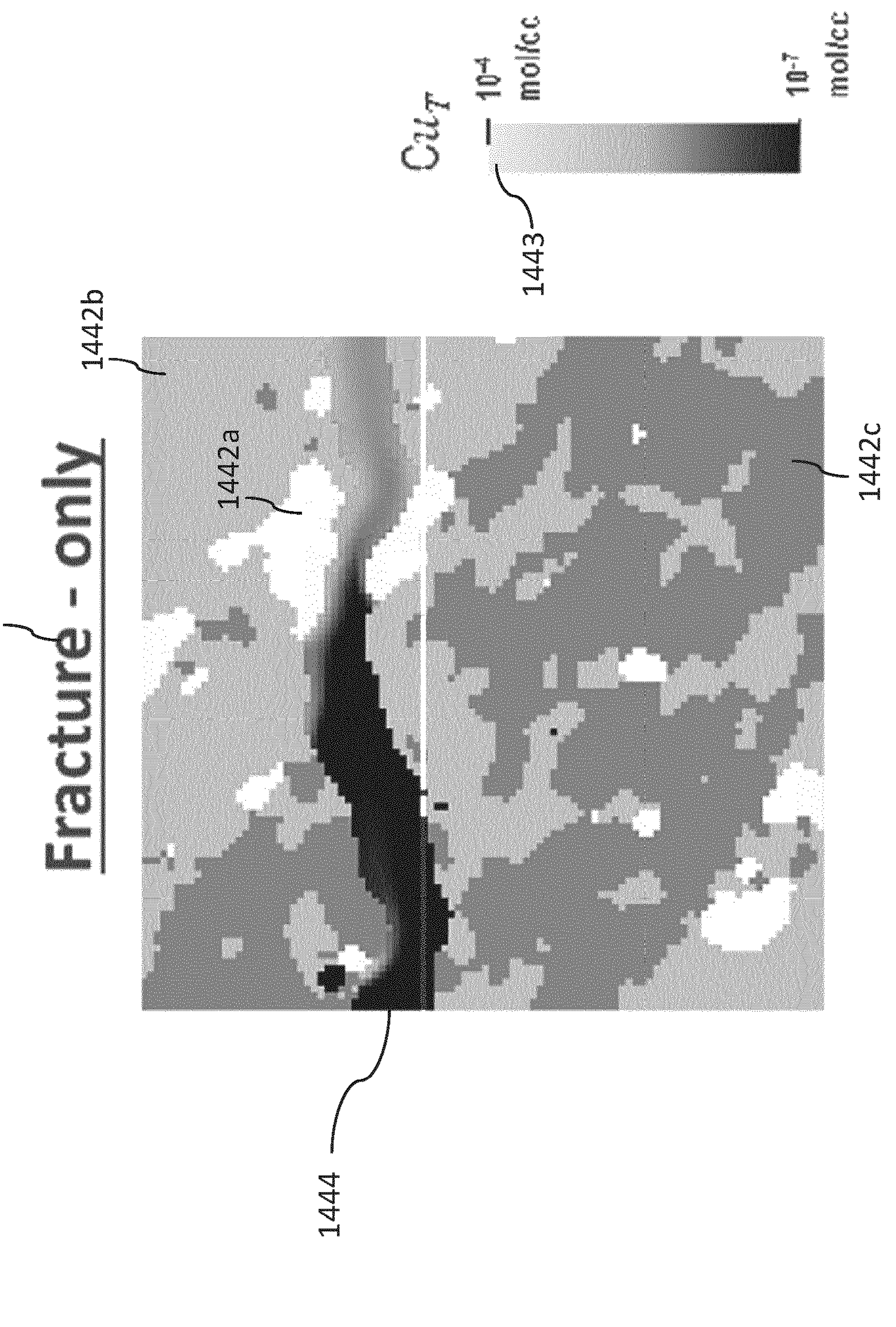
Fracture - only
1440
1442a
1442b
1442c
1443
1444
$Cu_T$
$10^{-4}$ mol/cc
$10^{-7}$ mol/cc

FIG. 14A

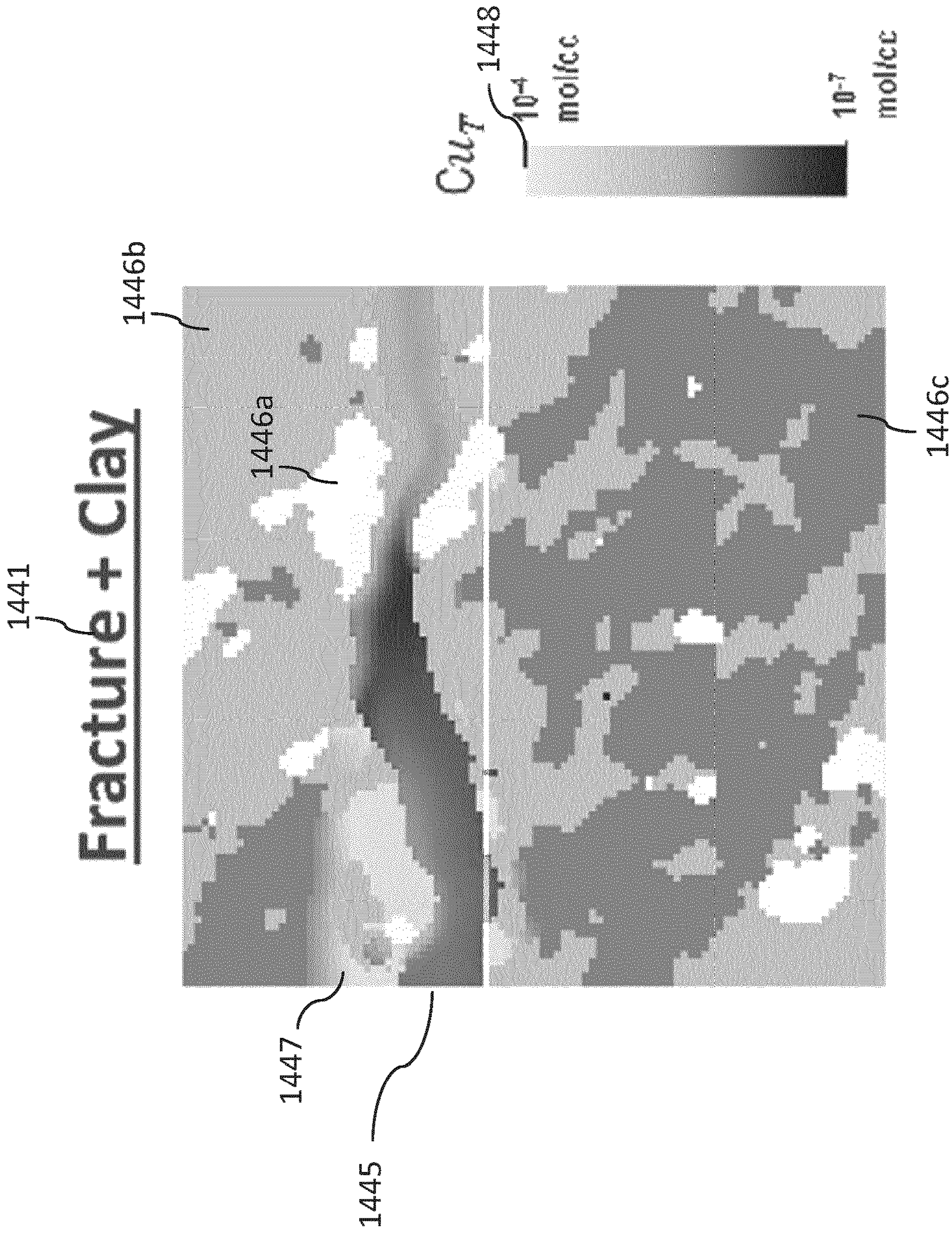
Fracture + Clay
1441
1446b
1446a
1447
1445
1448
1446c
$Cu_T$
$10^{-4}$ mol/cc
$10^{-7}$ mol/cc

FIG. 14B

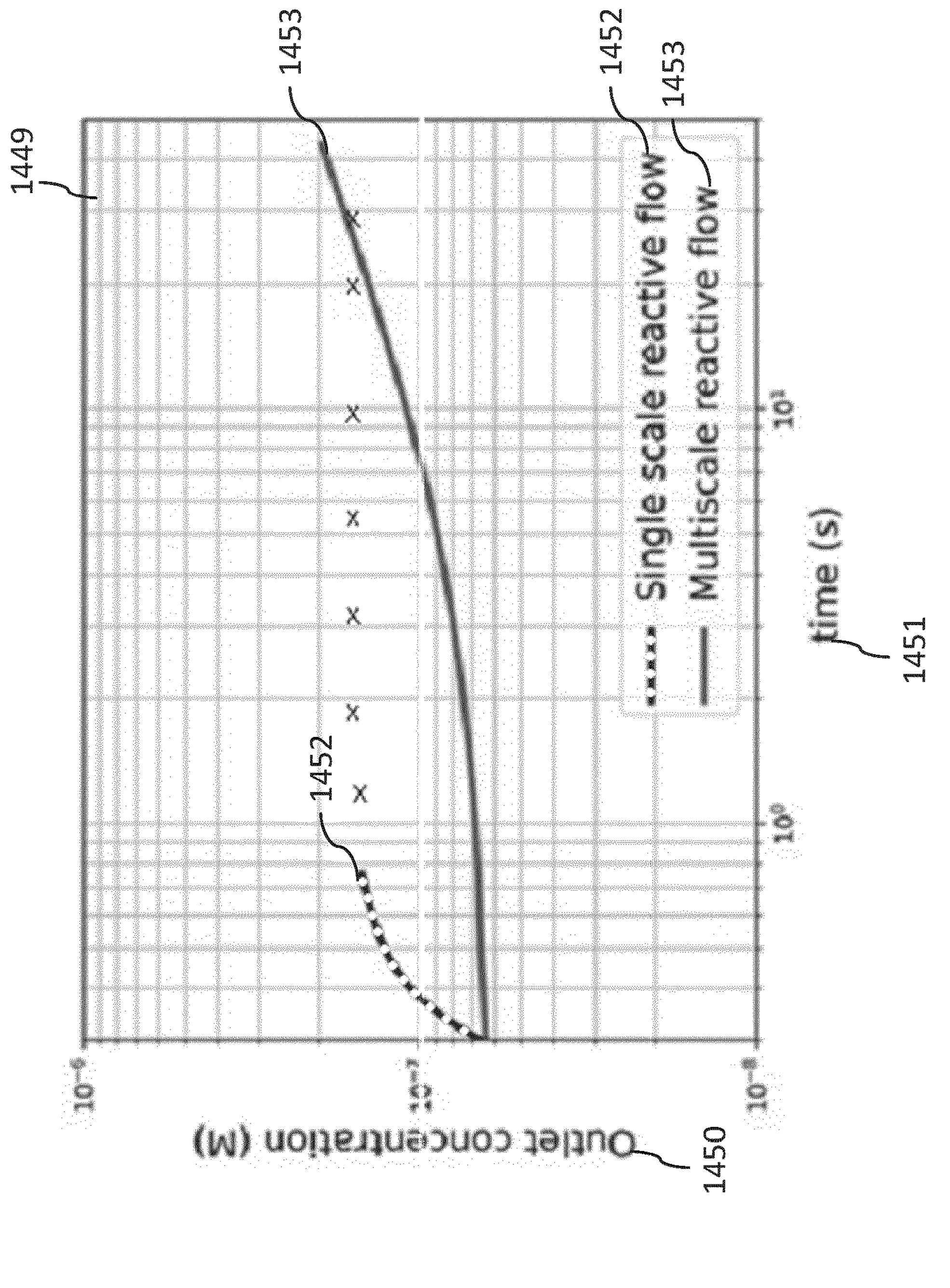
1449
1450
1451
1452
1453
Outlet concentration (M)
time (s)
$10^{-6}$
$10^{-7}$
$10^{-8}$
$10^{0}$
$10^{1}$
Single scale reactive flow
Multiscale reactive flow

FIG. 14C

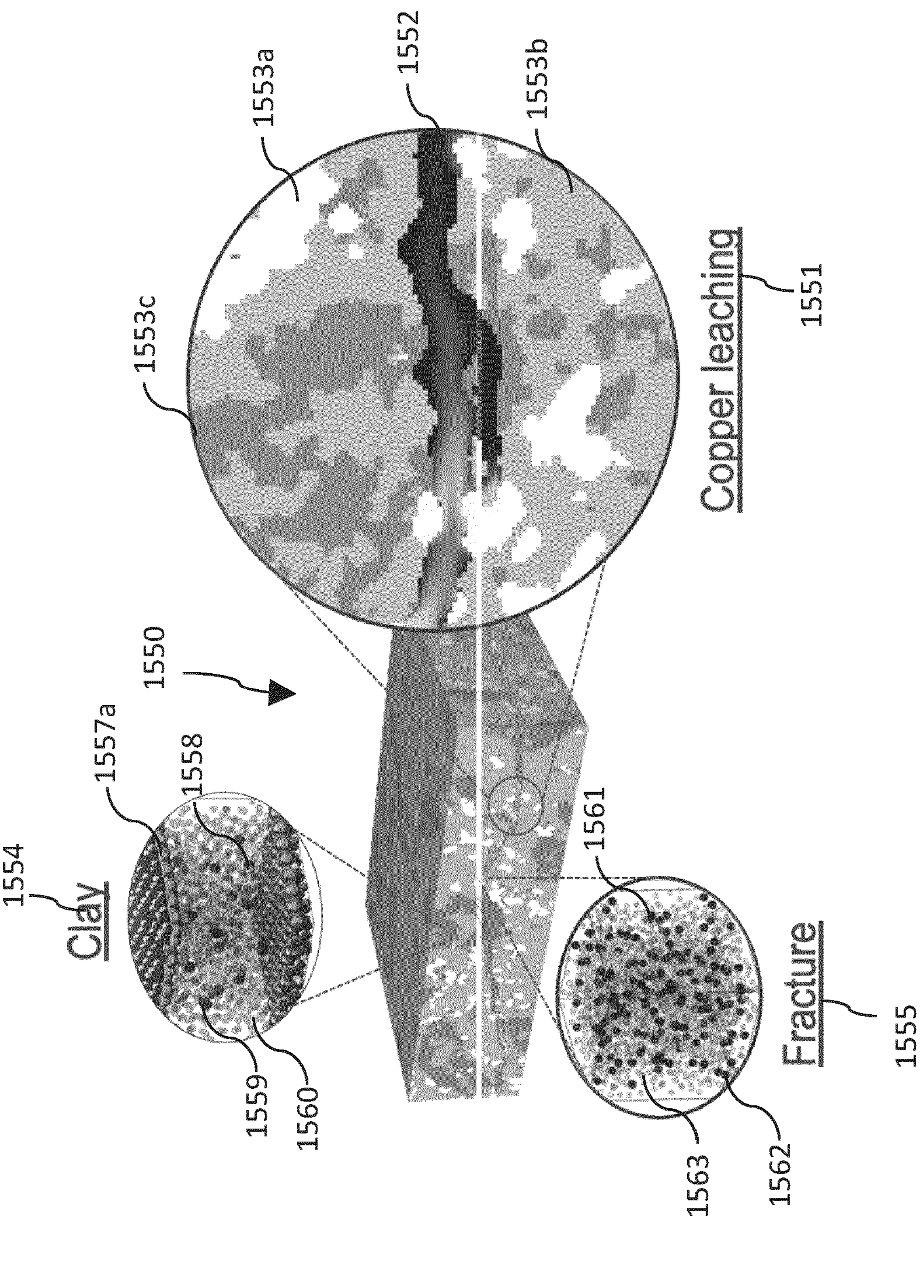
1552
1553a
1553b
1553c
Copper leaching
1551
1550
Clay
1554
1557a
1558
1559
1560
1561
Fracture
1555
1563
1562

FIG. 15

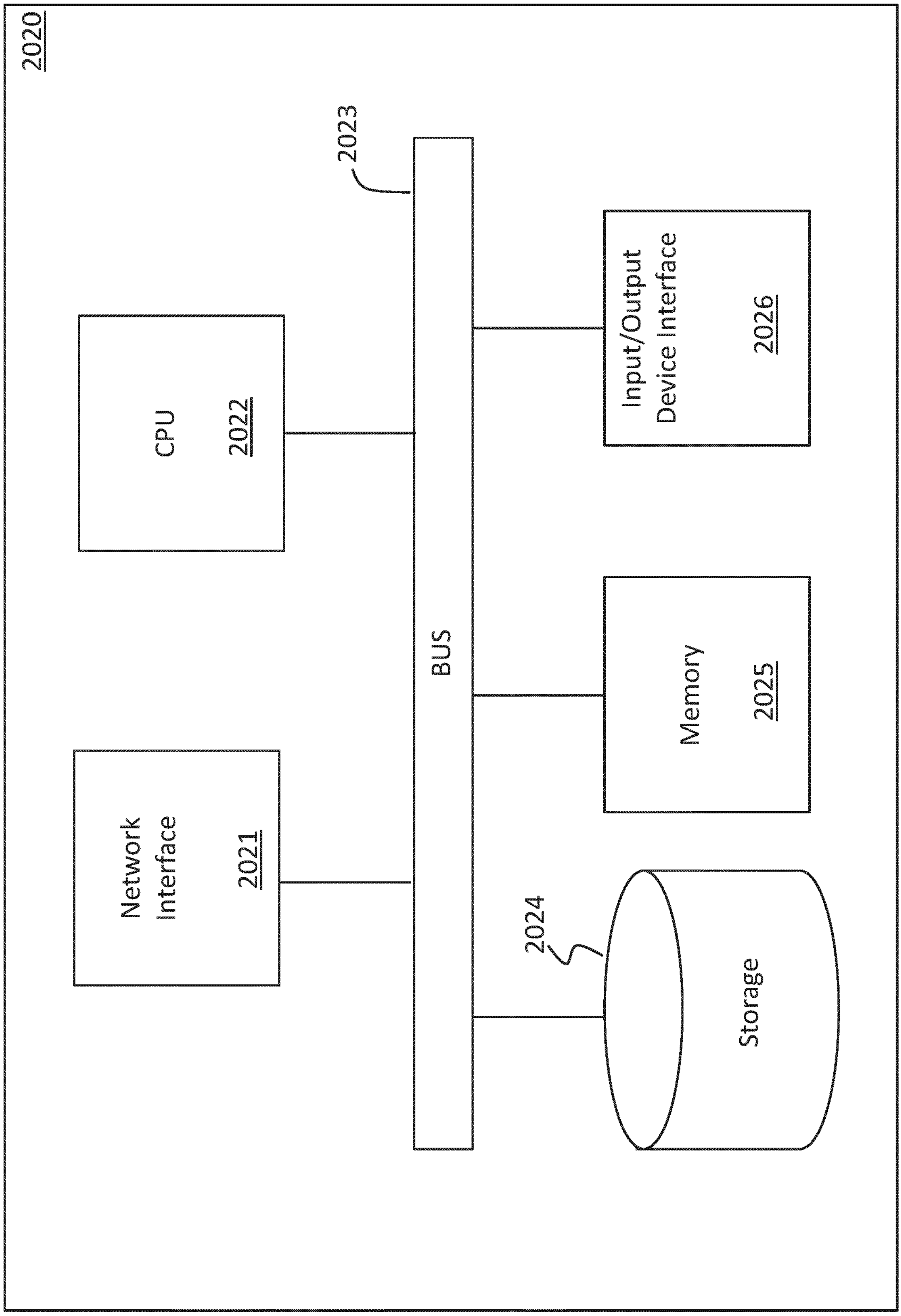
2020
2023
CPU
2022
Input/Output
Device Interface
2026
BUS
Network
Interface
2021
Memory
2025
2024
Storage

FIG. 16

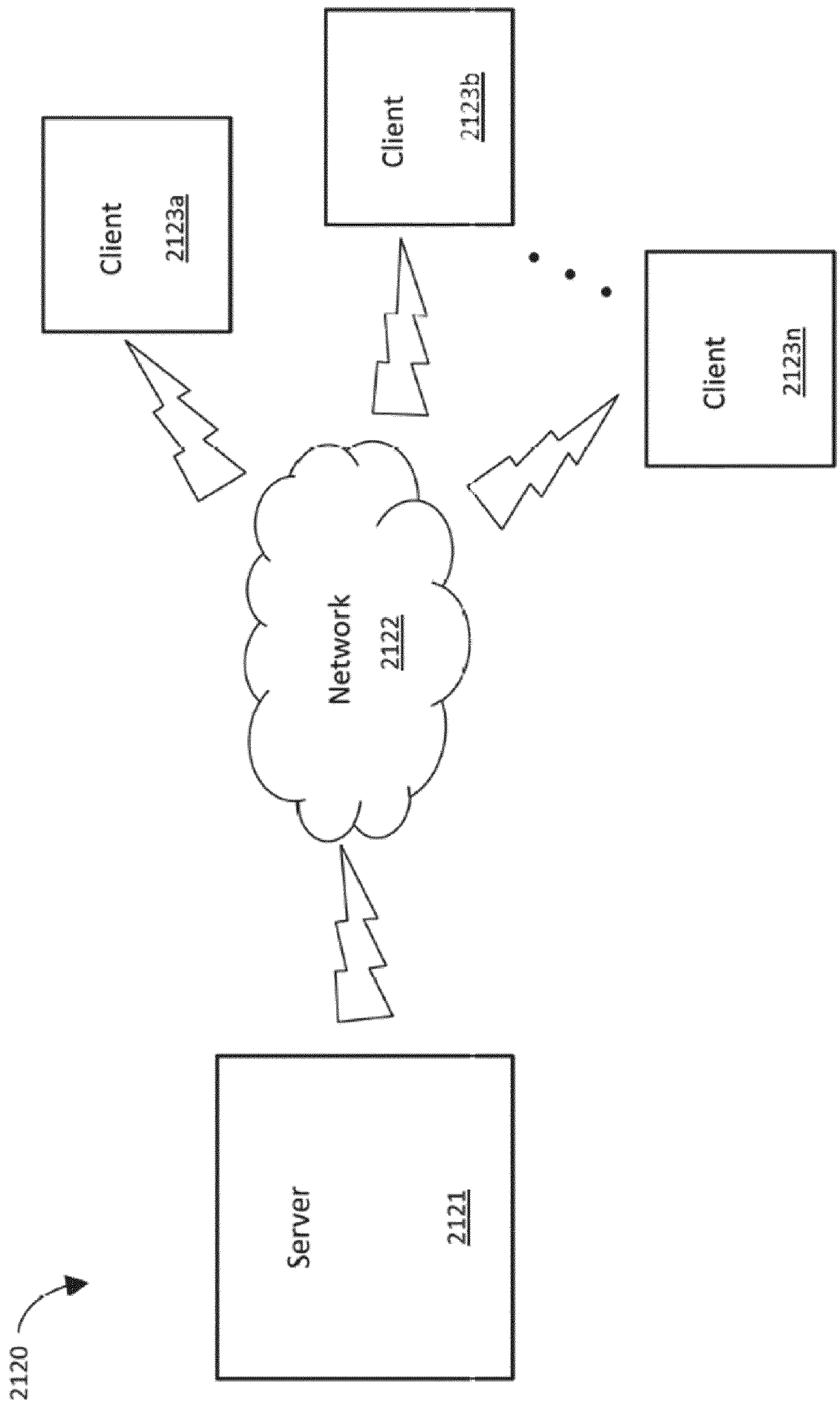
Client
2123a
Client
2123b
Client
2123n
Network
2122
Server
2121
2120

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20220207219 A1

### Non-patent literature cited in the description


- Lattice-Boltzmann simulation of dissolution of carbonate rock during CO2-saturated brine injection. **AN SENYOU et al.** CHEMICAL ENGENEERING JOURNAL. ELSEVIER, 21 October 2020, vol. 408
- **J. A. GREATHOUSE et al.** Molecular Dynamics Simulation of Diffusion and Electrical Conductivity in Montmorillonite Interlayers. *THE JOURNAL OF PHYSICAL CHEMISTRY C*, 20 January 2016, vol. 120 (3), 1640-1649
- **ABBOTT, J.W.** ; **HANKE, F.** Kinetically Corrected Monte Carlo-Molecular Dynamics Simulations of Solid Electrolyte Interphase Growth. *J. Chem. Theory Comput.*, 2022, vol. 18, 925-934, https://doi.org/10.1021/acs.jctc.1c00921
- **ABD, A.S.** ; **ABUSHAIKHA, A.S**. Reactive transport in porous media: a review of recent mathematical efforts in modeling geochemical reactions in petroleum subsurface reservoirs. *SN Appl. Sci.*, 2021, vol. 3, 1-28, https://doi.org/10.1007/s42452-021-04396-9
- **AGMON, N.** CHEMICAL PHYSICS The Grotthuss mechanism. *Chem. Phys. Lett.*, 1995, vol. 50, 456-462
- **AKKERMANS, R. L. C.** ; **SPENLEY, N. A.** ; **ROBERTSON, S. H**. COMPASS III: automated fitting workflows and extension to ionic liquids. *Molecular Simulation*, 2021, vol. 47, 540-551
- **AL-KHULAIFI, Y.** ; **LIN, Q.** ; **BLUNT, M.J.** ; **BIJELJIC, B**. Reaction Rates in Chemically Heterogeneous Rock: Coupled Impact of Structure and Flow Properties Studied by X-ray Microtomography. *Environ. Sci. Technol.*, 2017, vol. 51, 4108-4116, https://doi.org/10.1021/acs.est.6b06224
- **ALLEN, M. P** ; **TILDESLEY, D. J.** Computer Simulation of Liquids. Clarendon Press, 1987
- **BECKSTEIN, O.** ; **SANSOM, M.S.P.** Liquid-vapor oscillations of water in hydrophobic nanopores. *Proc. Natl. Acad. Sci. U. S. A.*, 2003, vol. 100, 7063-7068, https://doi.org/10.1073/pnas.1136844100
- **BOEK, E.S.** Molecular dynamics simulations of interlayer structure and mobility in hydrated Li-, Na- and K-montmorillonite clays. *Mol. Phys*, 2014, vol. 112, 1472-1483, https://doi.org/10.1080/00268976.2014.907630
- **CASEWIT, C.J.** ; **COLWELL, K.S** ; **RAPPÉ, A.K.** Application of a Universal Force Field to Main Group Compounds. *J. Am. Chem. Soc.*, 1992, vol. 114, 10046-10053, https://doi.org/10.1021/ja00051a042
- **CHATTERJEE A.** ; **EBINA T** ; **MIZUKAMI F**. Effects of Water on the Structure and Bonding of Resorcinol in the Interlayer of Montmorillonite Nanocomposite: A Periodic First Principle Study. *Phys. Chem. B*, 2005, vol. 109 (15), 7306-7313, https://doi.org/10.1021/jp045775z
- **CHEN, L.** ; **HE, Y.L.** ; **TAO, W.Q.** ; **ZELENAY, P** ; **MUKUNDAN, R** ; **KANG, Q**. Pore-scale study of multiphase reactive transport in fibrous electrodes of vanadium redox flow batteries. *Electrochim. Acta*, 2017, vol. 248, 425-439, https://doi.org/10.1016/j.electacta.2017.07.086
- **CYGAN, R.T** ; **LIANG, J.J** ; **KALINICHEV, A.G**. Molecular models of hydroxide, oxyhydroxide, and clay phases and the development of a general force field. *J. Phys. Chem. B*, 2004, vol. 108, 1255-1266, https://doi.org/10.1021/jp0363287
- **DAMIANI, L.H.** ; **KOSAKOWSKI, G** ; **GLAUS, M.A** ; **CHURAKOV, S. V**. A framework for reactive transport modeling using FEniCS-Reaktoro: governing equations and benchmarking results. *Comput. Geosci.*, 2020, vol. 24, 1071-1085, https://doi.org/10.1007/s10596-019-09919-3
- **DOOLEY, J.J.** ; **DAHOWSKI, R.T.** ; **DAVIDSON, C.L.** ; **WISE, M.A** ; **GUPTA, N** ; **KIM, S.H.** ; **MALONE, E.L.** Carbon dioxide capture and geologic storage-A core element of a global energy technology strategy to address climate change. *Jt. Glob. Chang. Res. Inst. 1-37*, 2006
- **ESSMANN, U.** ; **PERERA, L.** ; **BERKOWITZ, M.L** ; **DARDEN, T** ; **LEE, H.** ; **PEDERSEN, L.G**. A smooth particle mesh Ewald method. *J. Chem. Phys.*, 1995, vol. 103, 8577-8593, https://doi.org/10.1063/1.470117
- **FAGER A.** ; **CROUSE B** ; **SUN G** ; **XU R.** ; **FREED D.** Evaluation of Directly Simulated WAG Hysteresis at Pore Scale and its Effect on Injectivity Index. *SPE 195734, SPE Offshore Europe Conf. & Exhib.*, 2019

- **FAGER A** ; **OTOMO H** ; **SALAZAR-TIO R** ; **BALASUBRAMANIAN G** ; **CROUSE B.** ; **ZHANG R** ; **CHEN H.** ; **SCHEMBRE-MCCABE J**. Multi-scale Digital Rock: Application of a multi-scale multi-phase workflow to a Carbonate reservoir rock. *Int. Symp. Soc. Core Analysts*, 2021
- **FREED, D.** Lattice-Boltzmann Method for Macroscopic Porous Media Modeling.. *International Journal of Modern Physics C*, 1998, vol. 09 (08), 1491-1503, https://doi.org/10.1142/S0129183198001357
- **HOLMBOE, M.** ; **BOURG, I.C.** Molecular dynamics simulations of water and sodium diffusion in smectite interlayer nanopores as a function of pore size and temperature. *J. Phys. Chem. C*, 2014, vol. 118, 1001-1013, https://doi.org/10.1021/jp408884g
- **JERAULD, G** ; **FREDRICH J.** ; **LANE N.** ; **SHENG Q** ; **CROUSE B** ; **FREED D.** ; **FAGER A** ; **XU R.** Validation of a Workflow for Digitally Measuring Relative Permeability. *In. Society of Petroleum Engineers*, 2017, https://doi.org/10.2118/188688-MS
- **KANG, Q.** ; **LICHTNER, P.C** ; **ZHANG, D**. An improved lattice Boltzmann model for multicomponent reactive transport in porous media at the pore scale. *Water Resour. Res.*, 2007, vol. 43, 1-12, https://doi.org/10.1029/2006WR005551
- **KOSAKOWSKI, G.** ; **CHURAKOV, S. V** ; **THOENEN, T.** Diffusion of Na and Cs in montmorillonite. *Clays Clay Miner.*, 2008, vol. 56, 190-206, https://doi.org/10.1346/CCMN.2008.0560205
- **KREVOR, S** ; **BLUNT, M.J.** ; **BENSON, S.M.** ; **PENTLAND, C.** ; **REYNOLDS, C** ; **AL-MENHALI, A.** ; **NIU, B.** Capillary trapping for geologic carbon dioxide storage - From pore scale physics to field scale implications. *Int. J. Greenh. Gas Control*, 2015, vol. 40, 221-237, https://doi.org/10.1016/j.ijggc.2015.04.006
- **KRYNICKI, K** ; **GREEN, C.D** ; **SAWYER, D.W**. Pressure and temperature dependence of self-diffusion in water. *Faraday Discuss. Chem. Soc.*, 1978, vol. 66, 199-208, https://doi.org/10.1039/DC9786600199
- **LI, L.** ; **PETERS, C.A** ; **CELIA, M.A.** Upscaling geochemical reaction rates using pore-scale network modeling. *Adv. Water Resour.*, 2006, vol. 29, 1351-1370, https://doi.org/10.1016/j.advwatres.2005.10.011
- Modelling the injectivity, migration and trapping of CO2 in carbon capture and storage (CCS), Geological Storage of Carbon Dioxide (co2): Geoscience, Technologies. **MACKAY, E.J.** Environmental Aspects and Legal Frameworks. Woodhead Publishing Limited, 2013
- **MACQUARRIE, K.T.B** ; **MAYER, K.U.** Reactive transport modeling in fractured rock: A state-of-the-science review. *Earth-Science Rev*, 2005, vol. 72, 189-227, https://doi.org/10.1016/j.earscirev.2005.07.003
- **MAES, J** ; **MENKE, H.P.** GeoChemFoam: Direct Modelling of Multiphase Reactive Transport in Real Pore Geometries with Equilibrium Reactions. *Transp. Porous Media*, 2021, vol. 139, 271-299, https://doi.org/10.1007/s11242-021-01661-8
- **MOLINS, S.** ; **SOULAINE, C** ; **PRASIANAKIS, N.I.** ; **ABBASI, A** ; **PONCET, P** ; **LADD, A.J.C** ; **STARCHENKO, V** ; **ROMAN, S** ; **TREBOTICH, D** ; **TCHELEPI, H.A**. Simulation of mineral dissolution at the pore scale with evolving fluid-solid interfaces: review of approaches and benchmark problem set.. *Comput. Geosci.*, 2021, vol. 25, 1285-1318, https://doi.org/10.1007/s10596-019-09903-x
- **NOIRIEL, C.** Resolving Time-dependent Evolution of Pore-Scale Structure, Permeability and Reactivity using X-ray Microtomography. *Rev. Mineral. Geochemistry*, 2015, vol. 80, 247-285
- **NOIRIEL, C.** ; **DAVAL, D.** Pore-Scale Geochemical Reactivity Associated with CO2 Storage: New Frontiers at the Fluid-Solid Interface. *Acc. Chem. Res.*, 2017, vol. 50, 759-768, https://doi.org/10.1021/acs.accounts.7b00019
- **NORGATE, T.E.** ; **JAHANSHAHI, S** ; **RANKIN, W.J**. Assessing the environmental impact of metal production processes. *J. Clean. Prod.*, 2007, vol. 15, 838-848, https://doi.org/10.1016/j.jclepro.2006.06.018
- **NOSÉ, S.** A unified formulation of the constant temperature molecular-dynamics methods. *Journal of Chemical Physics.*, 1984, vol. 81 (1), 511-519
- **OLIVEIRA, T.D.S** ; **BLUNT, M.J.** ; **BIJELJIC, B**. Modelling of multispecies reactive transport on pore-space images. *Adv. Water Resour*, 2019, vol. 127, 192-208, https://doi.org/10.1016/j.advwatres.2019.03.012
- **PARKHURST, D.L.** ; **WISSMEIER, L.** PhreeqcRM: A reaction module for transport simulators based on the geochemical model PHREEQC. *Adv. Water Resour.*, 2015, vol. 83, 176-189, https://doi.org/10.1016/j.advwatres.2015.06.001
- **RAHROMOSTAQIM, M** ; **SAHIMI, M**. Molecular Dynamics Study of the Effect of Layer Charge and Interlayer Cations on Swelling of Mixed-Layer Chlorite-Montmorillonite Clays. *J. Phys. Chem. C*, 2020, vol. 124, 2553-2561, https://doi.org/10.1021/acs.jpcc.9b10919
- **RIGBY, D.** Fluid density predictions using the COMPASS force field. *Fluid Phase Equilib.*, 2004, vol. 217, 77-87, https://doi.org/10.1016/j.fluid.2003.08.019
- **SINCLAIR, L.** ; **THOMPSON, J**. In situ leaching of copper: Challenges and future prospects. *Hydrometallurgy*, 2015, 306-324
- **SMITH, D.E.** Molecular computer simulations of the swelling properties and interlayer structure of cesium montmorillonite. *Langmuir*, 1998, vol. 14, 5959-5967, https://doi.org/10.1021/la980015z

- **SPOSITO, G.** ; **SKIPPER, N.T** ; **SUTTON, R** ; **PARK, S.H** ; **SOPER, A.K.** ; **GREATHOUSE, J.A.** Surface geochemistry of the clay minerals. *Proc. Natl. Acad. Sci. U. S. A.*, 1999, vol. 96, 3358-3364, https://doi.org/10.1073/pnas.96.7.3358
- **SRIDHAR, N**. Local corrosion chemistry - A review. *Corrosion*, 2017, vol. 73, 18-30, https://doi.org/10.5006/2246
- **STEEFEL, C.I.** ; **APPELO, C.A.J.** ; **ARORA, B.** ; **JACQUES, D** ; **KALBACHER, T.** ; **KOLDITZ, O** ; **LAGNEAU, V.** ; **LICHTNER, P.C.** ; **MAYER, K.U.** ; **MEEUSSEN, J.C.L.** Reactive transport codes for subsurface environmental simulation. *Computational Geosciences*, 2015, https://doi.org/10.1007/s10596-014-9443-x
- **STEEFEL, C.I.** ; **DEPAOLO, D.J.** ; **LICHTNER, P.C**. Reactive transport modeling: An essential tool and a new research approach for the Earth sciences. *Earth Planet. Sci. Lett*, 2005, vol. 240, 539-558, https://doi.org/10.1016/j.epsl.2005.09.017
- **SUN G.** ; **SUN Z.** ; **FAGER A** ; **CROUSE B**. Pore-scale Analysis of CO2-brine Displacement in Berea Sandstone and Its Implications to CO2 Injectivity. *Int. Symp. Soc. Core Analysts*, 2022
- **TEICH-MCGOLDRICK, S.L.** ; **GREATHOUSE, J.A.** ; **JOVÉ-COLÓN, C.F** ; **CYGAN, R.T**. Swelling Properties of Montmorillonite and Beidellite Clay Minerals from Molecular Simulation: Comparison of Temperature, Interlayer Cation, and Charge Location Effects. *J. Phys. Chem. C*, 2015, vol. 119, 20880-20891, https://doi.org/10.1021/acs.jpcc.5b03253
- **WU, Y.** ; **TEPPER, H.L.** ; **VOTH, G.A.** Flexible simple point-charge water model with improved liquid-state properties. *J. Chem. Phys*, 2006, vol. 124, https://doi.org/10.1063/1.2136877
- **XU, T.** ; **SONNENTHAL, E.** ; **SPYCHER, N** ; **PRUESS, K.** TOUGHREACT - A simulation program for non-isothermal multiphase reactive geochemical transport in variably saturated geologic media: Applications to geothermal injectivity and CO2 geological sequestration.. *Comput. Geosci.*, 2006, vol. 32, 145-165, https://doi.org/10.1016/j.cageo.2005.06.014
- **XU, R.** ; **CROUSE B.** ; **FREED D.** ; **FAGER A.** ; **JERAULD G** ; **LANE N.** ; **SHENG Q.** Continuous vs Discontinuous Capillary Desaturation and Implications for IOR/EOR. *SCA 2018-066, Int. Symp. Soc. Core Analysts*, 2018
- **YOON, H.** ; **KANG, Q.** ; **VALOCCHI, A.J.** Lattice Boltzmann-based approaches for pore-scale reactive transport. *Pore Scale Geochemical Process.*, 2015, vol. 80, 393-431, https://doi.org/10.2138/rmg.2015.80.12